(19) Europäisches Patentamt
European Patent Office
Office européen des brevets

(11) Publication number: **0 388 994 B1**

# (12) EUROPEAN PATENT SPECIFICATION

(45) Date of publication of patent specification: **05.10.94**

(51) Int. Cl.$^5$: **C07D 401/12**, A01N 47/36, C07D 213/81, C07D 213/82, C07D 213/89, //C07D213/80

(21) Application number: **90107643.0**

(22) Date of filing: **21.01.87**

(60) Publication number of the earlier application in accordance with Art.76 EPC: **0 232 067**

The file contains technical information submitted after the application was filed and not included in this specification

(54) **Substituted pyridinesulfonamide compounds, herbicidal composition containing them, and method of preparing these compounds.**

(30) Priority: **30.01.86 JP 19006/86**
**31.01.86 JP 19863/86**
**15.04.86 JP 86847/86**
**29.07.86 JP 178489/86**

(43) Date of publication of application:
**26.09.90 Bulletin 90/39**

(45) Publication of the grant of the patent:
**05.10.94 Bulletin 94/40**

(84) Designated Contracting States:
**AT CH DE ES FR GB IT LI NL**

(56) References cited:
**EP-A- 0 007 687**
**EP-A- 0 013 480**
**EP-A- 0 030 138**
**EP-A- 0 035 893**
**EP-A- 0 101 407**
**EP-A- 0 101 670**
**EP-A- 0 237 292**

(73) Proprietor: **ISHIHARA SANGYO KAISHA, LTD.**
**No. 3-22, Edobori 1-chome**
**Nishi-ku Osaka (JP)**

(72) Inventor: **Kimura, Fumio, c/o Ishihara Sangyo K.K.**
**Chuo Kenkyusho,**
**3-1, Nishishibukawa 2-chome**
**Kusatsu-shi, Shiga-ken (JP)**
Inventor: **Haga, Takahiro, c/o Ishihara Sangyo K.K.**
**Chuo Kenkyusho,**
**3-1, Nishishibukawa 2-chome**
**Kusatsu-shi, Shiga-ken (JP)**
Inventor: **Sakashita, Nobuyuki, c/o Ishihara Sangyo K.K.**
**Chuo Kenkyusho,**
**3-1, Nishishibukawa 2-chome**
**Kusatsu-shi, Shiga-ken (JP)**
Inventor: **Honda, Chimoto, c/o Ishihara Sangyo K.K.**
**Chuo Kenkyusho,**
**3-1, Nishishibukawa 2-chome**
**Kusatsu-shi, Shiga-ken (JP)**

EP 0 388 994 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Inventor: **Murai, Shigeo, c/o Ishihara Sangyo K.K.**
**Chuo Kenkyusho,**
**3-1, Nishishibukawa 2-chome**
**Kusatsu-shi, Shiga-ken (JP)**

(74) Representative: **Pearce, Anthony Richmond et al**
**MARKS & CLERK,**
**Alpha Tower,**
**Suffolk Street Queensway**
**Birmingham B1 1TT (GB)**

## Description

The present invention relates to novel substituted pyridinesulfonamide compounds and their salts, herbicidal compositions containing them as active ingredients, and a process for producing these compounds.

A substituted pyridinesulfonamide compound of this invention is characterized in that a pyridine ring has an N-substituted aminocarbonyl group. Such substituted pyridinesulfonamide compound is known by general formulas in U.S.P. No. 4,518,776, European Patent Application Publication No. 101670, and U.S.P. No. 4,521,597. However, the substituted pyridinesulfonamide of this invention is not concretely disclosed in the above prior patents. A certain substituted pyridinesulfonamide compound is described in U.S.P. No. 4,435,206. However, this compound is not the one wherein the pyridine ring has an N-substituted aminocarbonyl group. A large number of analogues of a herbicide containing a sulfonamide compound as an active ingredient have been developed. However, no herbicides showing a high safety against corns and having a high herbicidal effect have been developed yet.

The present inventors have made extensive studies on the relation between chemical structures and physiological activities against plants with respect to the sulfonamide compounds. In particular, the present inventors have made further studies for finding a herbicide for corns. As a result, the present inventors reached a conclusion that a pyridinesulfonamide compound having a pyridine ring with an N-substituted amino-carbonyl group, and a particular substituted group (if any) and having sulfonamide moiety substituted on the nitrogen atom with specifically substituted pyrimidin-2-ylaminocarbonyl group was effective as the herbicide for corn fields, and thus the inventors attained the present invention.

According to a first aspect of the present invention, there is provided a substituted pyridinesulfonamide compound represented by the following general formula (I):

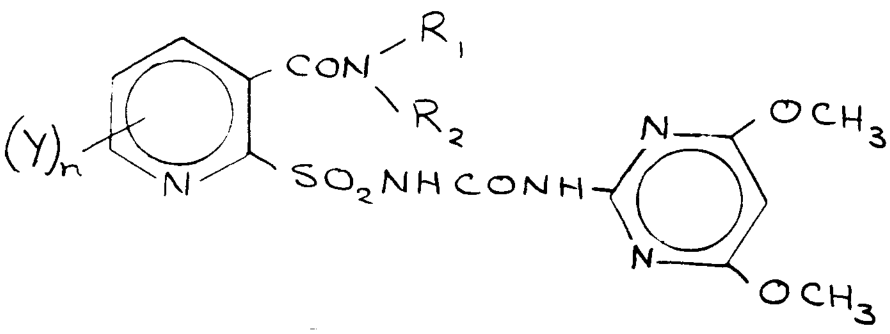

(I)

wherein each of $R_1$ and $R_2$ independently represents a hydrogen atom, a $(C_1-C_6)$alkyl group, a halo$(C_1-C_6)$-alkyl group, a $(C_2-C_6)$alkenyl group, a $(C_2-C_6)$alkynyl group, a $(C_1-C_6)$alkoxy group, a halo$(C_1-C_6)$alkoxy group, a $(C_1-C_6)$alkoxy$(C_1-C_6)$alkyl group, a halo$(C_1-C_6)$alkoxy$(C_1-C_6)$alkyl group, a $(C_3-C_6)$cycloalkyl group, a halo$(C_3-C_6)$cycloalkyl group, a $(C_1-C_6)$alkoxycarbonyl group, a halo$(C_1-C_6)$alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1-C_6)$alkyl group, a halo$(C_1-C_6)$alkyl group, a $(C_1-C_6)$alkoxy group, a halo$(C_1-C_6)$alkoxy group, a $(C_1-C_6)$alkylthio group, a halo$(C_1-C_6)$alkylthio group, a $(C_1-C_6)$alkoxy$(C_1-C_6)$alkyl group, a halo$(C_1-C_6)$alkoxy$(C_1-C_6)$alkyl group, or an

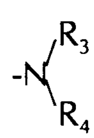

group (wherein each of $R_3$ and $R_4$ represents a hydrogen atom or a $(C_1-C_6)$alkyl group); and $\underline{n}$ represents 0 or an integer of 1 or 2; with the provisos that (a) when $\underline{n}$ is 0, $R_1$ and $R_2$ are not both methyl groups, (b) when $R_1$ and $R_2$ are both $(C_1-C_3)$alkyl or when $R_1$ is hydrogen and $R_2$ is $(C_1-C_3)$alkyl, and $\underline{n}$ is 1, Y is not Cl, F, Br, $CH_3$, $OCH_3$, $CF_3$ or $OCF_2H$, and (c) when $R_1$ is a hydrogen atom or a $(C_1-C_3)$alkyl group and $R_2$ is a $(C_1-C_2)$alkoxy group, and $\underline{n}$ is 1, Y is not $CF_3$ or $OCF_2H$; or a salt of such a compound.

Salts of the substituted pyridinesulfonamide compounds according to this invention include salts of alkali metals (e.g., sodium and potassium), salts of alkaline earth metals (e.g. magnesium and calcium), and salts of amines (e.g. monomethylamine, dimethylamine and triethylamine). These salts may be prepared by a conventional method.

In the above general formula (I), the halo($C_1$-$C_6$)alkyl, halo($C_1$-$C_6$)alkoxy, halo($C_1$-$C_6$)alkoxy($C_1$-$C_6$)alkyl, halo($C_1$-$C_6$)alkoxycarbonyl, halo($C_3$-$C_6$)cycloalkyl, or halophenyl group represented by $R_1$ and $R_2$, and the halo($C_1$-$C_6$) alkyl, halo($C_1$-$C_6$)alkoxy, halo($C_1$-$C_6$)alkoxythio, or halo($C_1$-$C_6$)alkoxy($C_1$-$C_6$) alkyl group represented by Y may be substituted with one or more halogen atoms.

Examples of the ($C_1$-$C_6$)alkyl group or moiety represented by $R_1$, $R_2$, $R_3$, $R_4$ and Y in the above general formula (I) are a methyl group, an ethyl group, a propyl group, and a butyl group. Examples of the ($C_2$-$C_6$)alkenyl group or moiety represented by $R_1$ and $R_2$ are a propenyl group and a butenyl group. Examples of the ($C_2$-$C_6$)alkynyl group or moiety are a propyenyl group and a butynyl group. Examples of the ($C_3$-$C_6$)cycloalkyl group are a cyclopropyl group, a cyclobutyl group, a cyclopentyl group, and a cyclohexyl group. Examples of the halogen atom in $R_1$, $R_2$ and Y are fluorine, chlorine, bromine and iodine. As defined above, $R_1$ and $R_2$ together with an adjacent nitrogen atom may constitute a heterocycle. Examples of such a heterocycle are a morpholine ring, an ethyleneimine ring, a pyrrolidine ring, and a piperidine ring.

Subject to the above provisos, more preferable embodiments will be described below:

(1) $R_1$ represents a hydrogen atom or an alkyl group, and preferably a hydrogen atom or a methyl group, and $R_2$ represents an alkyl group, and preferably a methyl group.

(2) Y represents, a halogen atom, a ($C_1$-$C_6$)alkyl group, a halo($C_1$-$C_6$)alkyl group, a ($C_1$-$C_6$)alkoxy group, or a ($C_1$-$C_6$)alkoxy($C_1$-$C_6$)alkyl group, and preferably, a chlorine atom, a bromine atom, a methyl group, or a difluoromethyl group, each of which is bonded at the 6-position of the pyridine nucleus; and n is 0, 1 or 2, and preferably 0 or 1.

(3) More specifically, the following compounds are preferred:

N-[(4,6-dimethoxypyrimidin-2-yl)aminocarbonyl]-3-methylaminocarbonyl-2-pyridinesulfonamide or 6-difluoromethyl-N-[(4,6-dimethoxypyrimidin-2-yl)aminocarbonyl]-3-dimethylaminocarbonyl-2-pyridinesulfonamide.

The substituted pyridinesulfonamide compounds represented by the above general formula (I) and their salts according to the present invention can be prepared by the following methods:

A pyridine compound represented by general formula (II)

$R - SO_2Z_1$ (II)

wherein R represents

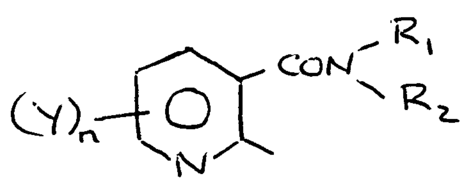

(wherein $R_1$, $R_2$, Y and n are as defined above)

and $Z_1$ represents an $-NH_2$ group, an -NCO group, an -NHCOCl group, or an $-NHCOOR_5$ group (wherein $R_5$ represents an alkyl or aryl group) is reacted with a pyrimidine compound represented by the following general formula:

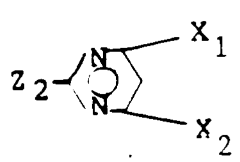

... (III)

wherein each of $X_1$ and $X_2$ independently represents a halogen atom or a methoxy group, $Z_2$ represents an $-NH_2$ group, an -NCO group, an -NHCOCl group, or an $-NHCOOR_5$ group (wherein $R_5$ is as defined above),

provided that when $Z_1$ represents an $-NH_2$ group, $Z_2$ represents an -NCO, -NHCOCl, or $-NHCOOR_5$ group, and that when $Z_2$ represents an $-NH_2$ group, $Z_1$ represents an -NCO, -NHCOCl or $-NHCOOR_5$ group. Subsequently, if $X_1$ and/or $X_2$ is a halogen atom, methoxylation is performed. In addition, a salt formation treatment is performed, if desired.

More specifically, the pyridinesulfonamide compound and its derivative can be prepared by methods [A] to [G].

[A] 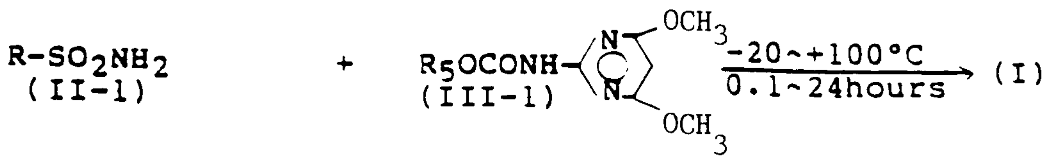

(II-1) (III-1) (I)

[B] 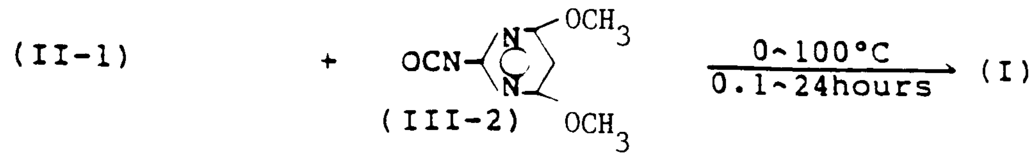

(II-1) (III-2) (I)

[C] 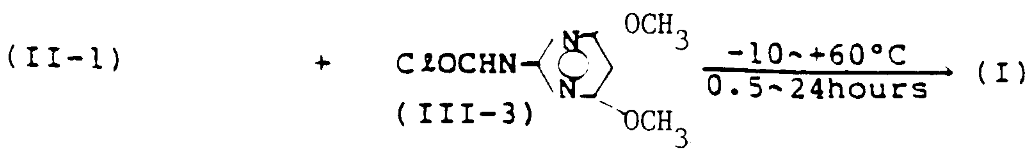

(II-1) (III-3) (I)

[D] 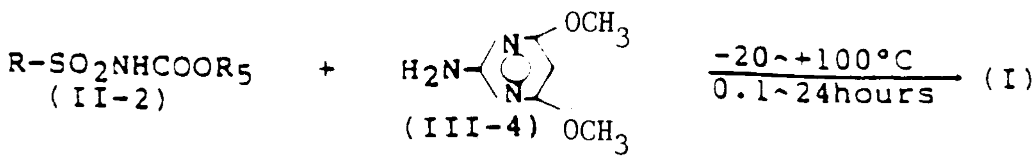

(II-2) (III-4) (I)

[E] 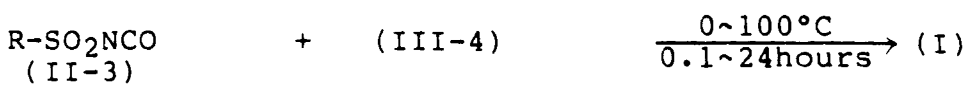

(II-3) (III-4) (I)

[F] 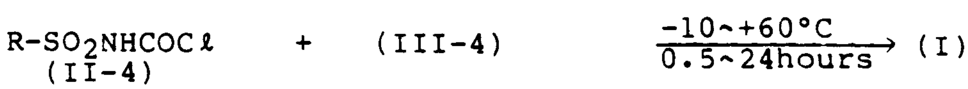

(II-4) (III-4) (I)

[G] 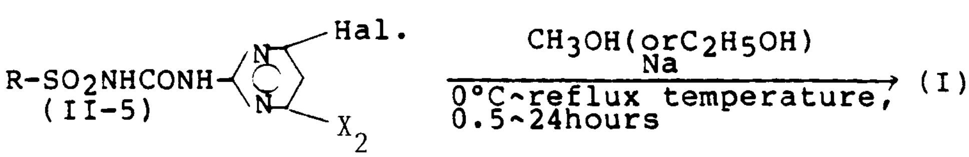

(II-5) (I)

The following method can also be utilized:

[H] 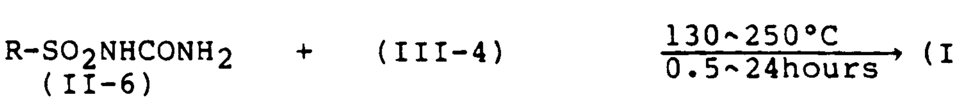

(II-6) (III-4) (I)

In the reaction equation of methods [A] to [H], Hal means a halogen atom, and R, $R_5$ and $X_2$ are as defined above. A compound represented by general formula (II-5) can be prepared according to the sequence of methods [A] to [F]. The compound represented by general formula (II-6) can be easily

prepared by reacting the compounds represented by general formulas (II-2) to (II-4) with ammonia. The aryl group represented by $R_5$ in general formulas (III-1) and (II-2) may be a phenyl or naphthyl group which may be substituted with one or more chlorine atom or one or more methyl groups. In methods [A] and [D], 1,8-diazabicyclo[5•4•0]-7-undecene may be added to accelerate the reactions, if desired. In methods [B], [C], [E], and [F], 1,4-diazabicyclo[2•2•2]-octane may be added, if desired. In methods [B], [C], [E], and [F], a base such as triethylamine or pyridine may be added, if desired. Methods [A] to [H] are practised in the presence of a solvent, if desired. Examples of the solvent are: aromatic hydrocarbons (e.g., benzene, toluene, xylene, and chlorobenzene); cyclic or acyclic aliphatic hydrocarbons (e.g., chloroform, carbon tetrachloride, methylene chloride, dichloroethane, trichloroethane, hexane, and cyclohexane); ethers (e.g., diethylether, dioxane, and tetrahydrofuran); nitriles (e.g., acetonitrile, propionitrile, or acrylonitrile); and aprotic polar solvents (e.g., dimethylsulfoxide and sulfolane).

(1) It is shown that the pyridine compound (II-1') in General Formula (II-1) as the starting material for the reaction formula can be prepared by the process in routes 1 and 2:

Route 1

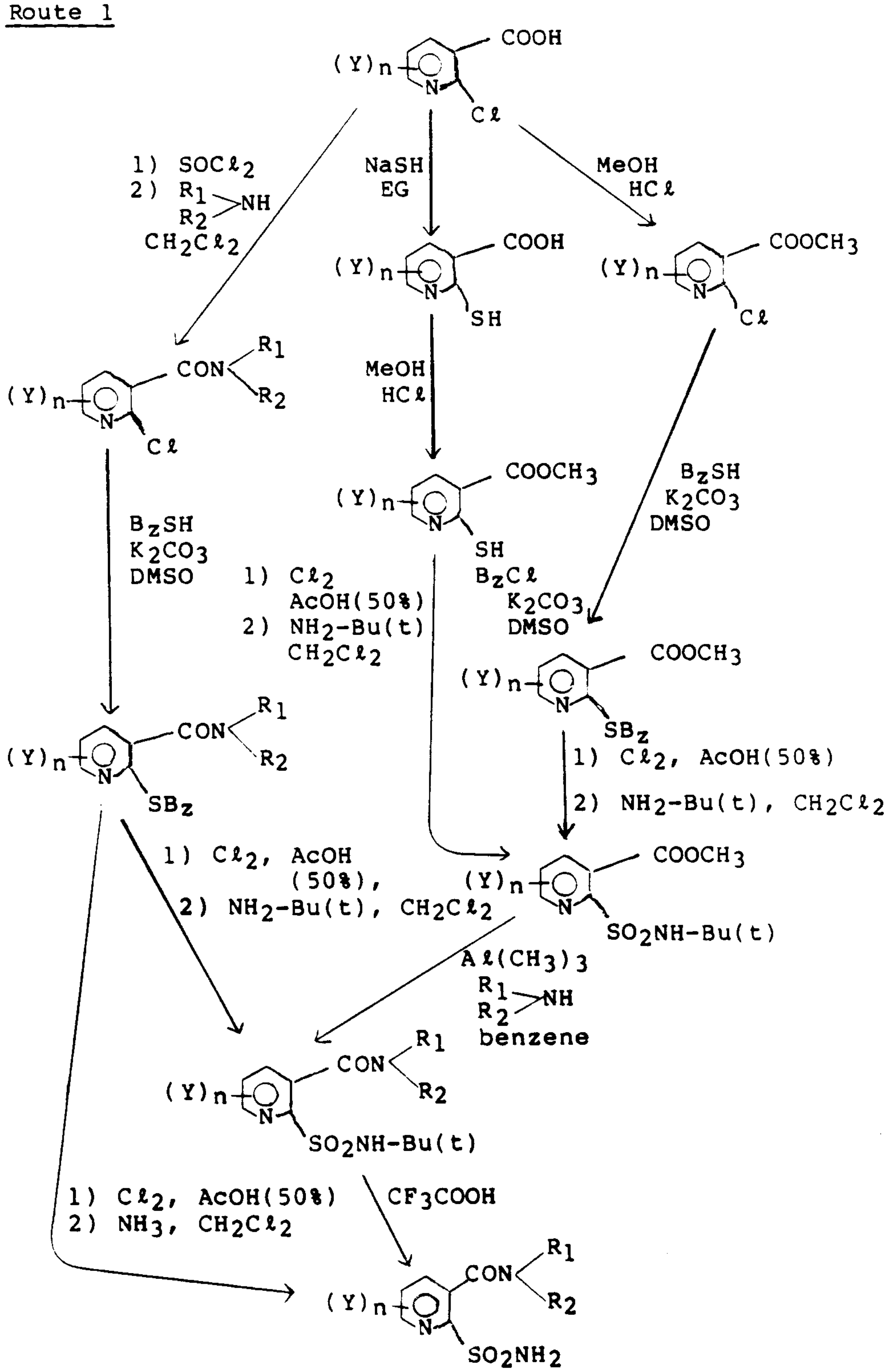
NaSH
EG
1) SOCℓ2
2) R1>NH R2
CH2Cℓ2
MeOH
HCℓ
MeOH
HCℓ
BzSH
K2CO3
DMSO
BzSH
K2CO3
DMSO
1) Cℓ2
AcOH(50%)
2) NH2-Bu(t)
CH2Cℓ2
BzCℓ
K2CO3
DMSO
1) Cℓ2, AcOH(50%)
2) NH2-Bu(t), CH2Cℓ2
1) Cℓ2, AcOH
(50%),
2) NH2-Bu(t), CH2Cℓ2
Aℓ(CH3)3
R1>NH R2
benzene
1) Cℓ2, AcOH(50%)
2) NH3, CH2Cℓ2
CF3COOH

(II-1')

Route 2

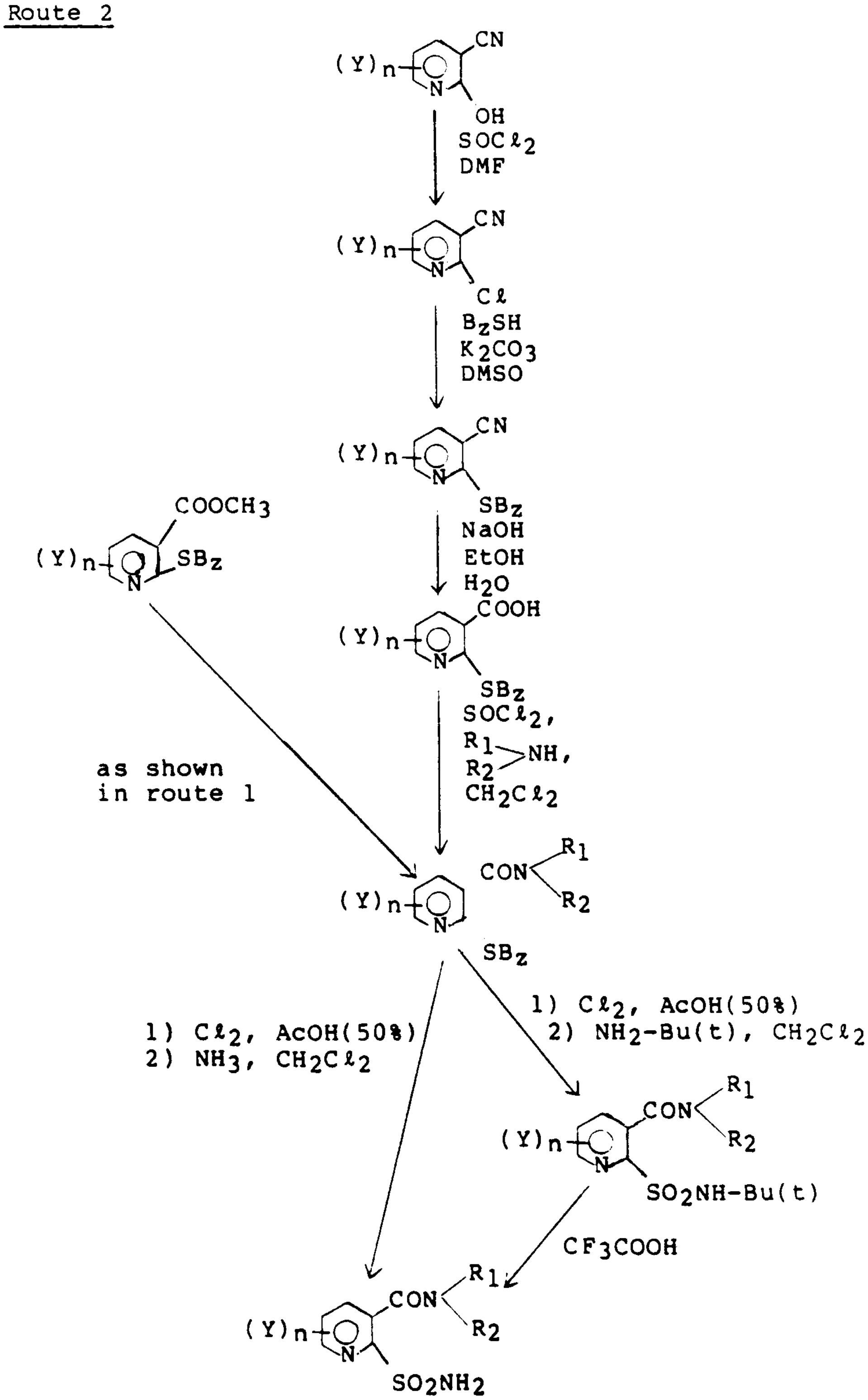
$SOCl_2$
DMF
$B_zSH$
$K_2CO_3$
DMSO
NaOH
EtOH
$H_2O$
$SOCl_2$,
$R_1R_2NH$,
$CH_2Cl_2$
as shown
in route 1
1) $Cl_2$, AcOH(50%)
2) $NH_3$, $CH_2Cl_2$
1) $Cl_2$, AcOH(50%)
2) $NH_2$-Bu(t), $CH_2Cl_2$
$CF_3COOH$

(II-1')

Note: EG is ethylene glycol, MeOH is methanol, EtOH is ethanol DMF is dimethylformamide, DMSO is dimethylsulfoxide, $B_z$ is a benzyl group, -Bu(t) is a tertiary butyl group, and AcOH is acetic acid.

(2) It is shown that if Y in General Formula (II-1') is an alkyl group substituted with one or more fluorine atoms, the product can also be prepared in the process indicated by route 3 below.

Route 3

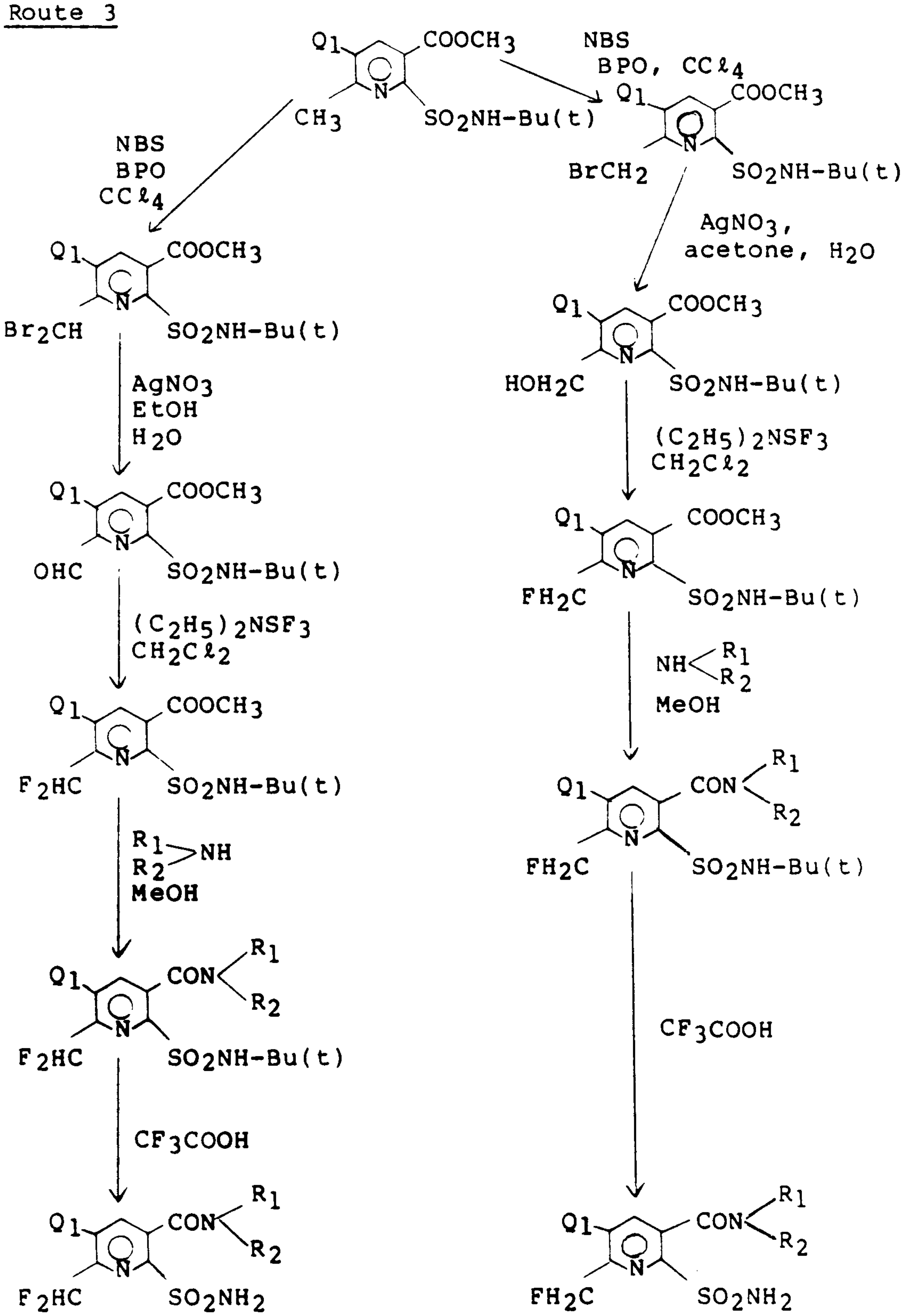
NBS
BPO, CCℓ4
NBS
BPO
CCℓ4
AgNO3,
acetone, H2O
AgNO3
EtOH
H2O
(C2H5)2NSF3
CH2Cℓ2
(C2H5)2NSF3
CH2Cℓ2
MeOH
MeOH
CF3COOH
CF3COOH

Note: Starting material 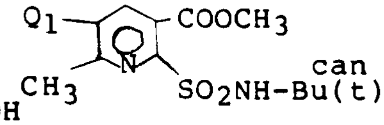 can be prepared using 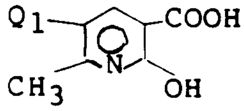 according to the processes in routes 1 and 2. $Q_1$ is a hydrogen or halogen atom, NBS is N-bromosuccinimide, and BPO is benzoylperoxide. Other abbreviations and symbols have the same meanings as those described above.

(3) It is shown that if Y in General Formula (II-1') is ($C_1$-$C_6$)alkoxy, halo($C_1$-$C_6$)alkoxy, alkylthio or halo($C_1$-$C_6$)alkylthio group, the product can also be prepared by the process in route 4 below.

Route 4

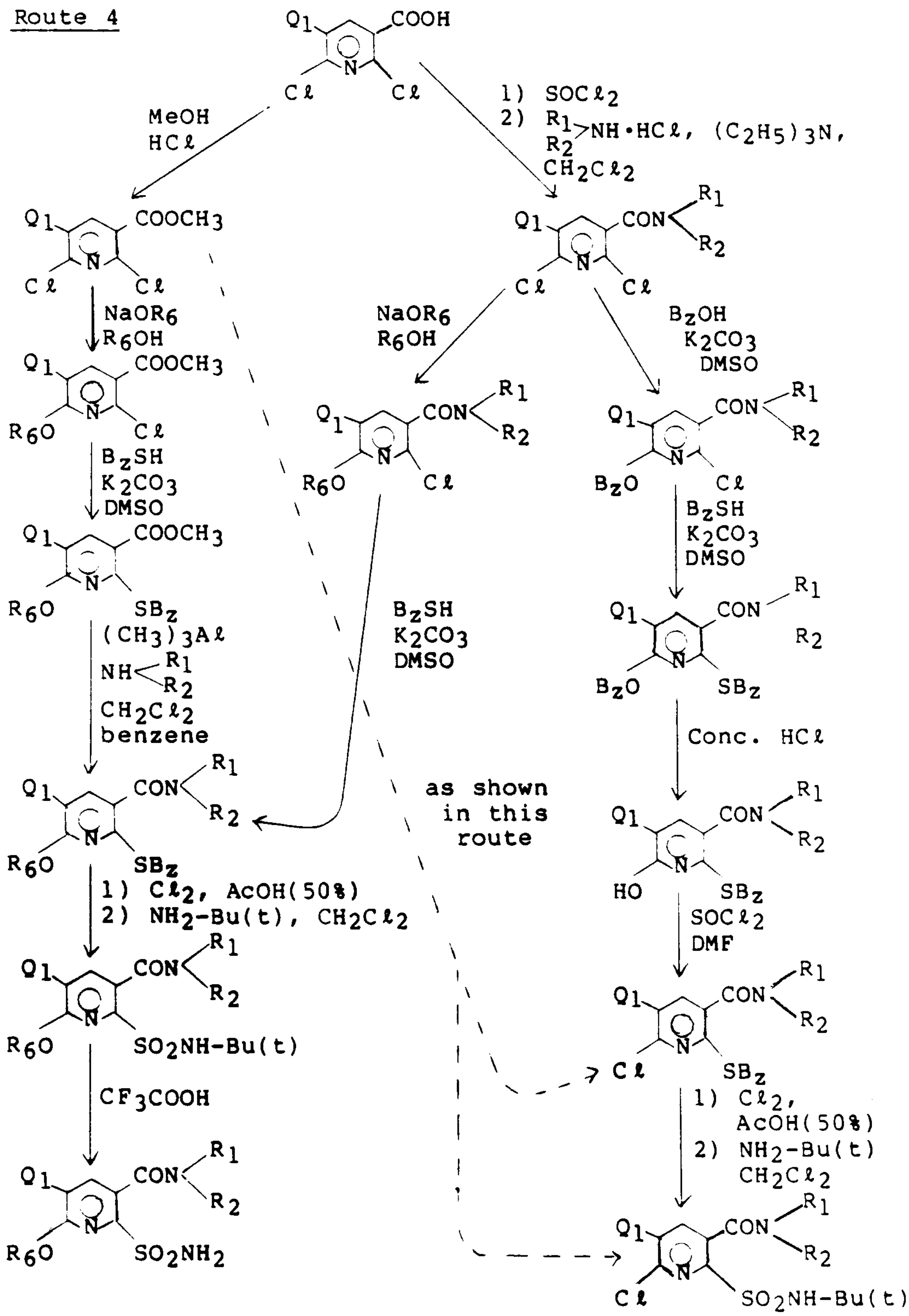


(continued to page 14)

(continued from page 13)

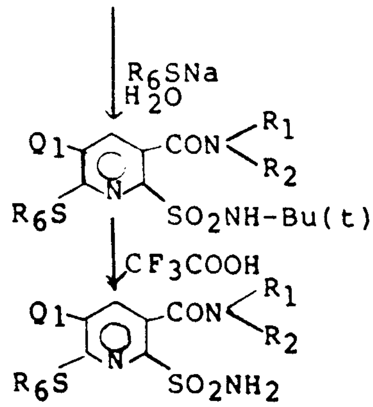


Note: $R_6$ is a $(C_1-C_6)$ alkyl or halo $(C_1-C_6)$ alkyl group. Other abbreviations and symbols have the same meanings as those described above.

(4) It is shown that if Y in General Formula (II-1') is $(C_1-C_6)$alkoxymethyl or halo$(C_1-C_6)$alkoxymethyl group, the product can also be prepared by the process in route 5 below.

Route 5

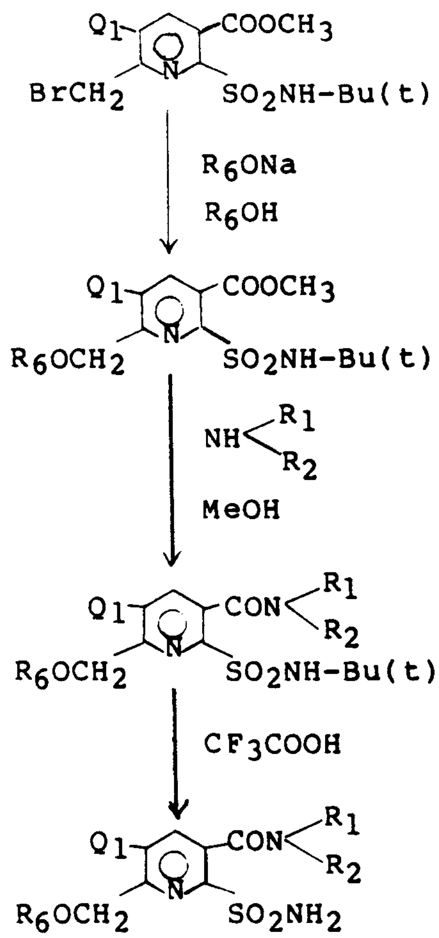


Note: Abbreviations and symbols have the same meanings as those described above.

(5) It is shown that if Y in General Formula (II-1') is an

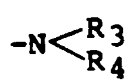

group, the product can also be prepared by the process in route 6 below.

Route 6

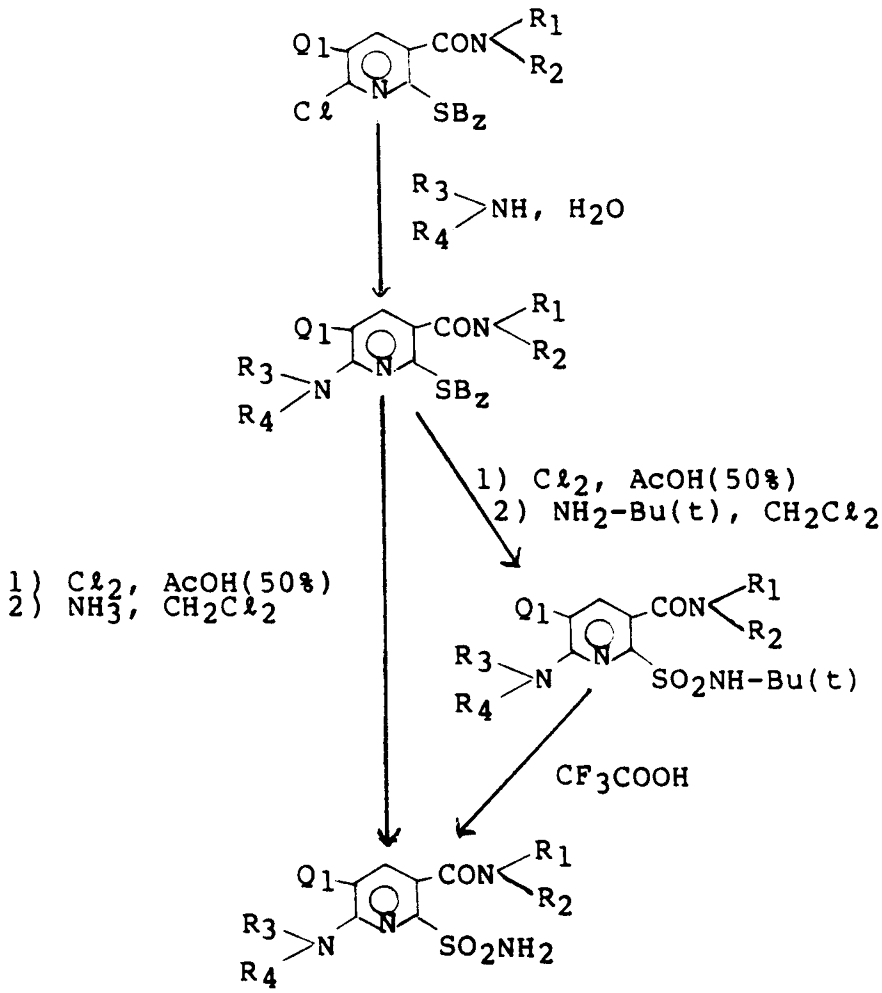


Note: Abbreviations and symbols have the same meanings as those described above.

(6) It is shown that if Y in General Formula (II-1') is a halogen atom, the product can also be prepared by the process in route 7 below.

Route 7

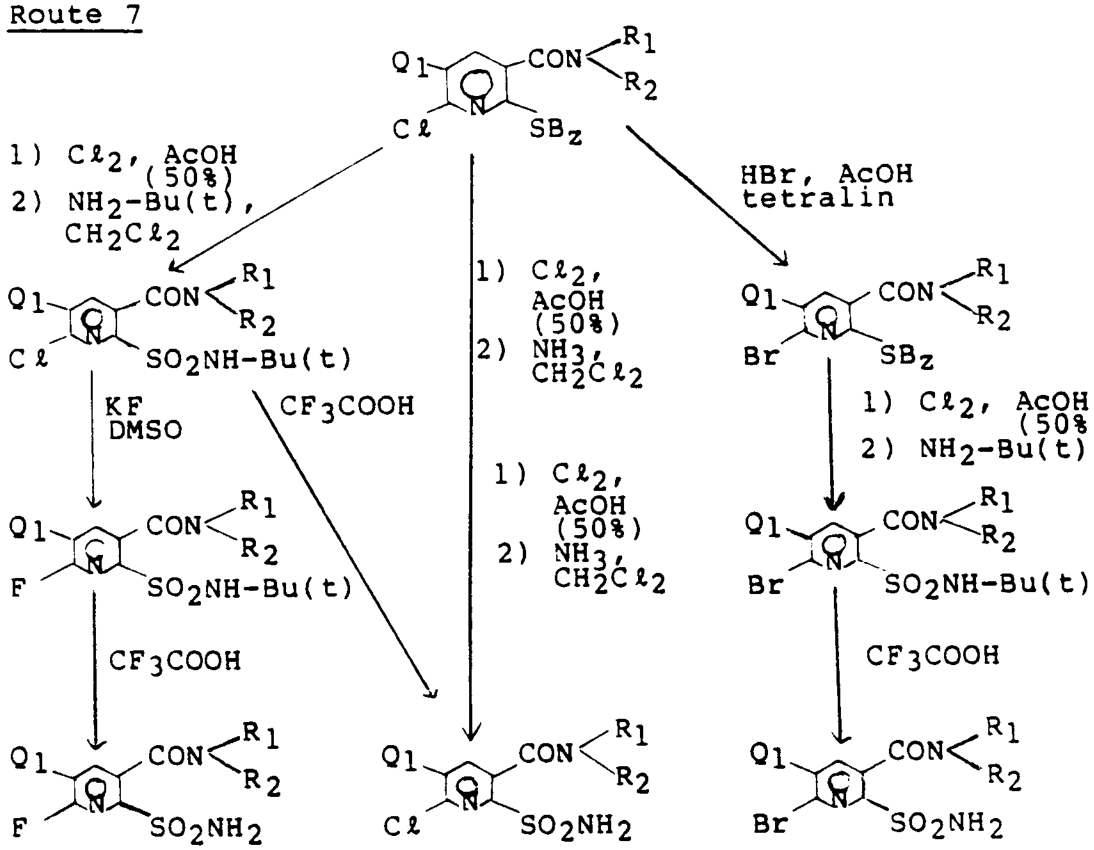


Note: Abbreviations and symbols have the same meanings as those described above.

(7) It is shown that compounds represented by General Formulas (II-2) to (II-4) can be prepared from compounds represented by General Formula (II-1) by the process in route 8 below.

Route 8

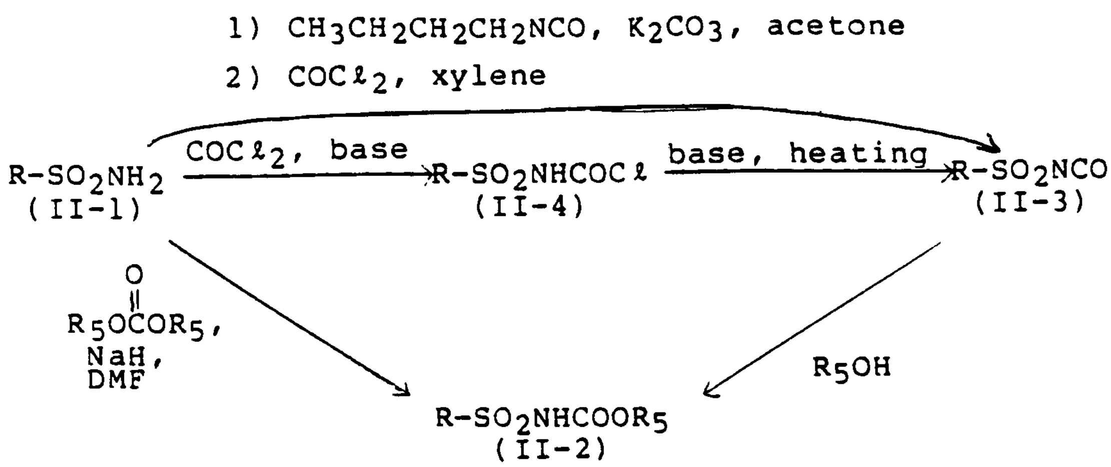


The reaction conditions for preparing and starting compound, such as reaction temperature, reaction time, a solvent which is optionally used, an alkaline substance, etc., can be suitably selected from those of the conventional analogous reactions.

According to a second aspect of the present invention, there is provided a herbicidal composition essentially consisting of a herbicidally effective amount of a compound or a salt thereof according to said one aspect of the present invention, and an agriculturally acceptable adjuvant.

According to a third aspect of the present invention, there is provided a process for producing a substituted pyridinesulfonamide compound represented by the following general formula:

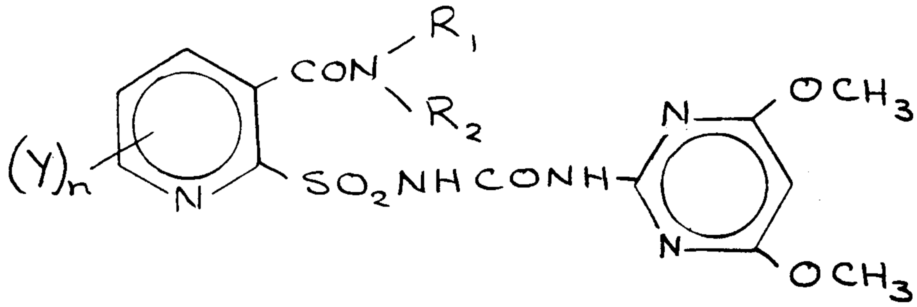

wherein each of $R_1$ and $R_2$ independently represents a hydrogen atom, a $(C_1\text{-}C_6)$alkyl group, a halo$(C_1\text{-}C_6)$-alkyl group, a $(C_2\text{-}C_6)$alkenyl group, a $(C_2\text{-}C_6)$alkynyl group, a $(C_1\text{-}C_6)$alkoxy group, a halo$(C_1\text{-}C_6)$alkoxy group, a $(C_1\text{-}C_6)$alkoxy$(C_1\text{-}C_6)$alkyl group, a halo$(C_1\text{-}C_6)$alkoxy$(C_1\text{-}C_6)$alkyl group, a $(C_3\text{-}C_6)$cycloalkyl group, a halo$(C_3\text{-}C_6)$cycloalkyl group, a $(C_1\text{-}C_6)$alkoxycarbonyl group, a halo$(C_1\text{-}C_6)$alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1\text{-}C_6)$alkyl group, a halo$(C_1\text{-}C_6)$alkyl group, a $(C_1\text{-}C_6)$alkoxy group, a halo$(C_1\text{-}C_6)$alkoxy group, a $(C_1\text{-}C_6)$alkylthio group, a halo$(C_1\text{-}C_6)$alkylthio group, a $(C_1\text{-}C_6)$alkoxy$(C_1\text{-}C_6)$alkyl group, a halo$(C_1\text{-}C_6)$alkoxy$(C_1\text{-}C_6)$alkyl group, or an

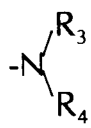

group (wherein each of $R_3$ and $R_4$ represents a hydrogen atom or a $(C_1\text{-}C_6)$alkyl group); and $\underline{n}$ represents 0 or an integer of 1 or 2; with the proviso that when $\underline{n}$ is 0, $R_1$ and $R_2$ are not both methyl groups; or a salt of such a compound;

said process comprising reacting a pyridine compound represented by the following general formula:

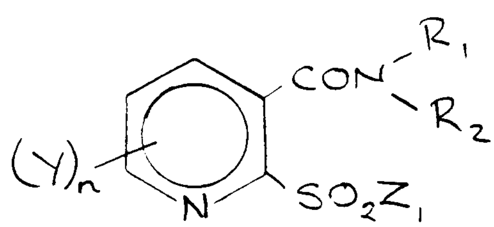

wherein $R_1$ and $R_2$ are as defined above and $Z_1$ represents an $-NH_2$ group, an -NCO group, an -NHCOCl group or an $-NHCOOR_5$ group (wherein $R_5$ represents an alkyl group or an aryl group), with a pyrimidine compound represented by the following general formula:

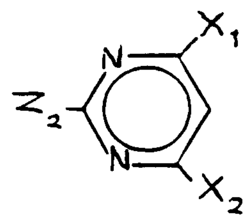

wherein each of $X_1$ and $X_2$ independently represents a halogen atom or a methoxy group, and $Z_2$ represents an $-NH_2$ group, an -NCO group, an -NHCOCl group or an $-NHCOOR_5$ group (wherein $R_5$ is as defined above), provided that when $Z_1$ represents the $-NH_2$ group, $Z_2$ represents the -NCO group, the -NHCOCl group or the $-NHCOOR_5$ group, and that when $Z_2$ represents the $-NH_2$ group, $Z_1$ represents the -NCO group, the -NHCOCl group or the $-NHCOOR_5$ group; then performing methoxylation when $X_1$ and/or $X_2$ represents a halogen atom; and, if desired, performing a salt formation treatment;
with the further provisos that, when (a) $R_1$ and $R_2$ are both $(C_1-C_3)$alkyl or $R_1$ is hydrogen and $R_2$ is $(C_1-C_3)$alkyl, $n$ is 1, and Y is Cl, F, Br, $CH_3$, $OCH_3$, $CF_3$ or $OCF_2H$, or when (b) $R_1$ is a hydrogen atom or a $(C_1-C_3)$alkyl group and $R_2$ is a $(C_1-C_2)$alkoxy group, $n$ is 1 and Y is $CF_3$ or $OCF_2H$, then $Z_1$ is not $-NH_2$ or $-NHCOOC_6H_5$ when $Z_2$ is $-NHCOOC_6H_5$ or $-NH_2$, respectively.

According to a fourth aspect of the present invention, there is provided a pyridine compound as an intermediate represented by the following general formula:

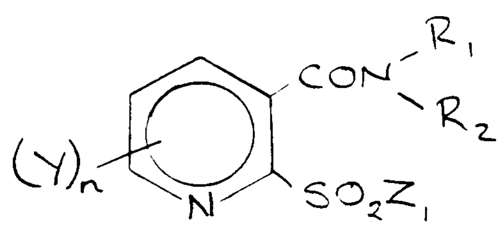

wherein each of $R_1$ and $R_2$ independently represents a hydrogen atom, a $(C_1-C_6)$alkyl group, a halo$(C_1-C_6)$-alkyl group, a $(C_2-C_6)$alkenyl group, a $(C_2-C_6)$alkynyl group, a $(C_1-C_6)$alkoxy group, a halo$(C_1-C_6)$alkoxy group, a $(C_1-C_6)$alkoxy$(C_1-C_6)$alkyl group, a halo$(C_1-C_6)$alkoxy$(C_1-C_6)$alkyl group, a $(C_3-C_6)$cycloalkyl group, a halo$(C_3-C_6)$cycloalkyl group, a $(C_1-C_6)$alkoxycarbonyl group, a halo$(C_1-C_6)$alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1-C_6)$alkyl group, a halo$(C_1-C_6)$alkyl group, a $(C_1-C_6)$alkoxy group, a halo$(C_1-C_6)$alkoxy group, a $(C_1-C_6)$alkylthio group, a halo$(C_1-C_6)$alkylthio group, a $(C_1-C_6)$alkoxy$(C_1-C_6)$alkyl group, a halo$(C_1-C_6)$alkoxy$(C_1-C_6)$alkyl group, or an

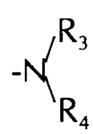

group (wherein each of $R_3$ and $R_4$ represents a hydrogen atom or a $(C_1-C_6)$alkyl group); $n$ represents 0 or an integer of 1 or 2; and $Z_1$ is an $NH_2$ group, an -NCO group, an -NHCOCl group or an $-NHCOOR_5$ group (wherein $R_5$ is an alkyl or an aryl group), with the provisos that (a) when $n$ is 0, $R_1$ and $R_2$ are not both methyl groups, (b) when $R_1$ and $R_2$ are both $(C_1-C_3)$alkyl or when $R_1$ is hydrogen and $R_2$ is $(C_1-C_3)$alkyl, $n$ is 1 and Y is Cl, F, Br, $CH_3$, $OCH_3$, $CF_3$ or $OCF_2H$, $Z_1$ is not $-NH_2$ or $-NHCOOC_6H_5$ and (c) when $R_1$ is a hydrogen atom or a $(C_1-C_3)$alkyl group and $R_2$ is a $(C_1-C_2)$alkoxy group, $n$ is 1 and Y is $CF_3$ or $OCF_2H$, $Z_1$ is not $-NH_2$ or $-NHCOOC_6H_5$.

Synthesis examples of intermediates of which some can be used to prepare compounds according to the present invention will be described below:

**Intermediate Synthesis Example 1 Synthesis of 2-aminosulfonyl-5-chloro-N,N-dimethyl-nicotinamide**

[I] 6.5g of 2,5-dichloronicotinic acid was mixed in 24.7 ml of thionyl chloride and reacted therewith under the reflux condition for 2 hours. After the reaction, excessive thionyl chloride was distilled and removed, and 39ml of methylene chloride was added to the mixture and 2.77g of dimethylamine hydrochloride salt was further added thereto. 8.60g of triethylamine was dropped within about an hour, and the resultant mixture was allowed to react at room temperature for about an hour.

After the completion of the reaction, the reaction product was poured into water for methylene chloride extraction. A methylene chloride layer was dried with anhydrous sodium sulfate, and methylene chloride was distilled off at reduced pressure. The resultant residue was purified by a silica gel column chromatography, thereby preparing 5.5 g of 2,5-dichloro-N,N-dimethylnicotinamide having a melting point of 120 to 122° C.

[II] A mixture of 3.0 g of 2,5-dichloro-N,N-dimethylnicotinamide prepared in process [I], 1.70 g of benzylmercaptane, and 5 mℓ of dimethylsulfoxide was added dropwise in a suspension of 1.89 g of anhydrous potassium carbonate in 28 mℓ of dimethylsulfoxide at 80° C for about an hour. The resultant mixture was reacted at 130 to 140° C for 30 minutes.

After the completion of the reaction, the reaction product was added to water for methylene chloride extraction. A methylene chloride layer was dried with anhydrous sodium sulfate, and methylene chloride was distilled off at reduced pressure. The resultant residue was purified by a silica gel column chromatography to prepare 2.18 g of oily 2-benzylthio-5-chloro-N,N-dimethylnicotineamide.

[III] Chlorine gas was introduced to 20 mℓ of a 50% acetic acid solution containing 2.36 g of 2-benzylthio-5-chloro-N,N-dimethylnicotinamide at 0 to 5° C. The reaction was interrupted when excessive chlorine appeared.

After the completion of the reaction, the reaction product was added to 150 g of ice and 200 mℓ of methylene chloride to separate a methylene chloride layer. The layer was washed with 300 mℓ of ice and water to cool the layer to 0° C. Subsequently, tert-butylamine was dropped and the mixture stirred until the temperature of the layer came to room temperature. The reaction solution was checked to be weakly alkaline and the reaction was finished.

After the completion of the reaction, the reaction product was added to water for methylene chloride extraction. The extracted methylene chloride was dried with anhydrous sodium sulfate, and methylene chloride was distilled off. The resultant residue was purified by a silica gel column chromatography to prepare 1.21 g of 2-tert-butylaminosulfonyl-5-chloro-N,N-dimethylnicotinamide having a melting point of 143 to 145° C.

[IV] 1.0 g of 2-tert-butylaminosulfonyl-5-chloro-N,N-dimethylnicotinamideprepared in process [III] was added to 10 mℓ of trifluoroacetic acid and reacted therewith under the reflux condition for about an hour.

After the completion of the reaction, trifluoroacetic acid was distilled from the reaction product at reduced pressure. The residue was then purified by a silica gel column chromatography to prepare 0.71 g of 2-aminosulfonyl-5-chloro-N,N-dimethylnicotinamide having a melting point of 155 to 157° C.

Intermediate Synthesis Example 2 Synthesis of 2-aminosulfonyl-6-chloro-N,N-dimethylnicotinamide

[I] A mixture of 7.0 g of 2,6-dichloro-N,N-dimethylnicotinamide (m.p. 62.5 to 65° C) prepared from 2,6-dichloronicotinic acid by following the same procedures as in process [I] of Intermediate Synthesis Example 1, 3.8 g of benzyl alcohol, and 20 mℓ of dimethylsulfoxide was dropped in a suspension of 6.6 g of anhydrous potassium carbonate in 50 mℓ of dimethylsulfoxide at 130 to 140° C for about an hour, and the resultant mixture was reacted at 150° C for about 2 hours.

After the completion of the reaction, the reaction product was added to water for methylene chloride extraction. A methylene chloride layer was dried with anhydrous sodium sulfate, and methylene chloride was distilled at reduced pressure. The resultant residue was purified by a silica gel column chromatography to prepare 6.0 g of oily 6-benzyloxy-2-chloro-N,N-dimethylnicontinamide.

[II] 0.69 g of 6-benzyloxy-2-benzylthio-N,N-dimethylnicotinamide (oily product) prepared by using 6-benzyloxy-2-chloro-N,N-dimethylnicotinamide by following the same procedures as in process [II] of Intermediate Synthesis Example 1 was mixed in 6 cc of concentrated hydrochloric acid, and the resultant mixture was stirred and reacted at room temperature for about 15 hours.

After the completion of the reaction, the reacted solution was added to water to extract the desired product with methylene chloride. A methylene chloride layer was dried with anhydrous sodium sulfate, and methylene chloride was distilled off at reduced pressure. The resultant residue was purified by a

silica gel column chromatography to prepare 0.41 g of 2-benzylthio-N,N-dimethyl-6-hydroxynicotinamide having a melting point of 52 to 60 ° C.

[III] 1.0 g of 2-benzylthio-N,N-dimethyl-6-hydroxynicotinamide prepared in the previous process [II] and 0.3 mℓ of dimethylformamide were added to 5 mℓ of thionyl chloride, and the resultant mixture was reacted under the reflux condition for about an hour.

After the completion of the reaction, the reaction product was added to water to extract the desired product with methylene chloride. A methylene chloride layer was washed with water and dried with anhydrous sodium sulfate. Methylene chloride was then distilled off at reduced pressure, and the resultant residue was purified by a silica gel column chromatography to prepare 0.45 g of oily 2-benzylthio-6-chloro-N,N-dimethylnicotinamide.

[IV] By using the above product and following the same procedures as in processes [III] and [IV] of Example 1, 2-aminosulfonyl-6-chloro-N,N-dimethylnicotinamide having a melting point of 171 to 173 ° C was prepared.

Intermediate Synthesis Example 3 Synthesis of 2-aminosulfonyl-N,N-dimethyl-6-ethoxynicotinamide

[I] 0.231 g of metal sodium was added to 50 mℓ of anhydrous ethanol to prepare an ethanol solution of sodium ethoxide. 2.0 g of 2,6-dichloro-N,N-dimethyl-nicotinamide prepared following the same procedures as in process [I] of Intermediate Synthesis Example 1 was added to the solution and the mixture was reacted under the reflux condition for about one hour. The reacted solution was added to water for methylene chloride extraction. A methylene chloride layer was dried with anhydrous sodium sulfate, and methylene chloride was distilled off at reduced pressure. The residue was purified by a silica gel column chromatography to prepare 1.95 g of oily 2-chloro-N,N-dimethyl-6-ethoxynicotinamide.

[II] By using the above product and following the same procedures as in processes [II], [III] and [IV] of Intermediate Synthesis Example 1, 2-aminosulfonyl-N,N-dimethyl-6-ethoxynicotinamide having a melting point of 150.5 to 151.5 ° C was prepared.

Intermediate Synthesis Example 4 Synthesis of 2-aminosulfonyl-N,N-dimethyl-6-fluoronicotinamide

[I] 0.302 g of 2-tert-butylaminosulfonyl-6-chloro-N,N-dimethylnicotinamide was added to 10 mℓ of a dimethylsulfoxide slurry solution containing 0.116 g of spray-dried potassium fluoride, and the resultant mixture was stirred and reacted at 150 ° C for about 3 hours.

After the completion of the reaction, the reacted solution was added to 100 mℓ of water to extract with methylene chloride. The extracted substance was repeatedly washed with water and dried with anhydrous sodium sulfate. Methylene chloride was distilled off at reduced pressure and the residue was purified by a silica gel column chromatography to prepare 0.204 g of 2-tert-butylaminosulfonyl-N,N-dimethyl-6-fluoronicotinamide.

[II] By using the product and following the same procedures as in process [IV] of Intermediate Synthesis Example 1, 2-aminosulfonyl-N,N-dimethyl-6-fluoronicotinamide having a melting point of 164 to 165 ° C was prepared.

Intermediate Synthesis Example 5 Synthesis of 2-aminosulfonyl-N-methylnicotinamide

[1] 250 mℓ of dried methanol was saturated with hydrochloric acid gas, and 250 mℓ of methanol and 30 g of 2-mercaptonicotinic acid were added thereto. The resultant solution was reacted through overnight at room temperature.

After the reaction, the reacted solution was converted into a weak alkaline solution with ammonia gas, and methanol was distilled at reduced pressure. Subsequently, the crystal obtained was washed with water and filtered out. The filtered crystal was dried at reduced pressure to prepare 24 g of methyl 2-mercaptonicotinate having a melting point of 136 to 140 ° C.

[II] 10 g of methyl 2-mercaptonicotinate prepared in process [I] was added to a suspension prepared by diluting 50 mℓ of acetic acid with 50 mℓ of water. Chlorine gas was introduced to the reaction system at a temperature of 0 ° C or less, and the reaction was finished when excessive chlorine appeared.

After the reaction, the resultant solution was added to a mixture of 150 g of ice and 500 mℓ of methylene chloride to separate a methylene chloride layer. The layer was washed with 500 mℓ of ice and water and cooled to 0 ° C. Subsequently, 12.9 g of tert-butylamine was dropped in the above solution and stirred until the resultant solution reached room temperature. After stirring, the reacted solution was added to 500 mℓ of water, and was extracted with methylene chloride. After dried with anhydrous

sodium sulfate and methylene chloride was distilled to prepare 13 g of a crude crystal. The crystal was recrystallized with methylene chloride and normal hexane to prepare methyl 2-tert-butylaminosulfonylnicotinate having a melting point of 169 to 171 ° C.

[III] 1.0 of this product was added to 20 mℓ of anhydrous methanol absorbed excess of methylamine, and stirred for 15 hours.

After the completion of the reaction, the reaction product was introduced in water to extract with methylene chloride. After the extraction, it was dried with anhydrous sodium sulfate and methylene chloride was distilled off under reduced pressure. 0.73 g of 2-tert-butylaminosulfonyl-N-methylnicotinamide having a melting point of 189 to 192 ° C was obtained.

[IV] By using the product and following the same procedures as in process [IV] of Intermediate Synthesis Example 1, 2-aminosulfonyl-N-methylnicotinamide having a melting point of 207 to 209.5 ° C was prepared.

Intermediate Synthesis Example 6 Synthesis of 2-aminosulfonyl-N,N-dimethylnicotinamide

[Method A]

[I] In a nitrogen atmosphere at 10 ° C or less, 10 mℓ of normal hexane containing 19% trimethylaluminum was added to a solution prepared by dissolving 1.3 mℓ of dimethylamine in 10 mℓ of anhydrous benzene. The resultant mixture was reacted until its temperature reached room temperature. A solution prepared by dissolving methyl 2-tert-butylaminosulfonylnicotinate in 40 mℓ of benzene and 20 mℓ of methylene chloride was dropped in the reacted solution. Subsequently, the resultant mixture was stirred under the reflux condition for about 9 hours.

After the completion of the reaction, the reacted solution was introduced to a dilute hydrochloric acid solution to extract with methylene chloride. The extracted substance was dried with anhydrous sodium sulfate, methylene chloride was distilled off at reduced pressure, and the residue was purified by a silica gel column chromatography to prepare 1.0 g of 2-tert-butylaminosulfonyl-N,N-dimethylnicotinamide.

[II] By using the above product and following the same procedures as in process [IV] of Intermediate Synthesis Example 1, 2-aminosulfonyl-N,N-dimethylnicotinamide having a melting point of 209 to 211 ° C was prepared.

[Method B]

Chlorine gas was introduced to 50 mℓ of 50% acetic acid solution of 2.7 g of 2-benzylthio-N,N-dimethylnicotinamide until excessive chlorine was appeared.

After the completion of the reaction, the reacted mixture was added to 200 mℓ of ice and water to extract with 60 mℓ of methylene chloride. Subsequently, a methylene chloride layer was washed with 100 mℓ of ice and water and cooled to 0 ° C. Ammonia gas was introduced to the solution until it was converted into an alkaline solution. After methylene chloride was distilled off, 15 mℓ of water were added to the residue, and the resultant mixture was stirred. The resultant crystal was filtered out and washed with a small amount of water. The crystal was then dried at reduced pressure to prepare 1.27 g of 2-aminosulfonyl-N,N-dimethyl-nicotinamide having a melting point of 209 to 211 ° C.

Intermediate Synthesis Example 7 Synthesis of 2-aminosulfonyl-N-ethyl-N-methylnicotinamide

[I] A mixture of 30 g of 2-chloronicotinic acid and thionyl chloride was reacted under a reflux condition for 4 hours. After excessive thionyl chloride was distilled off from the reacted solution at reduced pressure, 500 mℓ of methylene chloride was added thereto, and methylamine gas was supplied at room temperature until the reacted solution became a weakly alkaline solution. The resultant methylamine hydrochloride was filtered, and the filtered solution was condensed. The precipitated crystal was recrystallized by normal hexane and methylene chloride to prepare 27 g of 2-chloro-N-methylnicotinamide having a melting point of 106 to 107 ° C.

[II] 10 mℓ of dimethylformamide containing 5 g of 2-chloro-N-methylnicotinamide prepared following the same procedures as in process [I] of this example were dropped at 0 to 5 ° C in a suspension prepared by adding 1.17 g of 60% sodium hydride to 50 mℓ of dimethylformamide. The resultant mixture was reacted at 0 to 5 ° C for about 30 minutes, and 3.5 g of ethylbromide was dropped at 0 ° C. The resultant solution was reacted at room temperature for 2.5 hours, and the reacted solution was added to water for methylene chloride extraction. The extracted substance was washed with water and dried with anhydrous

sodium sulfate. Methylene chloride was then distilled off at reduced pressure and the residue was purified by a silica gel column chromatography, thereby preparing 5.7 g of oily 2-chloro-N-ethyl-N-methylnicotinamide.

[III] By using this product and following the same procedures as in processes [II] to [IV] of Intermediate Synthesis Example 1, 2-aminosulfonyl-N-ethyl-N-methylnicotinamide having a melting point of 202 to 203 °C was obtained.

Intermediate Synthesis Example 8 Synthesis of 2-aminosulfonyl-N,N-dimethyl-6-methylnicotinamide

[I] One droplet of dimethylformamide was added to a mixture of 1.35 g of 2-hydroxy-6-methylnicotinic acid and 15 mℓ of thionyl chloride, and the resultant solution was reacted under the reflux condition for about 2 hours. Excessive thionyl chloride was distilled off at reduced pressure, and 50 mℓ of methylene chloride was added to the residue. The resultant mixture was dropped in a mixture of 30 mℓ of 30% dimethylamine aqueous solution and 50 mℓ of methylene chloride, and the resultant solution was reacted at room temperature for 15 minutes.

After the completion of the reaction, the reacted mixture was added to water for methylene chloride extraction. A methylene chloride layer was washed with water and dried with anhydrous sodium sulfate. Methylene chloride was distilled off at reduced pressure and the residue was purified by a silica gel column chromatography to prepare 1.57 g of 2-chloro-3-N,N-dimethylaminocarbonyl-6-methylpyridine (m.p. 66 ~ 67.5 °C)

[II] By using this product and following the same procedures as in processes [II] to [IV] of Intermediate Synthesis Example 1, 2-aminosulfonyl-N,N-dimethyl-6-methylnicotinamide having a melting point of 188.5 to 190.5 °C was obtained.

Intermediate Synthesis Example 9 Synthesis of 2-aminosulfonyl-N,N-dimethyl-6-methoxynicotinamide

[I] 33 g of 2,6-dichloronicotinic acid and 100 mℓ of thionyl chloride were mixed and reacted in the reflux condition for about 3 hours.

After the completion of the reaction, excessive thionyl chloride was distilled off at reduced pressure, and 500 mℓ of methylene chloride was added thereto. Anhydrous methanol was dropped in the mixture in the range of room temperature to 40 °C. After dropping, the resultant mixture was reacted in the reflux condition for about an hour.

After the completion of the reaction, the reacted solution was added to 500 mℓ of water and separated with methylene chloride. After the residue was washed with water and dried with anhydrous sodium sulfate, methylene chloride was distilled off at reduced pressure. The residue was purified by a silica gel column chromatography to prepare 27 g of methyl 2,6-dichloronicotinate having a melting point of 45.5 to 48.0 °C.

[II] By using this product and following the same procedures as in processes [I] of Intermediate Synthesis Example 3, [II] of Intermediate Synthesis Example 1, [method A] [I] of Intermediate Synthesis Example 6 and [III] to [IV] of Intermediate Synthesis Example 1, 2-aminosulfonyl-N,N-dimethyl-6-methoxynicotineamide was obtained.

Intermediate Synthesis Example 10 Synthesis of 2-aminosulfonyl-6-difluoromethyl-N,N-dimethylnicotineamide

[I] A mixture of 11.5 g of methyl 2-tert-butylaminosulfonyl-6-methylnicotinate prepared following the same procedures as in processes [I] and [II] of Intermediate Synthesis Example 5, 186 mℓ of carbon tetrachloride, 14.84 g of N-bromosuccinimide, and 0.68 g of benzoyl peroxide were heated and refluxed for three hours.

After the completion of the reaction, the reacted mixture was filtered, and the filtered solution was condensed and purified by a silica gel column chromatography to prepare 4.5 g of methyl 2-tert-butylaminosulfonyl-6-dibromomethylnicotinate.

[II] 3.7 g of silver nitrate was added to a mixture of 4.4 g of the above product, 35 mℓ of ethanol, and 29 mℓ of water, and the resultant mixture was heated and refluxed for 3 hours.

After the completion of the reaction, the reacted mixture was added to water for methylene chloride extraction. The extracted substance was washed with water and dried with anhydrous sodium sulfate, and the solvent was distilled off therefrom. The residue was purified by a silica gel column chlormatography to prepare 2.92 g of methyl 2-tert-butylaminosulfonyl-6-formylnicotinate.

[III] 5 mℓ of methylene chloride solution of 0.62 g of sulfur diethylaminotrifluoride was added to 19 mℓ of methylene chloride solution of 0.48 g of methyl 2-tert-butylaminosulfonyl-6-formylnicotinate, and the mixture was stirred for an hour at room temperature.

After the completion of the reaction, the reacted mixture was added to water, extracted with methylene chloride, washed with water, and dried with anhydrous sodium sulfate. Thereafter the solvent was distilled off. The residue was purified by a silica gel column chromatography to prepare 0.25 g of methyl 2-tert-butylaminosulfonyl-6-difluoromethylnicotinate.

[IV] A mixture of 0.50 g of the above product and 10 mℓ of anhydrous methanol saturated with dimethylamine, was allowed overnight at room temperature.

After the completion of the reaction, the solvent was distilled off from the reaction mixture, and the residue was purified with silica gel column chromatography to obtain 0.15 g of 2-tert-butylaminosulfonyl-6-difluoromethyl-N,N-dimethylnicotinamide.

[V] By using the above product and following the same procedures as in process [IV] of Intermediate Synthesis Example 1, 2-aminosulfonyl-6-difluoromethyl-N,N-dimethylnicotinamide was obtained.

Intermediate Synthesis Example 11 Synthesis of 2-aminosulfonyl-6-bromo-N,N-dimethylnicotinamide

[I] 5 mℓ of acetic acid was added to 1.2 g of 2-benzylthio-6-chloro-N,N-dimethyl-nicotinamide, and the resultant mixture was heated to 70 °C. Hydrogen bromide gas was introduced to this mixture for 30 minutes and the mixture was reacted for another 30 minutes.

After the completion of the reaction, the reacted mixture was cooled and added to ice and water, extracted with methylene chloride, washed with sodium bicarbonate solution and then with water, and dried with anhydrous sodium sulfate. Methylene chloride was distilled and the residue was purified by a silica gel column chromatography to prepare 1.0 g of 2-benzylthio-6-bromo-N,N-dimethylnicotinamide having a melting point of 105 to 106 °C.

[II] By using this product and following the same procedures as in processes [III] and [IV] of Intermediate Synthesis Example 1, 2-aminosulfonyl-6-bromo-N,N-dimethylnicotineamide having a melting point of 154 to 156 °C was prepared.

Typical examples of the compounds represented by general formula (II-1′) are summarized in Table 1.

Table 1

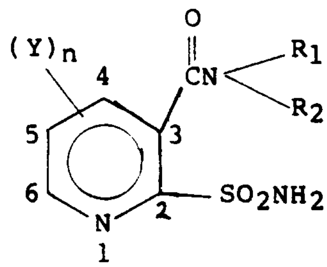

... [II-1']

| Intermediate Compound No. | (Y)n | 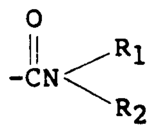 | | | Position of Sulfonamide Group | Melting Point (°C) |
|---|---|---|---|---|---|---|
| | | Position | $R_1$ | $R_2$ | | |
| 1 | H | 3 | $CH_3$ | phenyl | 2 | 235~238 |
| 2 | " | " | " | $CH_2CF_3$ | " | - |
| 3 | " | " | " | $OCH_3$ | " | - |
| | | | | | | |
| 4 | H | " | " | $COOCH_3$ | " | 168~170 |
| 5 | " | " | " | $C_3H_7(n)$ | " | - |
| 6 | $4,6(CH_3)_2$ | " | H | $CH_3$ | " | 220~221 |
| 7 | $6\text{-}OCH_2CH_3$ | " | $CH_3$ | " | " | 150.5~151.5 |
| 8 | H | " | H | " | " | 207~209.5 |
| 9 | " | " | " | $C_2H_5$ | " | 164~172 |

(Table 1, continued from page 33)

| | | | | | | |
|---|---|---|---|---|---|---|
| 10 | H | 3 | H | $C_3H_7$(iso) | 2 | 152~153 |
| | | | | | | |
| 11 | H | " | " | $C_2H_5$ | " | 202~203 |
| 12 | " | " | $C_2H_5$ | " | " | - |
| | | | | | | |
| 13 | 6-$C_2H_5$ | " | " | " | " | 153~154 |
| 14 | H | " | H | $CH_2CH=CH_2$ | " | 152~153.5 |
| 15 | " | " | " | $CH_2CF_3$ | " | 190~196 |
| 16 | " | " | " | phenyl | " | 186~189 |
| 17 | 4,6($CH_3$)$_2$ | " | $CH_3$ | $CH_3$ | " | 199~202 |
| | | | | | | |
| 18 | H | " | " | $CH_2CH_2OCH_3$ | " | 170.5~172.5 |
| 19 | 6-$CH_2OCH_3$ | " | $CH_3$ | $CH_3$ | " | - |
| 20 | 5-Cl,6-$OCH_3$ | " | " | " | " | 212~214 |
| 21 | H | " | H | cyclopropyl | " | 180~182 |
| 22 | " | " | " | $CH_2C\equiv CH$ | " | 203~205 |
| 23 | " | " | -($CH_2$)$_4$- | | " | 206~209 |

(Table 1, continued from page 34)

| | | | | | | |
|---|---|---|---|---|---|---|
| | | | | | | |
| | | | | | | |
| | | | | | | |
| 24 | 6-$C_2H_5$ | " | " | " | " | 153～154 |
| | | | | | | |
| 25 | H | " | -$CH_2CH_2OCH_2CH_2$- | | " | 236～238 |
| 26 | 6-$N(CH_3)_2$ | " | $CH_3$ | $CH_3$ | " | - |
| 27 | 6-$SCH_3$ | " | " | " | " | 195.5～197 |
| 28 | 6-$NHCH_3$ | " | " | " | " | 212～213 |
| 29 | 6-$CH_2F$ | " | " | " | " | - |
| 30 | 6-$CHF_2$ | " | " | " | " | - |
| 31 | H | " | " | 2,4-difluoro-phenyl | " | 232～237 |
| 32 | " | " | H | $(CH_2)_3CH_3$ | " | - |
| 33 | 6-$CH_2OCH_2CF_3$ | " | $CH_3$ | $CH_3$ | " | - |
| | | | | | | |
| | | | | | | |
| 34 | 6-$OCH_2CF_3$ | 3 | " | " | 2 | - |

(Table 1, continued from page 35)

| | | | | | | |
|---|---|---|---|---|---|---|
| 35 | 6-$C_3H_7$ (iso) | 3 | $CH_3$ | $CH_3$ | 2 | - |
| 36 | 5-$CHF_2$ | " | " | " | " | - |
| 37 | 6-$CHF_2$ | " | " | " | " | - |

Further synthesis examples will be described below.

Synthesis Example No. 1 Synthesis of 5-chloro-N-[(4,6-dimethoxypyrimidin-2-yl)aminocarbonyl]-3-dimethylaminocarbonyl-2-pyridinesulfonamide (compound No. 9)

0.19 g of 1,8-diazabicyclo[5•4•0]-7-undecene was added to a suspension prepared by adding 0.30 g of 2-aminosulfonyl-5-chloro-N,N-dimethylnicotinamide and 0.35 g of 2-phenoxycarbonylamino-4,6-dimethoxypyrimidine to 10 mℓ of anhydrous acetonitrile, and the resultant mixture was reacted at room temperature for 45 minutes.

After the completion of the reaction, the reacted mixture was added to water to filter an insoluble substance. The filtered solution was converted by concentrated hydrochloric acid to a weakly acidic solution and was extracted with methylene chloride. Subsequently, the solution was dried with anhydrous sodium sulfate, and the solvent was distilled off, thereby obtaining 0.26 g of the desired compound having a melting point of 152 to 155 °C.

Synthesis Example Nos. 2 to 7

The same procedures as in Synthesis Example 1 were followed, and results in Table 2 were obtained.

Table 2

| Synthesis Example No. | Reagent: Starting Compound: pyridines (g) | Reagent: Starting Compound: Pyrimidines (g) | Reagent: Anhydrous Acetonitrile (mℓ) | Reagent: 1,8-diazabicyclo[5·4·0]-7-undecene (g) | Reaction conditions: Temperature (°C) | Reaction conditions: Time (H) | Product (g) | Melting point (°C) |
|---|---|---|---|---|---|---|---|---|
| | | | | | | | | |
| 2 | Intermediate No. 7 0.100 | " 0.110 | 10 | 0.060 | " | 1 | Compound No. 15 0.140 | 208~209 |
| 3 | Intermediate No. 13 0.275 | " 0.278 | 10 | 0.167 | " | 0.75 | Compound No. 16 0.319 | 193~194.5 |
| 4 | Intermediate No.19 0.130 | " 0.14 | 10 | 0.080 | " | 1 | Compound No. 17 0.150 | 183~185 |

| Synthesis Example No. | Reagent | | | | Reaction conditions | | Product (g) | Melting point (°C) |
|---|---|---|---|---|---|---|---|---|
| | Starting Compound | | Anhydrous Acetonitrile (mℓ) | 1,8-diazabicyclo[5·4·0]-7-undecene (g) | Temperature (°C) | Time (H) | | |
| | pyridines (g) | Pyrimidines (g) | | | | | | |
| 5 | Intermediate No. 8<br>0.300 | "<br>0.420 | 10 | 0.230 | " | 2 | Compound No. 19<br>0.370 | 147~149.5 |
| 6 | Intermediate No. 11<br>0.300 | "<br>0.360 | 10 | 0.210 | " | 1 | Compound No. 25<br>0.470 | 170.5~172 |
| 7 | Intermediate No. 30<br>0.100 | "<br>0.110 | 10 | 0.600 | " | 1 | Compound No. 33<br>0.120 | 194~195 |

Note: Intermediate numbers in the column of pyridines and compound numbers in the column of products are based on itmes in Tables 1 and 3.

Synthesis Example No. 8 Synthesis of N-[(4,6-dimethoxypyrimidin-2-yl)aminocarbonyl]-3-dimethylaminocarbonyl-2-pyridinesulfonamide

Method 1

A mixture solution of 250 mg of 2-amino-4,6-dimethoxypyrimidine, 0.65 g of triethylamine, and 2.5 g of ethyl acetate was dropped in 6.3 g of an ethyl acetate solution containing 20% of phosgene at 15 °C, and the mixture was maintained at 15 °C and reacted for one hour. Subsequently, the mixture was heated to 90 °C on the oil bath, and excessive phosgene and ethyl acetate were distilled off. Then, a solution prepared by dissolving 300 mg of 2-aminosulfonyl-N,N-dimethylnicotinamide in 10 mℓ of acetonitrile was dropped in the above mixture, and 0.2 g of triethylamine was further dropped therein. The resultant mixture was reacted at room temperature for one hour.

After the completion of the reaction, the product was added to water and converted by hydrochloric acid into an acidic solution, and the precipitated crystal was filtered. The crystal was washed with water and dried to obtain 0.46 g of the desired compound.

Method 2

[I] 2.13 g of 2-aminosulfonyl-N,N-dimethylnicotinamide was added to 5 mℓ of dimethylformamide slurry containing 60% sodium hydride at -5 °C, and the resultant mixture was reacted for about one hour. 10 mℓ of dimethylformamide solution of 2.14 g of diphenylcarbonate was dropped in the above solution at -5 °C. The solution was heated to room temperature within about 30 minutes to complete the reaction. The reacted solution was added to water and washed with methylene chloride. The water layer was converted into an acidic solution by hydrochloric acid, thereby extracted with methylene chloride. The methylene chloride layer was dried with anhydrous sodium sulfate, and methylene chloride was distilled off at reduced pressure. The resultant crystal was recrystallized by ethyl acetate and hexane to prepare 0.91 g of N,N-dimethyl-2-phenoxycarbonylaminosulfonylnicotineamide having a melting point of 189 to 194 °C.

[II] 0.28 g of N,N-dimethyl-2-phenoxycarbonylaminosulfonylnicotinamide prepared by process [I] and 0.14 g of 2-amino-4-chloro-6-methoxypyrimidine were added to 8 mℓ of anhydrous dioxane, and the resultant mixture was reacted under the reflux condition for about 40 minutes.

After the completion of the reaction, the reacted mixture was added to 200 mℓ of water, and the resultant crystal was filtered to prepare 0.21 g of N-[(4-chloro-6-methoxypyrimidin-2-yl)aminocarbonyl]-3-dimethylaminocarbonyl-2-pyridinesulfonamide having a melting point of 157 to 158.5 °C.

[III] 18.3 mg of metal sodium was added to 7 mℓ of anhydrous methanol, and 0.11 g of N-[(4-chloro-6-methoxypyrimidin-2-yl)aminocarbonyl]-3-dimethylaminocarbonyl-2-pyridinesulfonamide prepared in process [II] was added thereto. The resultant mixture was heated and refluxed for 12 hours.

After the completion of the reaction, the reacted mixture was added to water and converted into an acidic solution by hydrochloric acid, and the precipitated crystal was filtered, washed with water, and dried to prepare 70 mg of the desired compound.

The typical compounds represented by the general formula [I] and salts thereof according to the present invention will be shown in Table 3.

Table 3

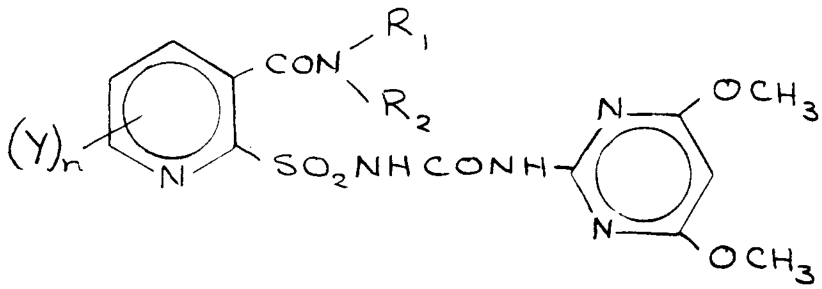

(I)

| Compound No. | $(Y)_n$ | | | | | | | Melting Point (°C) |
|---|---|---|---|---|---|---|---|---|
| | | | $R_1$ | $R_2$ | | | | |
| 1 | H | | H | $CH_2CH=CH_2$ | | | | 165.5~167.5 |
| 2 | " | | " | $CH_2CF_3$ | | | | 109~116 |
| 3 | " | | " | $CH_2CH_2OCH_3$ | | | | 144.5~146 |
| 4 | " | | " | cyclo-propyl | | | | 181~182 |
| 5 | " | | $-CH_2CH_2OCH_2CH_2-$ | | | | | 159~161 |
| 6 | " | | $CH_3$ | $OCH_3$ | | | | 179~182 |
| 7 | " | | " | phenyl | | | | 114~119 |

(Table 3, continued from page 43)

| | | | | |
|---|---|---|---|---|
| 8 | H | $CH_3$ | $CH_2CF_3$ | 171.5~173.5 |
| | | | | |
| 9 | H | H | phenyl | 188~190 |
| 10 | 4,6-$(CH_3)_2$ | $CH_3$ | $CH_3$ | 201~204 |
| 11 | 5-Cℓ,6-$OCH_3$ | " | " | 166~168 |
| 12 | H | H | $CH_2C{\equiv}CH$ | 154.5~157 |
| 13 | " | -$(CH_2)_4$- | | 158~162 |
| | | | | |
| 14 | H | " | $COOCH_3$ | 162~164 |
| 15 | 6-$OC_2H_5$ | " | $CH_3$ | 208~209 |
| 16 | 6-$C_2H_5$ | " | " | 193~194.5 |
| 17 | 6-$CH_2OCH_3$ | " | " | 183~185 |
| 18 | 4,6-$(CH_3)_2$ | H | " | 179~181 |

(Table 3, continued from page 44)

| | | | | |
|---|---|---|---|---|
| 19 | H | H | $CH_3$ | 147~149.5 |
| 20 | " | " | $C_2H_5$ | 163~168 |
| 21 | " | " | $C_3H_7$(iso) | 166.5~168.5 |
| 22 | 6-$C_2H_5$ | " | $CH_3$ | - |
| | | | | |
| 23 | 6-$C_3H_7$(iso) | " | " | - |
| 24 | 6-I | " | " | - |
| | | | | |
| 25 | H | $CH_3$ | $C_2H_5$ | 170.5~172 |
| | | | | |
| | | | | |
| 26 | 5-$CHF_2$ | " | " | - |
| 27 | H | $C_2H_5$ | $C_2H_5$ | 109~111 |
| 28 | 6-$N(CH_3)_2$ | " | $CH_3$ | 199~201.5 |

(Table 3, continued from page 45)

| | | | | |
|---|---|---|---|---|
| 29 | 6-$SCH_3$ | $CH_3$ | $CH_3$ | 200~201.5 |
| | | | | |
| 30 | 6-$NHCH_3$ | " | " | 183~185 |
| | | | | |
| | | | | |
| 31 | H | H | $(CH_2)_3CH_3$ | 156~158 |
| 32 | 6-$CH_2F$ | $CH_3$ | $CH_3$ | 180~181 |
| 33 | 6-$CHF_2$ | " | " | 194~195 |
| 34 | H | " | 2,4-difluorophenyl | 156~158 |
| 35 | " | " | 4-chlorophenyl | 241~243 |
| 36 | 6-$CH_2OCH_2CF_3$ | " | $CH_3$ | 179~180 |
| 37 | 6-$OCH_2CF_3$ | $CH_3$ | " | 186.5~189 |

(Table 3, continued from page 46)

| | | | | | | | | |
|---|---|---|---|---|---|---|---|---|
| | | | | | | | | |
| | | | | | | | | |
| | | | | | | | | |
| 38 | Potassium salt of compound No. 19 | | | | | | | - |
| 39 | 6-I | | H | $CH_3$ | | | | - |
| 40 | $6-CHF_2$ | | " | " | | | | - |
| | | | | | | | | |
| | | | | | | | | |
| | | | | | | | | |
| 40 | $6-CHF_2$ | | " | " | | | | - |
| 41 | Sodium salt of compound No. 19 | | | | | | | |
| 42 | Sodium salt of compound No. 49 | | | | | | | |

The compounds according to the present invention have herbicidal effects against a variety of weeds: cyperaceous weeds (Cyperaceae) such as rice flatsedge (Cyperus iria), japanese bulrush (Scirpus juncoides), and purple nutsedge (Cyperus rotundus); gramineous weeds (Gramineae) such as barnyard grass (Echinochloa crus-galli), crab-grass (Digitaria adscendens), green foxtail (Setaria viridis), goosegrass (Eleusine indica), wild oat (Avena fatua), johnsongrass (Sorghum halepense), and quackgrass (Agropyron repens); and broadleaved weeds such as velvetleaf (Abutilon theophrasti), tall morningglory (Ipomoea purpurea), common lambsquarters (Chenopodium album), prickly sida (Sida spinosa), common purslane (Portulaca oleracea), slender amaranth (Amaranthus viridis), sicklepod (Cassia tora), black nightshade (Solanum nigrum), smartweed (Polygonum longisetum), common chickweed (Stellaria media), common cocklebur (Xanthium strumarium), and wavy bittercress (Cardamine flexuosa). Therefore, herbicides according to the present invention will find an application such as upland farms and many other applications such as agricultural fields, e.g., orchards and mulberry fields and nonagricultural fields, e.g., forests, farm roads, playgrounds, factory sites, and turf fields. The herbicidal composition of the present invention can be applied by soil treatment or foliar treatment, if desired.

In particular, the compounds according to the present invention can have herbicidal effects against noxious weeds in corns and can be effectively used. The herbicidal compound of the present invention is applied in the form of granules, wettable powder, emulsifiable concentrate or aqueous solution prepared by mixing the compound with a carrier as well as additives such as a diluent, solvent, emulsifier, spreader or surfactant, as required. A suitable mixing ratio of the active ingredient to the agricultural adjuvant(s) ranges from 1 : 99 to 90 : 10 and preferably from 5 : 95 to 60 : 40 by weight. An optimal amount of the active

ingredient applied cannot be unequivocally defined because it varies according to various factors such as the climate condition, the weather condition, the soil condition, the form of the chemical, the type of weeds to be controlled, or the time of application, but the amount of the active ingredient is usually from 0.1 to 100 g per are, preferably from 0.2 to 50 g, and more preferably 0.5 g to 10 g.

The herbicidal composition of the present invention can be mixed or used together with other agricultural chemicals, fertilizers, soil, or safeners. These use brings about a more excellent effect or action. Examples of other herbicides which can be mixed with the herbicidal composition of the present invention are listed below. In some cases, synergism may be achieved.

3,6-dichloro-2-methoxybenzoic acid
2,5-dichloro-3-aminobenzoic acid
(2,4-dichlorophenoxy)acetic acid
(4-chloro-2-methylphenoxy)acetic acid
2-chloro-4,6-bis(ethylamino)-1,3,5-triazine
2-chloro-4-ethylamino-6-isopropylamino-1,3,5-triazine
2-(4-chloro-6-ethylamino-1,3,5-triazin-2-ylamino)-2-methylpropionitrile
2-ethylamino-4-isopropylamino-6-methythio-1,3,5,-triazine
2-chloro-2',6'-diethyl-N-(methoxymethyl)acetanilide
2-chloro-6'-ethyl-N-(2-methoxy-1-methylethyl)aceto-o-toluidide
2-chloro-N-isopropylacetanilide
2-chloro-N,N-di-2-propenylacetamide
S-ethyl dipropylthiocarbamate
S-ethyl di-isobutylthiocarbamate
S-propyl dipropylthiocarbamate
N-(1-ethylpropyl)-2,6-dinitro-3,4-xylidine
$\alpha,\alpha,\alpha$-trifluoro-2,6-dinitro-N,N-dipropyl-p-toluidine
2-(3,5-dichlorophenyl)-2-(2,2,2-trichloroethyl) oxirane
3-isopropyl-(1H)-benzo-2,1,3-thiadiazin-4-one-2,2-dioxide
3-(3,4-dichlorophenyl)-1-methoxy-1-methylurea
3,5-dibromo-4-hydroxybenzonitrile
2-chloro-4-trifluoromethylphenyl-3-ethoxy-4-nitrophenylether

For example, N-[(4,6-dimethoxypyrimidin-2-yl)aminocarbonyl]-3-methylaminocarbonyl-2-pyridinesulfonamide, or 6-difluoromethyl-N-[(4,6-dimethoxypyrimidin-2-yl)aminocarbonyl-3-dimethylaminocarbonyl-2-pyridinsulfonamide can be used in combination with 2-chloro-4-ethylamino-6-isopropylamino-1,3,5-triazine, 2-(4-chloro-6-ethylamino-1,3,5-triazin-2-ylamino)-2-methylpropionitrile, 2-chloro-2',6'-diethyl-N-(methoxymethyl) acetanilide, 2-chloro-6'-ethyl-N-(2-methoxy-1-methylethyl)aceto-o-toluidide or N-(1-ethylpropyl)-2,6-dinitro-3,4-xylidine. The herbicide of this combination is not noxious for corn and permits substantially completely killing weeds.

Test Example 1

1/1,500 are pots were charged with upland soil, and predetermined amounts of seeds of various test plants were sown. When the test plants had respectively reached the predetermined growth stages (i.e., 2.2- to 3.5-leaf stage for corn (Zea mays), 2.0- to 3.5-leaf stage for wheat (Triticum aestium), 2.0- to 3.5-leaf stage for common cocklebur, 0.5- to 1.2-leaf stage for tall morningglory, 0.5- to 1.2-leaf stage for smartweed, 0.1- to 1.5-leaf stage for prickly sida, 0.1-to 1.5-leaf stage for slender amaranth, and 2.0- to 2.5-leaf stage for barnyard grass), an aqueous disperson was prepared by diluting a wettable powder containing a predetermined amount of each of the test compounds with 5 ℓ/are of water, and 0.2% of an agricultural spreader was added to the aqueous solution. The resultant solution was foliarly applied to the plant with a small-sized sprayer. Twenty-two to thirty-seven days after the application, the degree of growth of the plant was visually observed. The weed control was evaluated on a scale of 10 grades in which 10 indicates that the plant was completely killed and 1 indicates no effects, as shown in Table 4 below.

Table 4

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 1 | 5 | 1 | 9 | 10 | 9 | 5 | 8 | 7 | 10 | 24th day |
| | 1.25 | 1 | 7 | 9 | 7 | 4 | 7 | 5 | 7 | |
| 2 | 5 | 1 | 3 | 10 | 8 | 7 | 6 | 7 | 8 | 25th day |
| | 1.25 | 1 | 2 | 9 | 6 | 5 | 4 | 5 | 8 | |
| 3 | 5 | 1 | 5 | ·9 | 9 | 5 | 7 | 8 | 6 | 28th day |
| | 1.25 | 1 | 1 | 6 | 6 | 4 | 5 | 6 | 3 | |
| 4 | 5 | 1 | 9 | 10 | 9 | 7 | 9 | 8 | 10 | 25th day |
| | 1.25 | 1 | 7 | 9 | 9 | 6 | 8 | 6 | 10 | |
| 5 | 5 | 1 | 3 | 8 | 9 | 6 | 4 | 10 | 5 | 28th day |
| | 1.25 | 1 | 1 | 7 | 7 | 4 | 4 | 7 | 4 | |
| 6 | 5 | 1 | 6 | 10 | 9 | 8 | 8 | 10 | 9 | 28th day |
| | 1.25 | 1 | 7 | 10 | 9 | 6 | 8 | 10 | 10 | |
| 7 | 5 | 1 | 6 | 10 | 7 | 6 | 10 | 7 | 6 | 24th day |
| | 1.25 | 1 | 4 | 9 | 6 | 6 | 7 | 7 | 4 | |

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 8 | 5 | 1 | 6 | 9 | 9 | 5 | 7 | 5 | 7 | 24th day |
| | 1.25 | 1 | 5 | 9 | 7 | 4 | 6 | 4 | 7 | |
| | | | | | | | | | | |
| | | | | | | | | | | |
| 9 | 5 | 1 | 4 | 10 | 8 | 5 | 10 | 10 | - | 37th day |
| | 1.25 | 1 | 1 | 10 | 8 | - | 7 | 8 | - | |
| 10 | 5 | 1 | 6 | 10 | 10 | 6 | 10 | 10 | - | 37th day |
| | 1.25 | 1 | 5 | 10 | 9 | 6 | 9 | 10 | - | |
| 11 | 5 | 1 | 8 | 10 | 10 | 6 | 10 | 10 | 9 | 28th day |
| | 1.25 | 1 | 7 | 10 | 10 | - | 9 | 8 | 7 | |
| 12 | 5 | 1 | 9 | 10 | 10 | 5 | 10 | 10 | 9 | 37th day |
| | 1.25 | 1 | 9 | 10 | 9 | 4 | 10 | 9 | 9 | |
| 13 | 5 | 2 | 9 | 9 | 10 | 8 | 8 | 10 | 10 | 28th day |
| | 1.25 | 1 | 8 | 8 | 8 | 7 | 7 | 9 | 8 | |

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| | | | | | | | | | | |
| | | | | | | | | | | |
| 14 | 5 | 1 | 9 | 9 | 9 | 8 | 9 | 8 | 7 | 28th day |
| | 1.25 | 1 | 4 | 8 | 7 | 6 | 8 | 6 | 6 | |
| 15 | 5 | 1 | 7 | 10 | 10 | 6 | 10 | 9 | 8 | 28th day |
| | 1.25 | 1 | 7 | 10 | 10 | 6 | 10 | 9 | 5 | |
| 16 | 5 | 1 | - | 10 | 9 | 6 | 10 | 10 | 8 | 28th day |
| | 1.25 | 1 | - | 10 | 9 | 4 | 10 | 10 | 6 | |
| 17 | 5 | 1 | 10 | 10 | 10 | 9 | 10 | 10 | 10 | 25th day |
| | 1.25 | 1 | 10 | 10 | 10 | 7 | 10 | 10 | 10 | |
| 18 | 5 | 1 | 10 | 10 | 10 | 6 | 9 | 10 | 7 | 37th day |
| | 1.25 | 1 | 5 | 7 | 9 | 5 | 9 | 9 | 5 | |
| | | | | | | | | | | |
| | | | | | | | | | | |

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 19 | 5 | 1 | 10 | 10 | 10 | 10 | 10 | 10 | 10 | 30th day |
| | 1.25 | 1 | 8 | 10 | 9 | 7 | 10 | 10 | 10 | |
| 20 | 5 | 1 | 8 | 10 | 10 | 7 | 8 | 9 | 10 | 24th day |
| | 1.25 | 1 | 8 | 9 | 7 | 7 | 7 | 9 | 9 | |
| 21 | 5 | 1 | 7 | 10 | 8 | 7 | 8 | 10 | 10 | 24th day |
| | 1.25 | 1 | 6 | 7 | 8 | 7 | 7 | 8 | 9 | |
| | | | | | | | | | | |
| | | | | | | | | | | |
| 25 | 5 | 2 | 10 | 10 | 9 | 9 | 9 | 10 | 10 | 25th day |
| | 1.25 | 1 | 9 | 10 | 9 | 8 | 9 | 9 | 10 | |
| | | | | | | | | | | |
| | | | | | | | | | | |
| | | | | | | | | | | |
| | | | | | | | | | | |

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| 27 | 5 | 1 | 5 | 10 | 9 | 6 | 8 | 10 | 9 | 28th day |
| | 1.25 | 1 | 6 | 9 | 8 | 6 | 8 | 10 | 9 | |
| | | | | | | | | | | |
| | | | | | | | | | | |
| 28 | 5 | 3 | 10 | 10 | 10 | 9 | 10 | 10 | 10 | 31th day |
| | 1.25 | 1 | 8 | 10 | 9 | 8 | 10 | 10 | 9 | |
| 29 | 5 | 1 | - | 10 | 9 | 6 | 10 | 10 | 8 | 28th day |
| | 1.25 | 1 | - | 10 | 9 | 4 | 10 | 10 | 6 | |
| | | | | | | | | | | |
| | | | | | | | | | | |
| 30 | 5 | 2 | - | 10 | 10 | 8 | 10 | 9 | 10 | 22nd day |
| | 1.25 | 1 | - | 10 | 7 | 7 | 9 | 4 | 9 | |
| | | | | | | | | | | |
| | | | | | | | | | | |

| Compound No. | Amount of Active Ingredient (g/a) | Weed Control | | | | | | | | Observation Day |
|---|---|---|---|---|---|---|---|---|---|---|
| | | Corn | Wheat | Common Cocklebur | Tall Morning Glory | Prickly Sida | Smart Weed | Slender Amaranth | Barnyard Grass | |
| | | | | | | | | | | |
| | | | | | | | | | | |
| 31 | 5 | 1 | 9 | 10 | 9 | 5 | 6 | 7 | 6 | 25th day |
| | 1.25 | 1 | 7 | 9 | 7 | 2 | 4 | 5 | 5 | |
| 32 | 5 | 1 | - | 10 | 9 | 6 | 10 | 10 | 10 | 26th day |
| | 1.25 | 1 | - | 8 | 7 | 5 | 10 | 7 | 10 | |
| 33 | 5 | 2 | 10 | 10 | 10 | 7 | 9 | 10 | 10 | 29th day |
| | 1.25 | 1 | 10 | 10 | 10 | 6 | 9 | 9 | 10 | |
| 34 | 5 | 1 | 10 | 10 | 6 | 4 | 7 | 7 | 6 | 29th day |
| | 1.25 | 1 | 4 | 10 | 6 | 3 | 4 | 6 | 5 | |
| 36 | 5 | 2 | - | 9 | 10 | 7 | 6 | 8 | 10 | 36th day |
| | 1.25 | 1 | - | 9 | 7 | 4 | 5 | 5 | 6 | |
| 37 | 5 | 1 | - | 10 | 9 | 2 | 8 | 5 | 7 | 22nd day |
| | 1.25 | 1 | - | 9 | 8 | 1 | 6 | 3 | 4 | |

**Test Example 2**

1/10,000 are pots were respectively charged with upland soil, and seeds of crab-grass and black nightshade were sown. Thereafter, when test plants of crab-grass and black nightshade had reached 2- and 0.5-leaf stages, respectively, the compound to be used was weighed such that the content of the active ingredient was a predetermined amount, and the compound was diluted in 5 l water per are. 0.2% of an agricultural spreader was added to this aqueous solution, and the resultant solution was sprayed with a small-sized sprayer. The rate of growth of crab-grass and black nightshade 23rd days after the application was visually checked, and weed control was evaluated in the same manner as in test example 1. The results are shown in Table 5

Table 5

| Compound No. | Amount of Active Ingredient (g/a) | Weed Control | |
|---|---|---|---|
| | | Crab-grass | Black nightshade |
| 19 | 5.0<br>2.5 | 8<br>7 | 10<br>10 |

**Test Example 3**

Two 15cm long germinated rhizomes of johnsongrass were planted in a 1/3000 are pot and grown in a greenhouse. When the johnsongrass had reached 4- to 5-leaf stage, the compound to be used was weighed such that the content of the active ingredient was a predetermined amount, and the compound was diluted with 5l water per are. In addition, 0.2% of an agricultural spreader was added to this aqueous solution, and the resultant solution was sprayed to the leaves and stems of the plants. Thirty-five days after the application, weed control for johnsongrass was visually checked. Weed control was evaluated in the same manner as in test example 1, and results are shown in Table 6 below.

Table 6

| Compound No | Amount of Active Ingredient (g/a) | Weed Control |
|---|---|---|
| 19 | 5.0<br>2.5<br>1.25<br>0.625 | 9<br>8 - 9<br>7<br>4 |

Formulation preparation examples of herbicidal compositions according to the present invention will be described below

**Formulation Example 1**

| | | **parts by weight** |
|---|---|---|
| (1) | Kaolin | 78 |
| (2) | Condensate of sodium naphthalensulfonate and formalin | 2 |
| (3) | Polyoxyethylenealkylallylethersulfate | 5 |
| (4) | Silica micropowder | 15 |

Ingredients (1) to (4) are mixed with the compounds of the present invention at a mixing ratio of 9 : 1 to prepare a wettable powder.

**Formulation Example 2**

| | | parts by weight |
|---|---|---|
| (1) | Sodium phenylsulfonate | 4 |
| (2) | Sodium polycarboxylate | 3 |
| (3) | Sodium alkylarylsulfonate | 1 |
| (4) | Kaolin | 12 |
| (5) | Compound No. 19 | 80 |

The above ingredients are mixed with an addition of water, after drying them and disintegrated to form a wettable powder.

## Claims

**Claims for the following Contracting States : DE, FR, GB, IT, NL, CH, LI**

**1.** A substituted pyridinesulfonamide compound represented by the following general formula:

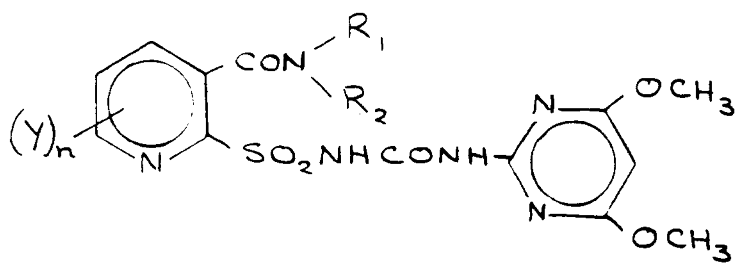

wherein each of $R_1$ and $R_2$ independently represents a hydrogen atom, a $(C_1\text{-}C_6)$alkyl group, a halo$(C_1\text{-}C_6)$alkyl group, a $(C_2\text{-}C_6)$alkenyl group, a $(C_2\text{-}C_6)$alkynyl group, a $(C_1\text{-}C_6)$alkoxy group, a halo$(C_1\text{-}C_6)$-alkoxy group, a $(C_1\text{-}C_6)$alkoxy$(C_1\text{-}C_6)$alkyl group, a halo$(C_1\text{-}C_6)$alkoxy$(C_1\text{-}C_6)$alkyl group, a $(C_3\text{-}C_6)$-cycloalkyl group, a halo$(C_3\text{-}C_6)$cycloalkyl group, a $(C_1\text{-}C_6)$alkoxycarbonyl group, a halo$(C_1\text{-}C_6)$-alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1\text{-}C_6)$alkyl group, a halo$(C_1\text{-}C_6)$alkyl group, a $(C_1\text{-}C_6)$alkoxy group, a halo$(C_1\text{-}C_6)$alkoxy group, a $(C_1\text{-}C_6)$alkylthio group, a halo$(C_1\text{-}C_6)$alkylthio group, a $(C_1\text{-}C_6)$alkoxy$(C_1\text{-}C_6)$alkyl group, a halo$(C_1\text{-}C_6)$alkoxy$(C_1\text{-}C_6)$alkyl group, or
an

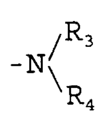

group (wherein each of $R_3$ and $R_4$ represents a hydrogen atom or a $(C_1\text{-}C_6)$alkyl group); and $\underline{n}$ represents 0 or an integer of 1 or 2; with the provisos that (a) when $\underline{n}$ is 0, $R_1$ and $R_2$ are not both methyl groups, (b) when $R_1$ and $R_2$ are both $(C_1\text{-}C_3)$alkyl or when $R_1$ is hydrogen and $R_2$ is $(C_1\text{-}C_3)$-alkyl, and $\underline{n}$ is 1, Y is not Cl, F, Br, $CH_3$, $OCH_3$, $CF_3$ or $OCF_2H$, and (c) when $R_1$ is a hydrogen atom or a $(C_1\text{-}C_3)$alkyl group and $R_2$ is a $(C_1\text{-}C_2)$alkoxy group, and $\underline{n}$ is 1, Y is not $CF_3$ or $OCF_2H$; or a salt of such a compound.

**2.** A compound as claimed in claim 1, wherein $R_1$ is a hydrogen atom or a $(C_1\text{-}C_6)$alkyl group, $R_2$ is a $(C_1\text{-}C_6)$alkyl group, Y is a halogen atom, a $(C_1\text{-}C_6)$alkyl group, a halo$(C_1\text{-}C_6)$alkyl group, a $(C_1\text{-}C_6)$-alkoxy group or a $(C_1\text{-}C_6)$alkoxy$(C_1\text{-}C_6)$alkyl group, and n is 0 or 1, with the provisos that (a) when $\underline{n}$ is 0, $R_1$ and $R_2$ are not both methyl groups, (b) when $R_1$ and $R_2$ are both $(C_1\text{-}C_3)$alkyl or when $R_1$ is

hydrogen and $R_2$ is ($C_1$-$C_3$) alkyl, and n is 1, Y is not Cl, F, Br, $CH_3$, $OCH_3$, $CF_3$ or $OCF_2H$, and (c) when $R_1$ is a hydrogen atom or a ($C_1$-$C_3$)alkyl group and $R_2$ is a ($C_1$-$C_2$)alkoxy group, and n is 1, Y is not $CF_3$ or $OCF_2H$; or a salt of such a compound.

**3.** A compound as claimed in claim 1, wherein $R_1$ is a hydrogen atom or a methyl group, $R_2$ is a methyl group, Y is a difluoromethyl group located at the 6-position of the pyridine nucleus, and n is 0 or 1, with the proviso that when n is 0, $R_1$ and $R_2$ are not both methyl groups; or a salt of such a compound.

**4.** 6-Difluoromethyl-N-[(4,6-dimethoxypyrimidin-2-yl)-aminocarbonyl]-3-dimethylaminocarbonyl-2-pyridinesulfonamide.

**5.** N-[(4,6-Dimethoxypyrimidin-2-yl)aminocarbonyl]-3-methylaminocarbonyl-2-pyridinesulfonamide.

**6.** N-[(4,6-Dimethoxypyrimidin-2-yl)aminocarbonyl]-3-dimethylaminocarbonyl-6-ethyl-2-pyridinesulfonamide.

**7.** N-[(4,6-Dimethoxypyrimidin-2-yl)aminocarbonyl]-3-dimethylaminocarbonyl-6-methoxymethyl-2-pyridinesulfonamide.

**8.** N-[(4,6-Dimethoxypyrimidin-2-yl)aminocarbonyl]-3-(N'-ethyl-N'-methylaminocarbonyl)-2-pyridinesulfonamide.

**9.** A herbicidal composition essentially consisting of a herbicidally effective amount of a compound or salt thereof as defined in any preceding claim, and an agriculturally acceptable adjuvant.

**10.** A herbicidal composition as claimed in claim 9, wherein the mixing ratio of the compound or salt thereof to the agriculturally acceptable adjuvant is 1:99 to 90:10.

**11.** A composition as claimed in claim 9, which also includes a further herbicide.

**12.** A composition as claimed in claim 11, wherein said further herbicide is selected from
3,6-dichloro-2-methoxybenzoic acid
2,5-dichloro-3-aminobenzoic acid
(2,4-dichlorophenoxy)acetic acid
(4-chloro-2-methylphenoxy)acetic acid
2-chloro-4,6-bis(ethylamino)-1,3,5-triazine
2-chloro-4-ethylamino-6-isopropylamino-1,3,5-triazine
2-(4-chloro-6-ethylamino-1,3,5-triazin-2-ylamino)-2-methylpropionitrile
2-ethylamino-4-isopropylamino-6-methythio-1,3,5-triazine
2-chloro-2',6'-diethyl-N-(methoxymethyl)acetanilide
2-chloro-6'-ethyl-N-(2-methoxy-1-methylethyl)aceto-o-toluidide
2-chloro-N-isopropylacetanilide
2-chloro-N,N-di-2-propenylacetamide
S-ethyl dipropylthiocarbamate
S-ethyl di-isobutylthiocarbamate
S-propyl dipropylthiocarbamate
N-(1-ethylpropyl)-2,6-dinitro-3,4-xylidine
$\alpha,\alpha,\alpha$-trifluoro-2,6-dinitro-N,N-dipropyl-p-toluidine
2-(3,5-dichlorophenyl)-2-(2,2,2-trichloroethyl) oxirane
3-isopropyl-(1H)-benzo-2,1,3-thiadiazin-4-one-2,2-dioxide
3-(3,4-dichlorophenyl)-1-methoxy-1-methylurea
3,5-dibromo-4-hydroxybenzonitrile
2-chloro-4-trifluoromethylphenyl-3-ethoxy-4-nitrophenylether.

**13.** A composition as claimed in claim 9, which also contains a further herbicide selected from
2-chloro-4-ethylamino-6-isopropylamino-1,3,5-triazine
2-(4-chloro-6-ethylamino-1,3,5-triazin-2-ylamino)-2-methylpropionitrile
2-chloro-2',6'-diethyl-N-(methoxymethyl)acetanilide

2-chloro-6'-ethyl-N-(2-methoxy-1-methylethyl)aceto-o-toluidide and
N-(1-ethylpropyl)-2,6-dinitro-3,4-xylidine.

**14.** A herbicidal composition comprising a herbicidally effective amount of a compound as claimed in any one of claims 1 to 8, but without said provisos (b) and (c), an agriculturally acceptable adjuvant, and at least one further herbicide selected from
2,5-dichloro-3-aminobenzoic acid
(4-chloro-2-methylphenoxy)acetic acid
2-chloro-4,6-bis(ethylamino)-1,3,5-triazine
2-ethylamino-4-isopropylamino-6-methythio-1,3,5-triazine
2-chloro-N-isopropylacetanilide
2-chloro-N,N-di-2-propenylacetamide
S-propyl dipropylthiocarbamate
N-(1-ethylpropyl)-2,6-dinitro-3,4-xylidine
α,α,α-trifluoro-2,6-dinitro-N,N-dipropyl-p-toluidine
3-isopropyl-(1H)-benzo-2,1,3-thiadiazin-4-one-2,2-dioxide
2-chloro-4-trifluoromethylphenyl-3-ethoxy-4-nitrophenylether.

**15.** A method of killing noxious weeds in a cornfield by applying a composition as claimed in any one of claims 9 to 14, in an amount of 0.1 to 100 g/are based on said compound or salt thereof.

**16.** A process for producing a substituted pyridinesulfonamide compound represented by the following general formula:

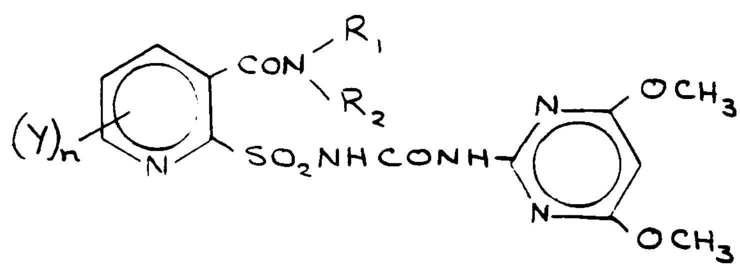

wherein each of $R_1$ and $R_2$ independently represents a hydrogen atom, a $(C_1-C_6)$alkyl group, a halo$(C_1-C_6)$alkyl group, a $(C_2-C_6)$alkenyl group, a $(C_2-C_6)$alkynyl group, a $(C_1-C_6)$alkoxy group, a halo$(C_1-C_6)$alkoxy group, a $(C_1-C_6)$alkoxy$(C_1-C_6)$alkyl group, a halo$(C_1-C_6)$alkoxy$(C_1-C_6)$alkyl group, a $(C_3-C_6)$cycloalkyl group, a halo$(C_3-C_6)$cycloalkyl group, a $(C_1-C_6)$alkoxycarbonyl group, a halo$(C_1-C_6)$alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1-C_6)$alkyl group, a halo$(C_1-C_6)$alkyl group, a $(C_1-C_6)$alkoxy group, a halo$(C_1-C_6)$alkoxy group, a $(C_1-C_6)$alkylthio group, a halo$(C_1-C_6)$alkylthio group, a $(C_1-C_6)$alkoxy$(C_1-C_6)$alkyl group, a halo$(C_1-C_6)$alkoxy$(C_1-C_6)$alkyl group, or
an

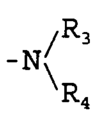

group (wherein each of $R_3$ and $R_4$ represents a hydrogen atom or a $(C_1-C_6)$alkyl group); and n represents 0 or an integer of 1 or 2; with the proviso that when n is 0, $R_1$ and $R_2$ are not both methyl groups; or a salt of such a compound;
said process comprising reacting a pyridine compound represented by the following general formula:

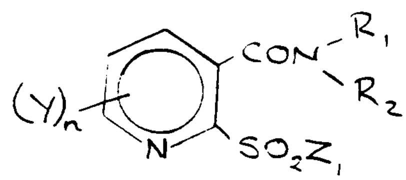

wherein $R_1$ and $R_2$ are as defined above and $Z_1$ represents an $-NH_2$ group, an -NCO group, an -NHCOCl group or an $-NHCOOR_5$ group (wherein $R_5$ represents an alkyl group or an aryl group), with a pyrimidine compound represented by the following general formula:

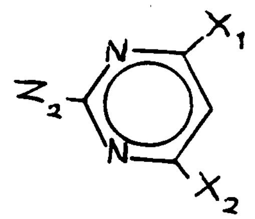

wherein each of $X_1$ and $X_2$ independently represents a halogen atom or a methoxy group, and $Z_2$ represents an $-NH_2$group, an -NCO group, an -NHCOCl group or an $-NHCOOR_5$ group (wherein $R_5$ is as defined above), provided that when $Z_1$ represents the $-NH_2$ group, $Z_2$ represents the -NCO group, the -NHCOCl group or the $-NHCOOR_5$ group, and that when $Z_2$ represents the $-NH_2$ group, $Z_1$ represents the -NCO group, the -NHCOCl group or the $-NHCOOR_5$ group; then performing methoxylation when $X_1$ and/or $X_2$ represents a halogen atom; and, if desired, performing a salt formation treatment;

with the further provisos that, when (a) $R_1$ and $R_2$ are both $(C_1-C_3)$alkyl or $R_1$ is hydrogen and $R_2$ is $(C_1-C_3)$alkyl, n is 1, and Y is Cl, F, Br, $CH_3$, $OCH_3$, $CF_3$ or $OCF_2H$, or when (b) $R_1$ is a hydrogen atom or a $(C_1-C_3)$alkyl group and $R_2$ is a $(C_1-C_2)$alkoxy group, n is 1 and Y is $CF_3$ or $OCF_2H$, then $Z_1$ is not $-NH_2$ or $-NHCOOC_6H_5$ when $Z_2$ is $-NHCOOC_6H_5$ or $-NH_2$, respectively.

**17.** A process as claimed in claim 16, wherein $Z_1$ represents an $-NH_2$ group, and $Z_2$ represents an -NCO group, an -NHCOCl group or an $-NHCOOR_5$ group.

**18.** A process as claimed in claim 16, wherein $Z_1$ represents an -NCO group, an -NHCOCl group or an $-NHCOOR_5$ group, and $Z_2$ represents an $-NH_2$ group.

**19.** A process according to claim 16, wherein the temperature of reaction of the pyridine compound with the pyrimidine compound is -20 to +100 °C.

**20.** A pyridine compound as an intermediate represented by the following general formula:

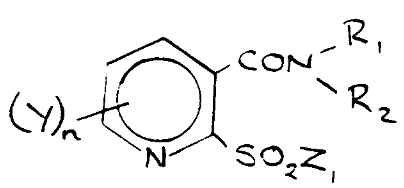

wherein each of $R_1$ and $R_2$ independently represents a hydrogen atom, a $(C_1-C_6)$alkyl group, a halo$(C_1-C_6)$alkyl group, a $(C_2-C_6)$alkenyl group, a $(C_2-C_6)$alkynyl group, a $(C_1-C_6)$alkoxy group, a halo$(C_1-C_6)$-alkoxy group, a $(C_1-C_6)$alkoxy$(C_1-C_6)$alkyl group, a halo$(C_1-C_6)$alkoxy$(C_1-C_6)$alkyl group, a $(C_3-C_6)$-cycloalkyl group, a halo$(C_3-C_6)$cycloalkyl group, a $(C_1-C_6)$alkoxycarbonyl group, a halo$(C_1-C_6)$-alkoxycarbonyl group, a phenyl group or a halophenyl group, provided that when one of $R_1$ and $R_2$ represents a hydrogen atom, the other represents one of the groups excluding the hydrogen atom; $R_1$

and $R_2$ together with an adjacent nitrogen atom may constitute a heterocycle; Y represents a halogen atom, a ($C_1$-$C_6$)alkyl group, a halo($C_1$-$C_6$)alkyl group, a ($C_1$-$C_6$)alkoxy group, a halo($C_1$-$C_6$)alkoxy group, a ($C_1$-$C_6$)alkylthio group, a halo($C_1$-$C_6$)alkylthio group, a ($C_1$-$C_6$)alkoxy($C_1$-$C_6$)alkyl group, a halo($C_1$-$C_6$)alkoxy($C_1$-$C_6$)alkyl group, or an

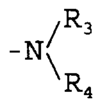

group (wherein each of $R_3$ and $R_4$ represents a hydrogen atom or a ($C_1$-$C_6$)alkyl group); n represents 0 or an integer of 1 or 2; and $Z_1$ is an $NH_2$ group, an -NCO group, an _NHCOCl group or an -NHCOOR$_5$ group (wherein $R_5$ is an alkyl or an aryl group), with the provisos that (a) when n is 0, $R_1$ and $R_2$ are not both methyl groups, (b) when $R_1$ and $R_2$ are both ($C_1$-$C_3$)alkyl or when $R_1$ is hydrogen and $R_2$ is ($C_1$-$C_3$)alkyl, n is 1 and Y is Cl, F, Br, $CH_3$, $OCH_3$, $CF_3$ or $OCF_2H$, $Z_1$ is not -$NH_2$ or -$NHCOOC_6H_5$ and (c) when $R_1$ is a hydrogen atom or a ($C_1$-$C_3$)alkyl group and $R_2$ is a ($C_1$-$C_2$)alkoxy group, n is 1 and Y is $CF_3$ or $OCF_2H$, $Z_1$ is not -$NH_2$ or -$NHCOOC_6H_5$.

**Claims for the following Contracting States : AT, ES**

**1.** A process for producing a substituted pyridinesulfonamide compound represented by the following general formula:

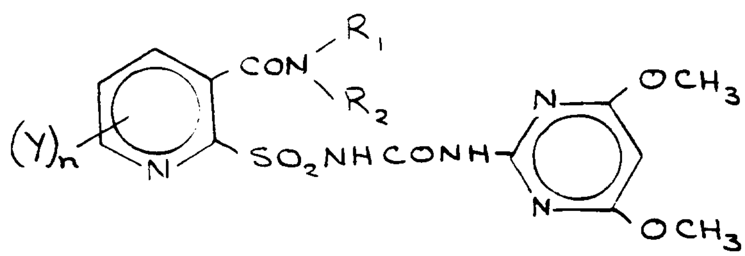

wherein each of $R_1$ and $R_2$ independently represents a hydrogen atom, a ($C_1$-$C_6$)alkyl group, a halo($C_1$-$C_6$)alkyl group, a ($C_2$-$C_6$)alkenyl group, a ($C_2$-$C_6$)alkynyl group, a ($C_1$-$C_6$)alkoxy group, a halo($C_1$-$C_6$)-alkoxy group, a ($C_1$-$C_6$)alkoxy($C_1$-$C_6$)alkyl group, a halo($C_1$-$C_6$)alkoxy($C_1$-$C_6$)alkyl group, a ($C_3$-$C_6$)-cycloalkyl group, a halo($C_3$-$C_6$)cycloalkyl group, a ($C_1$-$C_6$)alkoxycarbonyl group, a halo($C_1$-$C_6$)-alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a ($C_1$-$C_6$)alkyl group, a halo($C_1$-$C_6$)alkyl group, a ($C_1$-$C_6$)alkoxy group, a halo($C_1$-$C_6$)alkoxy group, a ($C_1$-$C_6$)alkylthio group, a halo($C_1$-$C_6$)alkylthio group, a ($C_1$-$C_6$)alkoxy($C_1$-$C_6$)alkyl group, a halo($C_1$-$C_6$)alkoxy($C_1$-$C_6$)alkyl group, or an

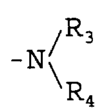

group (wherein each of $R_3$ and $R_4$ represents a hydrogen atom or a ($C_1$-$C_6$)alkyl group); and n represents 0 or an integer of 1 or 2; with the proviso that when n is 0, $R_1$ and $R_2$ are not both methyl groups; or a salt of such a compound;
said process comprising reacting a pyridine compound represented by the following general formula:

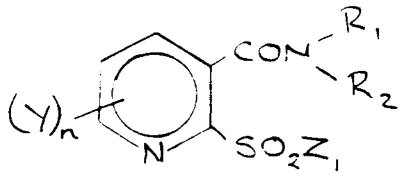

wherein $R_1$ and $R_2$ are as defined above and $Z_1$ represents an $-NH_2$ group, an -NCO group, an -NHCOCl group or an $-NHCOOR_5$ group (wherein $R_5$ represents an alkyl group or an aryl group), with a pyrimidine compound represented by the following general formula:

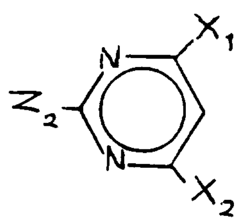

wherein each of $X_1$ and $X_2$ independently represents a halogen atom or a methoxy group, and $Z_2$ represents an $-NH_2$ group, an -NCO group, an -NHCOCl group or an $-NHCOOR_5$ group (wherein $R_5$ is as defined above), provided that when $Z_1$ represents the $-NH_2$ group, $Z_2$ represents the -NCO group, the -NHCOCl group or the $-NHCOOR_5$ group, and that when $Z_2$ represents the $-NH_2$ group, $Z_1$ represents the -NCO group, the -NHCOCl group or the $-NHCOOR_5$ group; then performing methoxylation when $X_1$ and/or $X_2$ represents a halogen atom; and, if desired, performing a salt formation treatment;

with the further provisos that, when (a) $R_1$ and $R_2$ are both $(C_1\text{-}C_3)$alkyl or $R_1$ is hydrogen and $R_2$ is $(C_1\text{-}C_3)$alkyl, n is 1, and Y is Cl, F, Br, $CH_3$, $OCH_3$, $CF_3$ or $OCF_2H$, or when (b) $R_1$ is a hydrogen atom or a $(C_1\text{-}C_3)$alkyl group and $R_2$ is a $(C_1\text{-}C_2)$alkoxy group, n is 1 and Y is $CF_3$ or $OCF_2H$, then $Z_1$ is not $-NH_2$ or $-NHCOOC_6H_5$ when $Z_2$ is $-NHCOOC_6H_5$ or $-NH_2$, respectively.

2. A process as claimed in claim 1, wherein $Z_1$ represents an $-NH_2$ group, and $Z_2$ represents an -NCO group, an -NHCOCl group or an $-NHCOOR_5$ group.

3. A process as claimed in claim 1, wherein $Z_1$ represents an -NCO group, an -NHCOCl group or an $-NHCOOR_5$ group, and $Z_2$ represents an $-NH_2$ group.

4. A process according to claim 1, wherein the temperature of reaction of the pyridine compound with the pyrimidine compound is -20 to +100 °C.

5. A process as claimed in any preceding claim, wherein $R_1$ is a hydrogen atom or a $(C_1\text{-}C_6)$alkyl group, $R_2$ is a $(C_1\text{-}C_6)$alkyl group, Y is a halogen atom, a $(C_1\text{-}C_6)$alkyl group, a halo$(C_1\text{-}C_6)$alkyl group, a $(C_1\text{-}C_6)$alkoxy group or a $(C_1\text{-}C_6)$alkoxy$(C_1\text{-}C_6)$alkyl group, and n is 0 or 1, with the provisos that (a) when n is 0, $R_1$ and $R_2$ are not both methyl groups, (b) when $R_1$ and $R_2$ are both $(C_1\text{-}C_3)$alkyl or when $R_1$ is hydrogen and $R_2$ is $(C_1\text{-}C_3)$ alkyl, and n is 1, Y is not Cl, F, Br, $CH_3$, $OCH_3$, $CF_3$ or $OCF_2H$, and (c) when $R_1$ is a hydrogen atom or a $(C_1\text{-}C_3)$alkyl group and $R_2$ is a $(C_1\text{-}C_2)$alkoxy group, and n is 1, Y is not $CF_3$ or $OCF_2H$.

6. A process as claimed in claim 1, wherein $R_1$ is a hydrogen atom or a methyl group, $R_2$ is a methyl group, Y is a difluoromethyl group located at the 6-position of the pyridine nucleus, and n is 0 or 1, with the proviso that when n is 0, $R_1$ and $R_2$ are not both methyl groups.

7. A process as claimed in any one of claims 1 to 4, wherein $R_1$ and $R_2$ are both methyl groups, n is 1 and Y is 6-difluoromethyl.

**8.** A process as claimed in any one of claims 1 to 4, wherein $R_1$ is hydrogen, $R_2$ is a methyl group, and n is 0.

**9.** A process as claimed in any one of claims 1 to 4, wherein $R_1$ and $R_2$ are both methyl groups, n is 1 and Y is 6-ethyl.

**10.** A process as claimed in any one of claims 1 to 4, wherein $R_1$ and $R_2$ are both methyl groups, n is 1 and Y is 6-methoxymethyl.

**11.** A process as claimed in any one of claims 1 to 4, wherein $R_1$ is methyl, $R_2$ is methyl, and n is 0.

**12.** A method of killing noxious weeds in a cornfield by applying 0.1 to 100 g/are, based on active ingredient, of a composition containing, as the active ingredient, a substituted pyridinesulfonamide compound, and an agriculturally acceptable adjuvant, said pyridinesulfonamide compound being represented by the general formula:

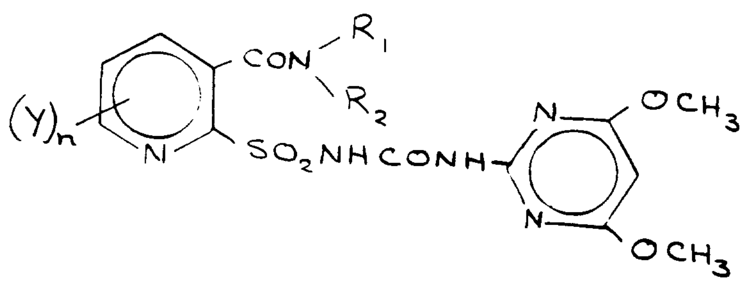

wherein each of $R_1$ and $R_2$ independently represents a hydrogen atom, a $(C_1\text{-}C_6)$alkyl group, a halo$(C_1\text{-}C_6)$alkyl group, a $(C_2\text{-}C_6)$alkenyl group, a $(C_2\text{-}C_6)$alkynyl group, a $(C_1\text{-}C_6)$alkoxy group, a halo$(C_1\text{-}C_6)$alkoxy group, a $(C_1\text{-}C_6)$alkoxy$(C_1\text{-}C_6)$alkyl group, a halo$(C_1\text{-}C_6)$alkoxy$(C_1\text{-}C_6)$alkyl group, a $(C_3\text{-}C_6)$cycloalkyl group, a halo$(C_3\text{-}C_6)$cycloalkyl group, a $(C_1\text{-}C_6)$alkoxycarbonyl group, a halo$(C_1\text{-}C_6)$alkoxycarbonyl group, a phenyl group or a halophenyl group, provided that when one of $R_1$ and $R_2$ represents a hydrogen atom, the other represents one of the groups excluding the hydrogen atom; $R_1$ and $R_2$ together with an adjacent nitrogen atom may constitute a heterocycle; Y represents a halogen atom, a $(C_1\text{-}C_6)$alkyl group, a halo$(C_1\text{-}C_6)$alkyl group, a $(C_1\text{-}C_6)$alkoxy group, a halo$(C_1\text{-}C_6)$alkoxy group, a $(C_1\text{-}C_6)$alkylthio group, a halo$(C_1\text{-}C_6)$alkylthio group, a $(C_1\text{-}C_6)$alkoxy$(C_1\text{-}C_6)$alkyl group, a halo$(C_1\text{-}C_6)$alkoxy$(C_1\text{-}C_6)$alkyl group, or
an

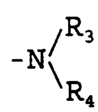

group (wherein each of $R_3$ and $R_4$ represents a hydrogen atom or a $(C_1\text{-}C_6)$alkyl group); and n represents 0 or an integer of 1 or 2; with the provisos that (a) when n is 0, $R_1$ and $R_2$ are not both methyl groups, (b) when $R_1$ and $R_2$ are both $(C_1\text{-}C_3)$alkyl or when $R_1$ is hydrogen and $R_2$ is $(C_1\text{-}C_3)$alkyl, and n is 1, Y is not Cl, F, Br, $CH_3$, $OCH_3$, $CF_3$ or $OCF_2H$, and (c) when $R_1$ is a hydrogen atom or a $(C_1\text{-}C_3)$alkyl group and $R_2$ is a $(C_1\text{-}C_2)$alkoxy group, and n is 1, Y is not $CF_3$ or $OCF_2H$; or a salt of such a compound.

**13.** A method as claimed in claim 12, wherein the mixing ratio of the compound or salt thereof to the agriculturally acceptable adjuvant is 1:99 to 90:10.

**14.** A method as claimed in claim 12, wherein the composition also contains a further herbicide.

**15.** A method as claimed in claim 14, wherein said further herbicide is selected from
3,6-dichloro-2-methoxybenzoic acid

2,5-dichloro-3-aminobenzoic acid
(2,4-dichlorophenoxy)acetic acid
(4-chloro-2-methylphenoxy)acetic acid
2-chloro-4,6-bis(ethylamino)-1,3,5-triazine
2-chloro-4-ethylamino-6-isopropylamino-1,3,5-triazine
2-(4-chloro-6-ethylamino-1,3,5-triazin-2-ylamino)-2-methylpropionitrile
2-ethylamino-4-isopropylamino-6-methythio-1,3,5-triazine
2-chloro-2',6'-diethyl-N-(methoxymethyl)acetanilide
2-chloro-6'-ethyl-N-(2-methoxy-1-methylethyl)aceto-o-toluidide
2-chloro-N-isopropylacetanilide
2-chloro-N,N-di-2-propenylacetamide
S-ethyl dipropylthiocarbamate
S-ethyl di-isobutylthiocarbamate
S-propyl dipropylthiocarbamate
N-(1-ethylpropyl)-2,6-dinitro-3,4-xylidine
α,α,α-trifluoro-2,6-dinitro-N,N-dipropyl-p-toluidine
2-(3,5-dichlorophenyl)-2-(2,2,2-trichloroethyl) oxirane
3-isopropyl-(1H)-benzo-2,1,3-thiadiazin-4-one-2,2-dioxide
3-(3,4-dichlorophenyl)-1-methoxy-1-methylurea
3,5-dibromo-4-hydroxybenzonitrile
2-chloro-4-trifluoromethylphenyl-3-ethoxy-4-nitrophenylether.

**16.** A method as claimed in claim 14, which also contains a further herbicide selected from
2-chloro-4-ethylamino-6-isopropylamino-1,3,5-triazine
2-(4-chloro-6-ethylamino-1,3,5-triazin-2-ylamino)-2-methylpropionitrile
2-chloro-2',6'-diethyl-N-(methoxymethyl)acetanilide
2-chloro-6'-ethyl-N-(2-methoxy-1-methylethyl)aceto-o-toluidide and
N-(1-ethylpropyl)-2,6-dinitro-3,4-xylidine.

**17.** A method as claimed in claim 14, wherein the herbicidal composition comprises a herbicidally effective amount of a compound as defined in claim 14, but without said provisos (b) and (c), an agriculturally acceptable adjuvant, and at least one further herbicide selected from
2,5-dichloro-3-aminobenzoic acid
(4-chloro-2-methylphenoxy)acetic acid
2-chloro-4,6-bis(ethylamino)-1,3,5-triazine
2-ethylamino-4-isopropylamino-6-methythio-1,3,5 triazine
2-chloro-N-isopropylacetanilide
2-chloro-N,N-di-2-propenylacetamide
S-propyl dipropylthiocarbamate
N-(1-ethylpropyl)-2,6-dinitro-3,4-xylidine
α,α,α-trifluoro-2,6-dinitro-N,N-dipropyl-p-toluidine
3-isopropyl-(1H)-benzo-2,1,3-thiadiazin-4-one-2,2 dioxide
2-chloro-4-trifluoromethylphenyl-3-ethoxy-4 nitrophenylether.

**18.** A herbicidal composition of the type defined in any one of claims 12 to 17.

**19.** A method of making a herbicidal composition comprising the step of mixing (a) a substituted pyridinesulfonamide of the type defined in claim 12 and optionally a further herbicide as defined in claim 14, 15 or 16, or (b) a substituted pyridine sulfonamide as defined in claim 17 and a further herbicide as defined in claim 17, with an agriculturally acceptable adjuvant.

## Patentansprüche

### Patentansprüche für folgende Vertragsstaaten : DE, FR, GB, IT, NL, CH, LI

1. Substituierte Pyridinsulfonamid-Verbindung, die durch die nachfolgende allgemeine Formel

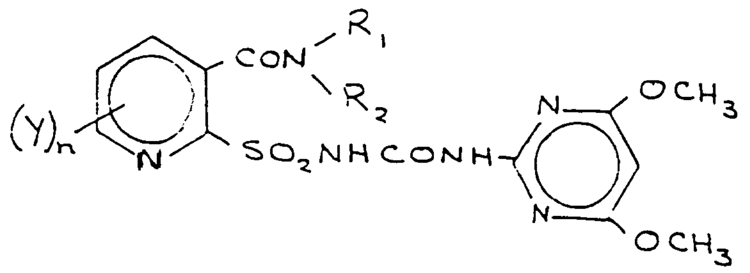

dargestellt ist, wobei sowohl $R_1$ wie auch $R_2$ unabhängig voneinander ein Wasserstoffatom, eine ($C_1$-$C_6$)Alkyl-Gruppe, eine Halogen($C_1$-$C_6$)alkyl-Gruppe, eine ($C_2$-$C_6$)Alkenyl-Gruppe, eine ($C_2$-$C_6$)Alkinyl-Gruppe, eine ($C_1$-$C_6$)Alkoxy-Gruppe, eine Halogen($C_1$-$C_6$)alkoxy-Gruppe, eine ($C_1$-$C_6$)Alkoxy($C_1$-$C_6$)-alkyl-Gruppe, eine Halogen($C_1$-$C_6$)alkoxy($C_1$-$C_6$)alkyl-Gruppe, eine ($C_3$-$C_6$)Cycloalkyl-Gruppe, eine Halogen($C_3$-$C_6$)cycloalkyl-Gruppe, eine ($C_1$-$C_6$)Alkoxycarbonyl-Gruppe, eine Halogen($C_1$-$C_6$)-Alkoxycarbonyl-Gruppe, eine Phenyl-Gruppe oder eine Halogenphenyl-Gruppe sind, unter der Voraussetzung daß, wenn einer der Reste $R_1$ und $R_2$ ein Wasserstoffatom darstellt, der andere eine der Gruppen mit Ausnahme des Wasserstoffatoms ist; $R_1$ und $R_2$ zusammen mit einem benachbarten Stickstoffatom einen Heterocyclus bilden können; Y ein Halogenatom, eine ($C_1$-$C_6$)Alkyl-Gruppe, eine Halogen($C_1$-$C_6$)alkyl-Gruppe, eine($C_1$-$C_6$)Alkoxy-Gruppe, eine Halogen($C_1$-$C_6$)alkoxy-Gruppe, eine ($C_1$-$C_6$)Alkylthio-Gruppe, eine Halogen($C_1$-$C_6$)alkylthio-Gruppe, eine ($C_1$-$C_6$)Alkoxy($C_1$-$C_6$)alkyl-Gruppe, eine Halogen($C_1$-$C_6$)alkoxy($C_1$-$C_6$)alkyl-Gruppe oder
eine

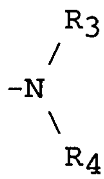

Gruppe (wobei sowohl $R_3$ wie auch $R_4$ ein Wasserstoffatom oder eine ($C_1$-$C_6$)Alkyl-Gruppe sind) ist; und n 0 oder die ganze Zahl 1 oder 2 ist; unter den Voraussetzungen daß, wenn (a) n 0 ist, $R_1$ und $R_2$ nicht beide Methyl-Gruppen sind, (b) wenn $R_1$ und $R_2$ beide ($C_1$-$C_3$)Alkyl sind, oder wenn $R_1$ Wasserstoff ist und $R_2$ ($C_1$-$C_3$)Alkyl ist, und n 1 ist, Y nicht Cl, F, Br, $CH_3$, $OCH_3$, $CF_3$ oder $OCF_2H$ ist, und (c) wenn $R_1$ ein Wasserstoffatom oder eine ($C_1$-$C_3$)Alkyl-Gruppe ist und $R_2$ eine ($C_1$-$C_2$)Alkoxy-Gruppe ist, und n 1 ist, Y nicht $CF_3$ oder $OCF_2H$ ist; oder ein Salz einer derartigen Verbindung.

2. Verbindung nach Anspruch 1, wobei $R_1$ ein Wasserstoffatom oder eine ($C_1$-$C_6$)Alkyl-Gruppe ist, $R_2$ eine ($C_1$-$C_6$)Alkyl-Gruppe ist, Y ein Halogenatom, eine ($C_1$-$C_6$)Alkyl-Gruppe, eine Halogen($C_1$-$C_6$)alkyl-Gruppe, eine ($C_1$-$C_6$)Alkoxy-Gruppe oder eine ($C_1$-$C_6$)Alkoxy($C_1$-$C_6$)alkyl-Gruppe ist und n 0 oder 1 ist, unter den Voraussetzungen daß, wenn (a) n 0 ist, $R_1$ und $R_2$ nicht beide Methyl-Gruppen sind, wenn (b) $R_1$ und $R_2$ beide ($C_1$-$C_3$)Alkyl sind oder wenn $R_1$ Wasserstoff ist und $R_2$ ($C_1$-$C_3$)Alkyl ist, und n 1 ist, Y nicht Cl, F, Br, $CH_3$, $OCH_3$, $CF_3$ oder $OCF_2H$ ist, und wenn (c) $R_1$ ein Wasserstoffatom oder eine ($C_1$-$C_3$)Alkyl-Gruppe ist und $R_2$ eine ($C_1$-$C_2$)Alkoxy-Gruppe ist, und n 1 ist, Y nicht $CF_3$ oder $OCF_2H$ ist; oder ein Salz einer derartigen Verbindung.

3. Verbindung nach Anspruch 1, wobei $R_1$ ein Wasserstoffatom oder eine Methyl-Gruppe ist, $R_2$ eine Methyl-Gruppe ist, Y eine Difluormethyl-Gruppe ist, die an der 6-Position des Pyridinkerns angeordnet ist und n 0 oder 1 ist, unter den Voraussetzungen daß, wenn n 0 ist, $R_1$ und $R_2$ nicht beide Methyl-Gruppen sind; oder ein Salz einer derartigen Verbindung.

4. 6-Difluormethyl-N-[(4,6-dimethoxypyrimidin-2-yl)aminocarbonyl]-3-dimethylaminocarbonyl-2-pyridinsulfonamid.

5. N-[(4,6-Dimethoxypyrimidin-2-yl)aminocarbonyl]-3-methylaminocarbonyl-2-pyridinsulfonamid.

6. N-[(4,6-Dimethoxypyrimidin-2-yl)aminocarbonyl]-3-dimethylaminocarbonyl-6-ethyl-2-pyridinsulfonamid.

7. N-[(4,6-Dimethoxypyrimidin-2-yl)aminocarbonyl]-3-dimethylaminocarbonyl-6-methoxymethyl-2-pyridinsulfonamid.

8. N-[(4,6-Dimethoxypyrimidin-2-yl)aminocarbonyl]-3-(N'-ethyl-N'-methylaminocarbonyl)-2-pyridinsulfonamid.

9. Herbicide Zusammensetzung, im wesentlichen bestehend aus einer herbicid wirksamen Menge einer Verbindung oder eines Salzes davon, wie in irgendeinem vorhergehenden Anspruch definiert, und einem landwirtschaftlich geeigneten Adjuvans.

10. Herbicide Zusammensetzung nach Anspruch 9, wobei das Mischungsverhältnis der Verbindung oder deren Salzes zum landwirtschaftlich geeigneten Adjuvans 1:99 bis 90:10 beträgt.

11. Zusammensetzung nach Anspruch 9, welche auch ein weiteres Herbicid umfaßt.

12. Zusammensetzung nach Anspruch 11, wobei das weitere Herbicid ausgewählt ist aus
3,6-Dichlor-2-methoxybenzoesäure
2,5-Dichlor-3-aminobenzoesäure
(2,4-Dichlorphenoxy)essigsäure
(4-Chlor-2-methylphenoxy)essigsäure
2-Chlor-4,6-bis(ethylamino)-1,3,5-triazin
2-Chlor-4-ethylamino-6-isopropylamino-1,3,5-triazin
2-(4-Chlor-6-ethylamino-1,3,5-triazin-2-ylamino)-2-methylpropionitril
2-Ethylamino-4-isopropylamino-6-methylthio-1,3,5-triazin
2-Chlor-2',6'-diethyl-N-(methoxymethyl)acetanilid
2-Chlor-6'-ethyl-N-(2-methoxy-1-methylethyl)aceto-o-toluidid
2-Chlor-N-isopropylacetanilid
2-Chlor-N,N-di-2-propenylacetamid
S-Ethyldipropylthiocarbamat
S-Ethyl-di-isobutylthiocarbamat
S-Propyl-di-propylthiocarbamat
N-(1-Ethylpropyl)-2,6-dinitro-3,4-xylidin
$\alpha,\alpha,\alpha$-Trifluor-2,6-dinitro-N,N-dipropyl-p-toluidin
2-(3,5-Dichlorphenyl)-2-(2,2,2-trichlorethyl)oxiran
3-Isopropyl-(1H)-benzo-2,1,3-thiadiazin-4-on-2,2-dioxid
3-(3,4-Dichlorphenyl)-1-methoxy-1-methylharnstoff
3,5-Dibrom-4-hydroxybenzonitril
2-Chlor-4-trifluormethylphenyl-3-ethoxy-4-nitrophenylether.

13. Zusammensetzung nach Anspruch 9, welche weiterhin auch ein Herbicid ausgewählt aus
2-Chlor-4-ethylamino-6-isopropylamino-1,3,5-triazin,
2,-(4-Chlor-6-ethylamino-1,3,5-triazin-2-ylamino)-2-methylpropionitril,
2-Chlor-2',6'-diethyl-N-(methoxymethyl)acetanilid,
2-Chlor-6'-ethyl-N-(2-methoxy-1-methylethyl)aceto-o-toluidid und N-(1-Ethylpropyl)-2,6-dinitro-3,4-xylidin enthält.

14. Herbicide Zusammensetzung, umfassend eine herbicid wirksame Menge einer Verbindung nach einem der Ansprüche 1 bis 8 aber ohne die Vorbehalte (b) und (c), einen landwirtschaftlich geeigneten Hilfsstoff und mindestens ein weiteres Herbicid, ausgewählt aus
2,5-Dichlor-3-aminobenzoesäure
(4-Chlor-2-methylphenoxy)essigsäure

2-Chlor-4,6-bis(ethylamino)-1,3,5-triazin
2-Ethylamino-4-isopropylamino-6-methylthio-1,3,5-triazin
2-Chlor-N-isopropylacetanilid
2-Chlor-N,N-di-2-propenylacetamid
S-Propyl-di-propylthiocarbamat
N-(1-Ethylpropyl)-2,6-dinitro-3,4-xylidin
α,α,α-Trifluor-2,6-dinitro-N,N-dipropyl-p-toluidin
3-Isopropyl-(1H)-benzo-2,1,3-thiadiazin-4-on-2,2-dioxid
2-Chlor-4-trifluormethylphenyl-3-ethoxy-4-nitrophenylether.

**15.** Verfahren zum Abtöten schädlicher Unkräuter in einem Kornfeld, wobei eine Zusammensetzung nach einem der Ansprüche 9 bis 14 in einer Menge von 0,1 bis 100 g/Ar, bezogen auf die Verbindung oder deren Salz, verwendet wird.

**16.** Verfahren zum Herstellen einer substituierten Pyridinsulfonamid-Verbindung, die durch die nachfolgende allgemeine Formel

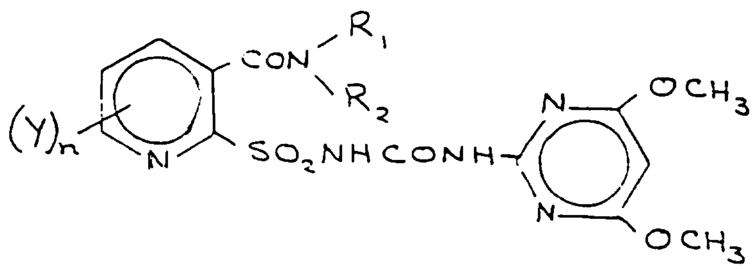

dargestellt ist, wobei sowohl $R_1$ wie auch $R_2$ unabhängig voneinander ein Wasserstoffatom, eine $(C_1$-$C_6)$Alkyl-Gruppe, eine Halogen$(C_1$-$C_6)$alkyl-Gruppe, eine $(C_2$-$C_6)$Alkenyl-Gruppe, eine $(C_2$-$C_6)$Alkinyl-Gruppe, eine $(C_1$-$C_6)$Alkoxy-Gruppe, eine Halogen$(C_1$-$C_6)$alkoxy-Gruppe, eine $(C_1$-$C_6)$Alkoxy$(C_1$-$C_6)$-alkyl-Gruppe, eine Halogen$(C_1$-$C_6)$alkoxy$(C_1$-$C_6)$alkyl-Gruppe, eine $(C_3$-$C_6)$Cycloalkyl-Gruppe, eine Halogen$(C_3$-$C_6)$cycloalkyl-Gruppe, eine$(C_1$-$C_6)$Alkoxycarbonyl-Gruppe, eine Halogen$(C_1$-$C_6)$-alkoxycarbonyl-Gruppe, eine Phenyl-Gruppe oder eine Halogenphenyl-Gruppe sind, unter der Voraussetzung daß, wenn einer der Reste $R_1$ und $R_2$ ein Wasserstoffatom darstellt, der andere eine der Gruppen mit Ausnahme des Wasserstoffatomes ist; $R_1$ und $R_2$ zusammen mit einem benachbarten Stickstoffatom einen Heterocyclus bilden können, Y ein Halogenatom, eine $(C_1$-$C_6)$Alkyl-Gruppe, eine Halogen$(C_1$-$C_6)$alkyl-Gruppe, eine $(C_1$-$C_6)$Alkoxy-Gruppe, eine Halogen$(C_1$-$C_6)$alkoxy-Gruppe, eine $(C_1$-$C_6)$Alkylthio-Gruppe, eine Halogen$(C_1$-$C_6)$alkylthio-Gruppe, eine $(C_1$-$C_6)$Alkoxy$(C_1$-$C_6)$alkyl-Gruppe, eine Halogen$(C_1$-$C_6)$alkoxy$(C_1$-$C_6)$alkyl-Gruppe oder eine

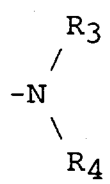

Gruppe ist (wobei sowohl $R_3$ wie auch $R_4$ ein Wasserstoffatom oder eine $(C_1$-$C_6)$Alkyl-Gruppe sind); und n 0 oder die ganze Zahl 1 oder 2 ist; mit dem Vorbehalt daß, wenn n 0 ist, $R_1$ und $R_2$ nicht beide Methyl-Gruppen sind, oder eines Salzes einer derartigen Verbindung, wobei das Verfahren umfaßt: Umsetzen einer der durch die nachfolgende allgemeine Formel

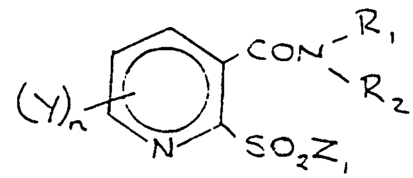

dargestellten Pyridin-Verbindung, wobei $R_1$ und $R_2$ wie zuvor angegeben definiert sind, und $Z_1$ eine $-NH_2$-Gruppe, eine -NCO-Gruppe, eine -NHCOCl-Gruppe oder eine $-NHCOOR_5$-Gruppe (wobei $R_5$ eine Alkyl-Gruppe oder eine Aryl-Gruppe ist) darstellt, mit einer durch die nachfolgende allgemeine Formel

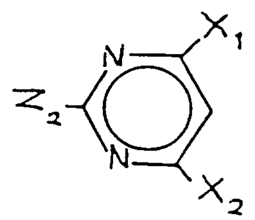

dargestellten Pyrimidin-Verbindung, wobei sowohl $X_1$ wie auch $X_2$ unabhängig voneinander ein Halogenatom oder eine Methoxy-Gruppe sind, und $Z_2$ eine $-NH_2$-Gruppe, eine -NCO-Gruppe, eine -NHCOCl-Gruppe oder eine $-NHCOOR_5$-Gruppe (wobei $R_5$ wie zuvor angegeben definiert ist) darstellt, unter der Voraussetzung daß, wenn $Z_1$ die $-NH_2$-Gruppe ist, $Z_2$ die -NCO-Gruppe, die -NHCOCl-Gruppe oder die $-NHCOOR_5$-Gruppe darstellt, und daß, wenn $Z_2$ die $-NH_2$-Gruppe ist, $Z_1$ die -NCO-Gruppe, die -NHCOCl-Gruppe oder die $-NHCOOR_5$-Gruppe ist; anschließendes Methoxylieren, wenn $X_1$ und/oder $X_2$ ein Halogenatom sind, und gewünschtenfalls Durchführen einer Salzbildungsbehandlung, unter den weiteren Voraussetzungen daß, wenn (a) $R_1$ und $R_2$ beide $(C_1-C_3)$Alkyl sind oder $R_1$ Wasserstoff ist und $R_2$ $(C_1-C_3)$Alkyl ist, n 1 ist, und Y Cl, F, Br, $CH_3$, $OCH_3$, $CF_3$ oder $OCF_2H$ ist, oder daß, wenn (b) $R_1$ ein Wasserstoffatom oder eine $(C_1-C_3)$Alkyl-Gruppe ist und $R_2$ eine $(C_1-C_2)$Alkoxy-Gruppe ist, n 1 ist und Y $CF_3$ oder $OCF_2H$ ist, $Z_1$ dann nicht $-NH_2$ oder $-NHCOOC_6H_5$ ist, wenn $Z_2$ $-NHCOOC_6H_5$ oder $-NH_2$ ist.

**17.** Verfahren nach Anspruch 16, wobei $Z_1$ eine $-NH_2$-Gruppe darstellt und $Z_2$ eine -NCO-Gruppe, eine -NHCOCl-Gruppe oder eine $-NHCOOR_5$-Gruppe ist.

**18.** Verfahren nach an Anspruch 16, wobei $Z_1$ eine -NCO-Gruppe, eine -NHCOCl-Gruppe oder eine $-NHCOOR_5$-Gruppe ist, und $Z_2$ eine $-NH_2$-Gruppe ist.

**19.** Verfahren nach Anspruch 16, wobei die Temperatur der Reaktion der Pyridin-Verbindung mit der Pyrimidin-Verbindung -20 bis +100 °C beträgt.

**20.** Pyridin-Verbindung als durch die nachfolgende allgemeine Formel

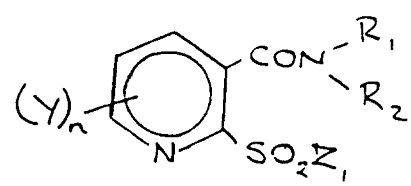

dargestellte Zwischenverbindung, wobei sowohl $R_1$ wie auch $R_2$ unabhängig voneinander ein Wasserstoffatom, eine $(C_1-C_6)$Alkyl-Gruppe, eine Halogen$(C_1-C_6)$alkyl-Gruppe, eine $(C_2-C_6)$Alkenyl-Gruppe, eine $(C_2-C_6)$Alkinyl-Gruppe, eine $(C_1-C_6)$Alkoxy-Gruppe, eine Halogen$(C_1-C_6)$alkoxy-Gruppe, eine $(C_1-C_6)$Alkoxy$(C_1-C_6)$alkyl-Gruppe, eine Halogen$(C_1-C_6)$alkoxy$(C_1-C_6)$alkyl-Gruppe, eine $(C_3-C_6)$Cycloalkyl-Gruppe, eine Halogen$(C_3-C_6)$cycloalkyl-Gruppe, eine$(C_1-C_6)$Alkoxycarbonyl-Gruppe, eine Halogen$(C_1-$

$C_6$)alkoxycarbonyl-Gruppe, eine Phenyl-Gruppe oder eine Halogenphenyl-Gruppe sind, unter der Voraussetzung, daß wenn einer der Reste $R_1$ und $R_2$ ein Wasserstoffatom ist, der andere eine der Gruppen mit Ausnahme des Wasserstoffatomes ist; $R_1$ und $R_2$ zusammen mit einem benachbarten Stickstoffatom einen Heterocyclus bilden können, Y ein Halogenatom, eine ($C_1$-$C_6$)Alkyl-Gruppe, eine Halogen($C_1$-$C_6$)alkyl-Gruppe, eine ($C_1$-$C_6$)Alkoxy-Gruppe, eine Halogen($C_1$-$C_6$)alkoxy-Gruppe, eine ($C_1$-$C_6$)Alkylthio-Gruppe, eine Halogen($C_1$-$C_6$)alkylthio-Gruppe, eine ($C_1$-$C_6$)Alkoxy($C_1$-$C_6$)alkyl-Gruppe, eine Halogen($C_1$-$C_6$)alkoxy($C_1$-$C_6$)alkyl-Gruppe oder eine

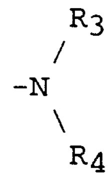

Gruppe ist (wobei sowohl $R_3$ wie auch $R_4$ ein Wasserstoffatom oder eine ($C_1$-$C_6$)Alkyl-Gruppe sind) darstellt; und n 0 oder die ganze Zahl 1 oder 2 ist; und $Z_1$ eine $-NH_2$-Gruppe, eine -NCO-Gruppe, eine -NHCOCl-Gruppe oder eine $-NHCOOR_5$-Gruppe (wobei $R_5$ eine Alkyl- oder Aryl-Gruppe ist) ist, unter den Voraussetzungen, daß wenn (a) n 0 ist, $R_1$ und $R_2$ nicht beide Methyl-Gruppen sind; daß wenn (b) $R_1$ und $R_2$ beide ($C_1$-$C_3$)Alkyl sind, oder wenn $R_1$ Wasserstoff ist und $R_2$ ($C_1$-$C_3$)Alkyl ist, n 1 ist und Y Cl, F, Br, $CH_3$, $OCH_3$, $CF_3$ oder $OCF_2H$ ist, $Z_1$ nicht $-NH_2$ oder $-NHCOOC_6H_5$ ist, und daß wenn (c) $R_1$ ein Wasserstoffatom oder eine ($C_1$-$C_3$)Alkyl-Gruppe und $R_2$ eine ($C_1$-$C_2$)Alkoxy-Gruppe ist, n 1 ist und Y $CF_3$ oder $OCF_2H$ ist, $Z_1$ nicht $-NH_2$ oder $-NHCOOC_6H_5$ ist.

## Patentansprüche für folgende Vertragsstaaten : AT, ES

1. Verfahren zum Herstellen einer substituierten Pyridinsulfonamid-Verbindung, die durch die nachfolgende allgemeine Formel

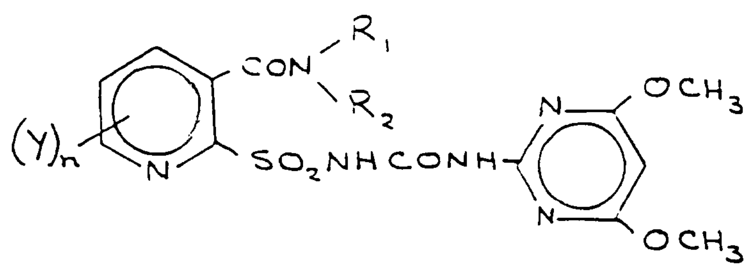

dargestellt ist, wobei sowohl $R_1$ wie auch $R_2$ unabhängig voneinander ein Wasserstoffatom, eine ($C_1$-$C_6$)Alkyl-Gruppe, eine Halogen($C_1$-$C_6$)alkyl-Gruppe, eine ($C_2$-$C_6$)Alkenyl-Gruppe, eine ($C_2$-$C_6$)Alkinyl-Gruppe, eine ($C_1$-$C_6$)Alkoxy-Gruppe, eine Halogen($C_1$-$C_6$)alkoxy-Gruppe, eine ($C_1$-$C_6$)Alkoxy($C_1$-$C_6$)-alkyl-Gruppe, eine Halogen($C_1$-$C_6$)alkoxy($C_1$-$C_6$)alkyl-Gruppe, eine ($C_3$-$C_6$)Cycloalkyl-Gruppe, eine Halogen($C_3$-$C_6$)cycloalkyl-Gruppe, eine ($C_1$-$C_6$)Alkoxycarbonyl-Gruppe, eine Halogen($C_1$-$C_6$)-Alkoxycarbonyl-Gruppe, eine Phenyl-Gruppe oder eine Halogenphenyl-Gruppe sind, unter der Voraussetzung, daß wenn einer der Reste $R_1$ und $R_2$ ein Wasserstoffatom ist, der andere eine der Gruppen mit Ausnahme des Wasserstoffatoms darstellt; $R_1$ und $R_2$ zusammen mit einem benachbarten Stickstoffatom einen Heterocyclus bilden können; Y ein Halogenatom, eine ($C_1$-$C_6$)Alkyl-Gruppe, eine Halogen($C_1$-$C_6$)alkyl-Gruppe, eine($C_1$-$C_6$)Alkoxy-Gruppe, eine Halogen($C_1$-$C_6$)alkoxy-Gruppe, eine ($C_1$-$C_6$)Alkylthio-Gruppe, eine Halogen($C_1$-$C_6$)alkylthio-Gruppe, eine ($C_1$-$C_6$)Alkoxy($C_1$-$C_6$)alkyl-Gruppe, eine Halogen($C_1$-$C_6$)alkoxy($C_1$-$C_6$)alkyl-Gruppe oder
eine

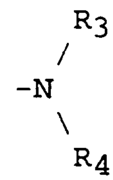

Gruppe (wobei sowohl $R_3$ wie auch $R_4$ ein Wasserstoffatom oder eine $(C_1\text{-}C_6)$Alkyl-Gruppe sind) darstellt; und n 0 oder die ganze Zahl 1 oder 2 ist; unter der Voraussetzung, daß wenn n 0 ist, $R_1$ und $R_2$ nicht beide Methyl-Gruppen sind, oder eines Salzes einer derartigen Verbindung, wobei das Verfahren umfaßt: Umsetzen einer durch die nachfolgende allgemeine Formel

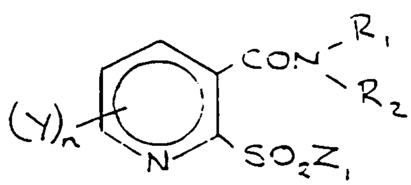

dargestellten Pyridin-Verbindung, wobei $R_1$ und $R_2$ wie zuvor angegeben definiert sind, und $Z_1$ eine $-NH_2$-Gruppe, eine -NCO-Gruppe, eine -NHCOCl-Gruppe oder eine $-NHCOOR_5$-Gruppe (wobei $R_5$ eine Alkyl-Gruppe oder eine Aryl-Gruppe ist) darstellt, mit einer durch die nachfolgende allgemeine Formel

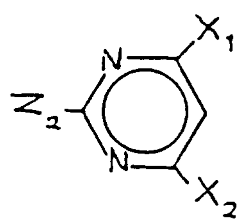

dargestellten Pyrimidin-Verbindung, wobei sowohl $X_1$ wie auch $X_2$ unabhängig voneinander ein Halogenatom oder eine Methoxy-Gruppe sind, und $Z_2$ eine $-NH_2$-Gruppe, eine -NCO-Gruppe, eine -NHCOCl-Gruppe oder eine $-NHCOOR_5$-Gruppe (wobei $R_5$ wie zuvor angegeben definiert ist) ist, unter der Voraussetzung, daß wenn $Z_1$ die $-NH_2$-Gruppe ist, $Z_2$ die -NCO-Gruppe, die -NHCOCl-Gruppe oder die $-NHCOOR_5$-Gruppe ist, und daß, wenn $Z_2$ die $-NH_2$-Gruppe ist, $Z_1$ die -NCO-Gruppe, die -NHCOCl-Gruppe oder die $-NHCOOR_5$-Gruppe darstellt; anschließendes methoxylieren, wenn $X_1$ und/oder $X_2$ ein Halogenatom darstellen; gewünschtenfalls Durchführung einer Salzbildungsbehandlung; unter den weiteren Vorbehalten, daß wenn (a) $R_1$ und $R_2$ beide $(C_1\text{-}C_3)$Alkyl sind oder $R_1$ Wasserstoff ist und $R_2$ $(C_1\text{-}C_3)$Alkyl ist, n 1 ist, und Y Cl, F, Br, $CH_3$, $OCH_3$, $CF_3$ oder $OCF_2H$ ist, oder daß wenn (b) $R_1$ ein Wasserstoffatom oder eine $(C_1\text{-}C_3)$Alkyl-Gruppe ist und $R_2$ eine $(C_1\text{-}C_2)$Alkoxy-Gruppe ist, n 1 ist und Y $CF_3$ oder $OCF_2H$ ist, $Z_1$ dann nicht $-NH_2$ oder $-NHCOOC_6H_5$ ist, wenn $Z_2$ $-NHCOOC_6H_5$ oder $-NH_2$ ist.

2. Verfahren nach Anspruch 1, wobei $Z_1$ eine $-NH_2$-Gruppe ist, und $Z_2$ eine -NCO-Gruppe, eine -NHCOCl-Gruppe oder eine $-NHCOOR_5$-Gruppe ist.

3. Verfahren nach Anspruch 1, wobei $Z_1$ eine -NCO-Gruppe, eine -NHCOCl-Gruppe oder eine $-NHCOOR_5$-Gruppe ist, und $Z_2$ eine $-NH_2$-Gruppe darstellt.

4. Verfahren nach Anspruch 1, wobei die Temperatur der Reaktion der Pyridin-Verbindung mit der Pyrimidin-Verbindung -20 bis + 100 °C beträgt.

5. Verfahren nach einem der vorhergehenden Ansprüche, wobei $R_1$ ein Wasserstoffatom oder eine eine $(C_1\text{-}C_6)$Alkyl-Gruppe ist, $R_2$ eine $(C_1\text{-}C_6)$Alkyl-Gruppe ist, Y ein Halogenatom, eine $(C_1\text{-}C_6)$Alkyl-Gruppe, eine Halogen$(C_1\text{-}C_6)$alkyl-Gruppe, eine $(C_1\text{-}C_6)$Alkoxy-Gruppe oder eine $(C_1\text{-}C_6)$Alkoxy$(C_1\text{-}C_6)$alkyl-Gruppe ist, und n 0 oder 1 ist, mit den Vorbehalten, daß, wenn (a) n 0 ist, $R_1$ und $R_2$ nicht beide

Methyl-Gruppen sind, daß wenn (b) $R_1$ und $R_2$ beide $(C_1-C_3)$Alkyl sind, oder wenn $R_1$ Wasserstoff ist und $R_2$ $(C_1-C_3)$Alkyl ist, und n 1 ist, Y nicht Cl, F, Br, $CH_3$, $OCH_3$, $CF_3$ oder $OCF_2H$ ist, und daß wenn (c) $R_1$ ein Wasserstoffatom oder eine $(C_1-C_3)$Alkyl-Gruppe ist, und $R_2$ eine $(C_1-C_2)$Alkoxy-Gruppe ist, und n 1 ist, Y nicht $CF_3$ oder $OCF_2H$ ist.

**6.** Verfahren nach Anspruch 1, wobei $R_1$ ein Wasserstoffatom oder eine Methyl-Gruppe ist, $R_2$ eine Methyl-Gruppe ist, Y eine in der 6-Position des Pyridinkerns angeordnete Difluormethyl-Gruppe ist, und n 0 oder 1 ist, unter dem Vorbehalt daß wenn n 0 ist, $R_1$ und $R_2$ nicht beide Methyl-Gruppen sind.

**7.** Verfahren nach einem der Ansprüche 1 bis 4, wobei $R_1$ und $R_2$ beide Methyl-Gruppen sind, n 1 ist und Y 6-Difluormethyl ist.

**8.** Verfahren nach einem der Ansprüche 1 bis 4, wobei $R_1$ Wasserstoff ist, $R_2$ eine Methyl-Gruppe ist, und n 0 ist.

**9.** Verfahren nach einem der Ansprüche 1 bis 4, wobei $R_1$ und $R_2$ beide Methyl-Gruppen sind, n 1 ist und Y 6-Ethyl ist.

**10.** Verfahren nach einem der Ansprüche 1 bis 4, wobei $R_1$ und $R_2$ beide Methyl-Gruppen sind, n 1 ist, und Y 6-Methoxymethyl ist.

**11.** Verfahren nach einem der Ansprüche 1 bis 4, wobei $R_1$ Methyl ist, $R_2$ Methyl ist und n 0 ist.

**12.** Verfahren zum Abtöten schädlicher Unkräuter in einem Getreidefeld, wobei 0,1 bis 100 g/Ar, bezogen auf den aktiven Bestandteil, einer Zusammensetzung verwendet werden, welche als aktiven Bestandteil eine substituierte Pyridinsulfonamid-Verbindung und ein landwirtschaftlich geeignetes Adjuvans enthält, wobei die Pyridinsulfonamid-Verbindung durch die allgemeine Formel

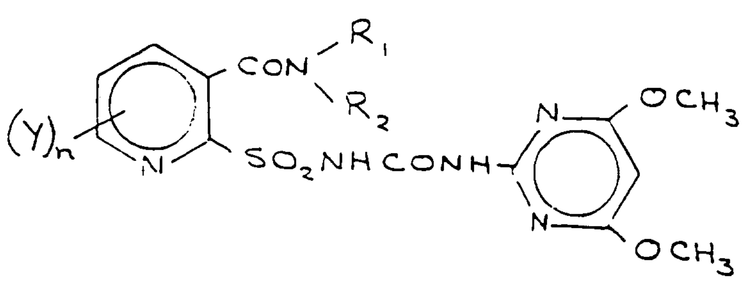

dargestellt ist, wobei sowohl $R_1$ wie auch $R_2$ unabhängig voneinander ein Wasserstoffatom, eine $(C_1-C_6)$Alkyl-Gruppe, eine Halogen$(C_1-C_6)$alkyl-Gruppe, eine $(C_2-C_6)$Alkenyl-Gruppe, eine $(C_2-C_6)$Alkinyl-Gruppe, eine $(C_1-C_6)$Alkoxy-Gruppe, eine Halogen$(C_1-C_6)$alkoxy-Gruppe, eine $(C_1-C_6)$Alkoxy$(C_1-C_6)$-alkyl-Gruppe, eine Halogen$(C_1-C_6)$alkoxy$(C_1-C_6)$alkyl-Gruppe, eine $(C_3-C_6)$Cycloalkyl-Gruppe, eine Halogen$(C_3-C_6)$cycloalkyl-Gruppe, eine$(C_1-C_6)$Alkoxycarbonyl-Gruppe, eine Halogen$(C_1-C_6)$-alkoxycarbonyl-Gruppe, eine Phenyl-Gruppe oder eine Halogenphenyl-Gruppe sind, unter der Voraussetzung, daß wenn einer der Reste $R_1$ und $R_2$ ein Wasserstoffatom ist, der andere eine der Gruppen mit Ausnahme des Wasserstoffatomes darstellt; $R_1$ und $R_2$ zusammen mit einem benachbarten Stickstoffatom einen Heterocyclus bilden können, Y ein Halogenatom, eine $(C_1-C_6)$Alkyl-Gruppe, eine Halogen$(C_1-C_6)$alkyl-Gruppe, eine $(C_1-C_6)$Alkoxy-Gruppe, eine Halogen$(C_1-C_6)$alkoxy-Gruppe, eine $(C_1-C_6)$Alkylthio-Gruppe, eine Halogen$(C_1-C_6)$alkylthio-Gruppe, eine $(C_1-C_6)$Alkoxy$(C_1-C_6)$alkyl-Gruppe, eine Halogen$(C_1-C_6)$alkoxy$(C_1-C_6)$alkyl-Gruppe oder eine

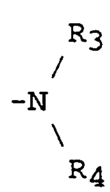

Gruppe ist (wobei sowohl $R_3$ wie auch $R_4$ ein Wasserstoffatom oder eine ($C_1$-$C_6$)Alkyl-Gruppe sind) ist; und n 0 oder die ganze Zahl 1 oder 2 ist; mit den Vorbehalten, daß, wenn (a) n 0 ist, $R_1$ und $R_2$ nicht beide Methyl-Gruppen sind, daß wenn (b) $R_1$ und $R_2$ beide ($C_1$-$C_3$)Alkyl sind, oder wenn $R_1$ Wasserstoff ist, und $R_2$ ($C_1$-$C_3$)Alkyl ist, und n 1 ist, Y nicht Cl, F, Br, $CH_3$, $OCH_3$, $CF_3$ oder $OCF_2H$ ist, und daß wenn (c) $R_1$ ein Wasserstoffatom oder eine ($C_1$-$C_3$)Alkyl-Gruppe ist und $R_2$ eine ($C_1$-$C_2$)-Alkoxy-Gruppe ist, und n 1 ist, Y nicht $CF_3$ oder $OCF_2H$ ist; oder ein Salz einer derartigen Verbindung.

**13.** Verfahren nach Anspruch 12, wobei das Mischungsverhältnis der Verbindung oder deren Salzes zu dem landwirtschaftlich geeigneten Adjuvans 1:99 bis 90:10 beträgt.

**14.** Verfahren nach Anspruch 12, wobei die Zusammensetzung auch ein weiteres Herbicid enthält.

**15.** Verfahren nach Anspruch 14, wobei das weitere Herbicid aus
3,6-Dichlor-2-methoxybenzoesäure
2,5-Dichlor-3-aminobenzoesäure
(2,4-Dichlorphenoxy)essigsäure
(4-Chlor-2-methylphenoxy)essigsäure
2-Chlor-4,6-bis(ethylamino)-1,3,5-triazin
2-Chlor-4-ethylamino-6-isopropylamino-1,3,5-triazin
2-(4-Chlor-6-ethylamino-1,3,5-triazin-2-ylamino)-2-methylpropionitril
2-Ethylamino-4-isopropylamino-6-methylthio-1,3,5-triazin
2-Chlor-2',6'-diethyl-N-(methoxymethyl)acetanilid
2-Chlor-6'-ethyl-N-(2-methoxy-1-methylethyl)aceto-o-toluidid
2-Chlor-N-isopropylacetanilid
2-Chlor-N,N-di-2-propenylacetamid
S-Ethyldipropylthiocarbamat
S-Ethyl-di-isobutylthiocarbamat
S-Propyl-di-propylthiocarbamat
N-(1-Ethylpropyl)-2,6-dinitro-3,4-xylidin
a,a,a-Trifluor-2,6-dinitro-N,N-dipropyl-p-toluidin
2-(3,5-Dichlorphenyl)-2-(2,2,2-trichlorethyl)oxiran
3-Isopropyl-(1H)-benzo-2,1,3-thiadiazin-4-on-2,2-dioxid
3-(3,4-Dichlorphenyl)-1-methoxy-1-methylharnstoff
3,5-Dibrom-4-hydroxybenzonitril
2-Chlor-4-trifluormethylphenyl-3-ethoxy-4-nitrophenylether
ausgewählt ist.

**16.** Verfahren nach Anspruch 14, welches auch ein weiteres Herbicid ausgewählt aus
2-Chlor-4-ethylamino-6-isopropylamino-1,3,5-triazin,
2-(4-Chlor-6-ethylamino-1,3,5-triazin-2-ylamino)-2-methylpropionitril,
2-Chlor-2',6'-diethyl-N-(methoxymethyl)acetanilid,
2-Chlor-6'-ethyl-N-(2-methoxy-1-methylethyl)aceto-o-toluidid und N-(1-Ethylpropyl)-2,6-dinitro-3,4-xylidin enthält.

**17.** Verfahren nach Anspruch 14, wobei die herbicide Zusammensetzung eine herbicid wirksame Menge einer in Anspruch 14 definierten Verbindung, aber ohne die Vorbehalte (b) und (c), ein landwirtschaftlich geeignetes Adjuvans und mindestens ein weiteres Herbicid, ausgewählt aus
2,5-Dichlor-3-aminobenzoesäure
(4-Chlor-2-methylphenoxy)essigsäure
2-Chlor-4,6-bis(ethylamino)-1,3,5-triazin
2-Ethylamino-4-isopropylamino-6-methylthio-1,3,5-triazin
2-Chlor-N-isopropylacetanilid
2-Chlor-N,N-di-2-propenylacetamid
S-Propyl-di-propylthiocarbamat
N-(1-Ethylpropyl)-2,6-dinitro-3,4-xylidin
a,a,a-Trifluor-2,6-dinitro-N,N-dipropyl-p-toluidin
3-Isopropyl-(1H)-benzo-2,1,3-thiadiazin-4-on-2,2-dioxid
2-Chlor-4-trifluormethylphenyl-3-ethoxy-4-nitrophenylether

enthält.

**18.** Herbizide Zusammensetzung der in einem der Ansprüche 12 bis 17 definierten Art.

**19.** Verfahren zum Herstellen einer herbiciden Zusammensetzung, umfassend die Stufe des Mischens (a) eines substituierten Pyridinsulfonamids der in Anspruch 12 definierten Art und gewünschtenfalls eines weiteren wie in Anspruch 14, 15 oder 16 definierten Herbicids, oder (b) eines wie in Anspruch 17 definierten substituierten Pyridinsulfonamids und eines wie in Anspruch 17 definierten weiteren Herbicids, mit einem landwirtschaftlich geeigneten Adjuvans.

**Revendications**
**Revendications pour les Etats contractants suivants : DE, FR, GB, IT, NL, CH, LI**

**1.** Dérivé substitué de pyridthesulfonamide représenté par la formule générale :

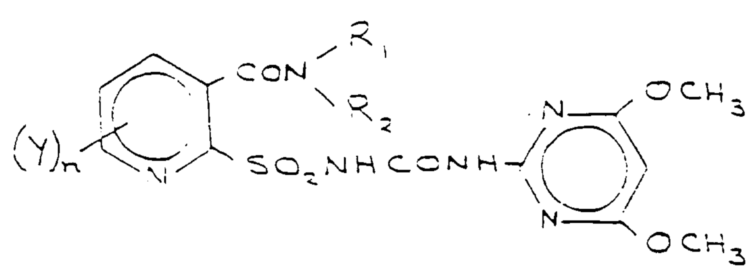

dans laquelle $R_1$ et $R_2$ représentent chacun indépendamment un atome d'hydrogène, un groupe alkyle en $C_1$-$C_6$, un groupe halogénoalkyle en $C_1$-$C_6$, un groupe alcényle en $C_2$-$C_6$, un groupe alcynyle en $C_2$-$C_6$, un groupe alcoxy en $C_1$-$C_6$, un groupe halogénoalcoxy en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$, un groupe cycloalkyle en $C_3$-$C_6$, un groupe halogénocycloalkyle en $C_3$-$C_6$, un groupe alcoxy (en $C_1$-$C_6$) carbonyle, un groupe halogénoalcoxy (en $C_1$-$C_6$) carbonyle, un groupe phényle ou un groupe halogénophényle, pourvu que, lorsque l'un des restes $R_1$ et $R_2$ représente un atome d'hydrogène, l'autre représente l'un des groupes à l'exclusion de l'atome d'hydrogène ; $R_1$ et $R_2$ peuvent former avec l'atome d'azote voisin un hétérocycle ; Y représente un atome d'halogène, un groupe alkyle en $C_1$-$C_6$, un groupe halogénoalkyle en $C_1$-$C_6$, un groupe alcoxy en $C_1$-$C_6$, un groupe halogénoalcoxy en $C_1$-$C_6$, un groupe alkylthio en $C_1$-$C_6$, un groupe halogénoalkylthio en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$ ou un groupe

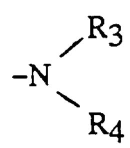

(dans lequel $R_3$ et $R_4$ représentent chacun un atome d'hydrogène ou un groupe alkyle en $C_1$-$C_6$); et n représente 0, 1 ou 2; avec les conditions que (a) lorsque $\underline{n}$ = 0, $R_1$ et $R_2$ ne soient pas tous deux des groupes méthyles, (b) lorsque $R_1$ et $R_2$ sont tous deux des groupes alkyles en $C_1$-$C_3$ ou lorsque $R_1$ est un atome d'hydrogène et $R_2$ est un groupe alkyle en $C_1$-$C_3$, et $\underline{n}$ = 1, Y ne soit pas Cl, F, Br, $CH_3$, $OCH_3$, $CF_3$ ou $OCF_2H$ et (c) lorsque $R_1$ est un atome d'hydrogène ou un groupe alkyle en $C_1$-$C_3$ et $R_2$ est un groupe alcoxy en $C_1$ ou $C_2$ et n = 1, Y ne soit pas $CF_3$ ou $OCF_2H$ ; ou un sel de ce composé.

**2.** Composé selon la revendication 1, dans lequel $R_1$ est un atome d'hydrogène ou un groupe alkyle en $C_1$-$C_6$, $R_2$ est un groupe alkyle en $C_1$-$C_6$, Y est un atome d'halogène, un groupe alkyle en $C_1$-$C_6$, un groupe halogénoalkyle en $C_1$-$C_6$, un groupe alcoxy en $C_1$-$C_6$, ou un groupe alcoxy(en $C_1$-$C_6$) alkyle en $C_1$-$C_6$ et n est égal à 0 ou 1, avec les conditions que (a) lorsque $\underline{n}$ = 0, $R_1$ et $R_2$ ne soient pas tous deux des groupes méthyles, (b) lorsque $R_1$ et $R_2$ sont tous deux des groupes alkyles en $C_1$-$C_3$ ou lorsque $R_1$ est l'hydrogène et $R_2$ est un groupe alkyle en $C_1$-$C_3$ et $\underline{n}$ = 1, Y ne soit pas Cl, F, Br, $CH_3$, $OCH_3$, $CF_3$ ou $OCF_2H$ et (c) lorsque $R_1$ est un atome d'hydrogène ou un groupe alkyle en $C_1$-$C_3$ et $R_2$

est un groupe alcoxy en $C_1$-$C_3$ et n est égal 1, Y ne soit pas $CF_3$ ou $OCF_2H$; ou un sel de ce composé.

3. Composé selon la revendication 1, dans lequel $R_1$ est un atome d'hydrogène ou un groupe méthyle, $R_2$ est un groupe méthyle, Y est un groupe difluorométhyle en position 6 du noyau pyridine et n est égal à 0 ou 1, avec la condition que lorsque n = 0, $R_1$ et $R_2$ ne soient pas tous deux des groupes méthyles; ou un sel de ce composé.

4. Le 6-difluorométhyl-N-[(4,6-diméthoxypyrimidine-2-yl)aminocarbonyl]-3-diméthylaminocarbonyl-2-pyridinesulfonamide.

5. Le N--[(4,6--diméthoxypyrimidine--2--yl)aminocarbonyl]--3-méthylaminocarbonyl-2-pyridinesulfonamide.

6. N-[(4,6-diméthoxypyrimidine-2-yl)aminocarbonyl]-3-diméthylaminocarbonyl-6-éthyl-2-pyridinesulfonamide.

7. Le N-[(4,6--diméthoxypyrimidine--2--yl)aminocarbonyl]--3-diméthylaminocarbonyl-6-méthoxyméthyl-2-pyridinesulfonamide.

8. Le N-[(4,6--diméthoxypyridine--2--yl)aminocarbonyl]-3-(N'-éthyl-N'-méthylaminocarbonyl)-2-pyridinesulfonamide.

9. Composition herbicide consistant essentiellement en une quantité efficace comme herbicide d'un composé tel que défini dans l'une quelconque des revendications précédentes ou d'un de ses sels et un adjuvant acceptable en agriculture.

10. Composition herbicide selon la revendication 9, dans laquelle le rapport de mélange du composé ou de son sel à l'adjuvant acceptable en agriculture est de 1:99 à 99:10.

11. Composition selon la revendication 9, qui contient aussi un autre herbicide.

12. Composition selon la revendication 11, dans laquelle ledit autre herbicide est choisi parmi les suivants :
acide 3,6-dichloro-2-méthoxybenzoïque
acide 2,4-dichloro-3-aminobenzoïque
acide (2,4-dichlorophénoxy)acétique
acide (4-chloro-2-méthylphénoxy)acétique
2-chloro-4,6-bis(éthylamino)-1,3,5-triazine
2-chloro-4-éthylamino-6-isopropylamino-1,3,5-triazine
2-(4-chloro-6-éthylamino-1,3,5-triazine-2-ylamino)-2-méthylpropionitrile
2-éthylamino-4-isopropylamino-6-méthylthio-1,3,5-triazine
2-chloro-2',6'-diéthyl-N-(méthoxyméthyl)acétanilide
2-chloro-6'-éthyl-N-(2-méthoxy-1-méthyléthyl)acéto-o-toluidide
2-chloro-N-isopropylacétanilide
2-chloro-N,N-di-2-propénylacétamide
dipropylthiocarbamate de S-éthyle
di-isobutylthiocarbamate de S-éthyle
dipropylthiocarbamate de S-propyle
N-(1-éthylpropyl)-2,6-dinitro-3,4-xylidine
$\alpha,\alpha,\alpha$-trifluoro-2,6-dinitro-N,N-dipropyl-p-toluidine
2-(3,5-dichlorophényl)-2-(2,2,2-trichloroéthyl)oxiranne
3-isopropyl-(1H)-benzo-2,1,3-thiadiazine-4-one-2,2-dioxyde
3-(3,4-dichlorophényl)-1-méthoxy-1-méthylurée
3,5-dibromo-4-hydroxybenzonitrile
oxyde de 2-chloro-4-trifluorométhylphényle et de 3-éthoxy-4-nitrophényle

13. Composition selon la revendication 9, qui contient aussi un autre herbicide choisi parmi les suivants :
2-chloro-4-éthylamino-4-isopropylamino-1,3,5-triazine
2-(4-chloro-6-éthylamino-1,3,5-triazine-2-ylamino)-2-méthyl-propionitrile
2-chloro-2',6'-diéthyl-N-(méthoxyméthyl)acétanilide

2-chloro-6'-éthyl-N-(2-méthoxy-1-méthyléthyl)acéto-o-toluidide et
N-(1-éthylpropyl)-2,6-dinitro-3,4-xylidine.

**14.** Composition herbicide comprenant une quantité efficace comme herbicide d'un composé selon l'une quelconque des revendications 1 à 8, mais sans les conditions (b) et (c), un auxiliaire acceptable en agriculture et au moins un autre herbicide choisi parmi les suivants :
acide 2,5-dichloro-3-aminobenzoïque
(4-chloro-2-méthylphénoxy)acétique
2-chloro-4,6-bis(éthylamino)-1,3,5-triazine
2-éthylamino-4-isopropylamino-6-méthylthio-1,3,5-triazine
2-chloro-N-isopropylacétanilide
2-chloro-N,N-di-2-propénylacétamide
dipropylthiocarbamate de S-propyle
N-(1-éthylpropyl)-2,6-dinitro-3,4-xylidine
α,α,α-trifluoro-2,6-dinitro-N,N-dipropyl-p-toluidine
3-isopropyl-(1H)-benzo-2,1,3-thiadiazine-4-one-2,2-dioxyde
oxyde de 2-chloro-4-trifluorométhylphényle et de 3-éthoxy-4-nitrophényle

**15.** Procédé pour tuer les mauvaises herbes nuisibles dans un champ de maïs par application d'une composition selon l'une quelconque des revendications 9 à 14, en quantités de 0,1 à 100 g/are, rapportée audit composé ou à son sel.

**16.** Procédé pour produire un dérivé substitué de pyridinesulfonamide représenté par la formule générale suivante :

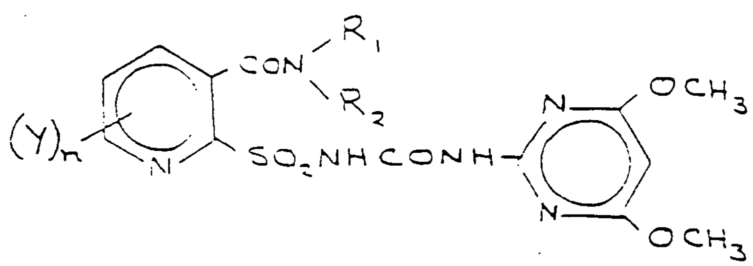

dans laquelle $R_1$ et $R_2$ représentent chacun indépendamment un atome d'hydrogène, un groupe alkyle en $C_1$-$C_6$, un groupe halogénoalkyle en $C_1$-$C_6$, un groupe alcényle en $C_2$-$C_6$, un groupe alcynyle en $C_2$-$C_6$, un groupe alcoxy en $C_1$-$C_6$, un groupe halogénoalcoxy en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$, un groupe cycloalkyle en $C_3$-$C_6$, un groupe halogénocycloalkyle en $C_3$-$C_6$, un groupe alcoxy (en $C_1$-$C_6$) carbonyle, un groupe halogénoalcoxy (en $C_1$-$C_6$) carbonyle, un groupe phényle ou un groupe halogénophényle, pourvu que, lorsque l'un des restes $R_1$ et $R_2$ représente un atome d'hydrogène, l'autre représente l'un des groupes à l'exclusion de l'atome d'hydrogène ; $R_1$ et $R_2$ peuvent former avec l'atome d'azote voisin un hétérocycle ; Y représente un atome d'halogène, un groupe alkyle en $C_1$-$C_6$, un groupe halogénoalkyle en $C_1$-$C_6$, un groupe alcoxy en $C_1$-$C_6$, un groupe halogénoalcoxy en $C_1$-$C_6$, un groupe alkylthio en $C_1$-$C_6$, un groupe halogénoalkylthio en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$ ou un groupe

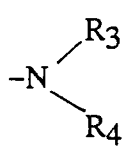

(dans lequel $R_3$ et $R_4$ représentent chacun un atome d'hydrogène ou un groupe alkyle en $C_1$-$C_6$); et n représente 0, 1 ou 2; avec la condition que lorsque n = 0, $R_1$ et $R_2$ ne soient pas tous deux des groupes méthyles ; ou un sel de ce composé;

ledit procédé comprenant la réaction d'un dérivé de pyridine représenté par la formule générale suivante :

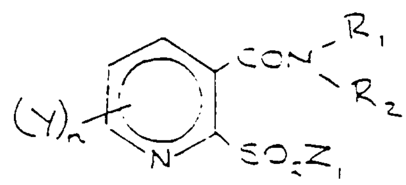

dans laquelle $R_1$ et $R_2$ sont tels que définis ci-dessus et $Z_1$ représente un groupe $-NH_2$, un groupe -NCO, un groupe -NHCOCl ou un groupe $-NHCOOR_5$ (dans lequel $R_5$ représente un groupe alkyle ou un groupe aryle) avec un dérivé de pyrimidine représenté par la formule générale suivante

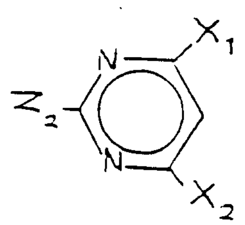

dans laquelle $X_1$ et $X_2$ représentent chacun indépendamment un atome d'halogène ou un groupe méthoxy et $Z_2$ représente un groupe $-NH_2$, un groupe -NCO, un groupe -NHCOCl ou un groupe $-NHCOOR_5$ (dans lequel $R_5$ est tel que défini ci-dessus), pourvu que lorsque $Z_1$ représente le groupe $-NH_2$, $Z_2$ représente le groupe -NCO, le groupe -NHCOCl ou le groupe $-NHCOOR_5$ et que lorsque $Z_2$ représente le groupe $-NH_2$, $Z_1$ représente le groupe -NCO, le groupe -NHCOCl ou le groupe $-NHCOR_5$ ; ensuite, la méthoxylation lorsque $X_1$ et/ou $X_2$ représentent un atome d'halogène; et, si on le désire, le traitement de salification; avec les autres conditions que (a) lorsque $R_1$ et $R_2$ sont tous deux des groupes alkyles en $C_1$-$C_3$ ou $R_1$ est un atome d'hydrogène et $R_2$ est un groupe alkyle en $C_1$-$C_3$, $\underline{n}$ = 1 et Y est Cl, F, Br, $CH_3$, $OCH_3$, $CF_3$ ou $OCF_2H$ ou bien (b) lorsque $R_1$ est un atome d'hydrogène ou un groupe alkyle en $C_1$-$C_3$ et $R_2$ est un groupe alcoxy en $C_1$ ou $C_2$, $\underline{n}$ = 1 et Y est un groupe $CF_3$ ou $OCF_2H$, alors $Z_1$ ne soit pas $-NH_2$ ou $-NHCOOC_6H_5$ lorsque $Z_2$ est $-NHCOOC_6H_5$ ou $-NH_2$, respectivement.

**17.** Procédé selon la revendication 16, dans lequel $Z_1$ représente un groupe $-NH_2$ et $Z_2$ représente un groupe -NCO, un groupe -NHCOCl ou un groupe $-NHCOR_5$.

**18.** Procédé selon la revendication 16, dans lequel $Z_1$ représente un groupe -NCO, un groupe -NHCOCl ou un groupe $-NHCOOR_5$ et $Z_2$ représente un groupe $-NH_2$.

**19.** Procédé selon la revendication 16, dans lequel la température de réaction du dérivé de pyridine avec le dérivé de pyrimidine est de -20 à +100 °C.

**20.** Dérivé de pyridine comme intermédiaire représenté par la formule générale suivante :

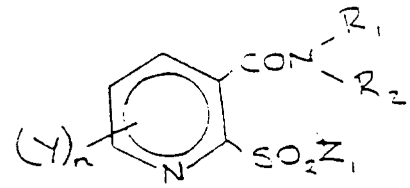

dans laquelle $R_1$ et $R_2$ représentent chacun indépendamment un atome d'hydrogène, un groupe alkyle

en $C_1$-$C_6$, un groupe halogénoalkyle en $C_1$-$C_6$, un groupe alcényle en $C_2$-$C_6$, un groupe alcynyle en $C_2$-$C_6$, un groupe alcoxy en $C_1$-$C_6$, un groupe halogénoalcoxy en $C_1$-$C_6$, un groupe alcoxy (en $C_1$-$C_6$) alkyle en $C_1$-$C_6$, un groupe halogénoalcoxy en ($C_1$-$C_6$) alkyle en $C_1$-$C_6$, un groupe cycloalkyle en $C_3$-$C_6$, un groupe halogénocycloalkyle en $C_3$-$C_6$, un groupe alcoxy (en $C_1$-$C_6$) carbonyle, un groupe halogénoalcoxy (en $C_1$-$C_6$) carbonyle, un groupe phényle ou un groupe halogénophényle, pourvu que lorsque l'un des restes $R_1$ et $R_2$ représente un atome d'hydrogène, l'autre représente l'un des groupes à l'exclusion de l'atome d'hydrogène ; $R_1$ et $R_2$ pris ensemble avec un atome d'azote voisin peuvent fournir un hétérocycle ; Y représente un atome d'halogène, un groupe alkyle en $C_1$-$C_6$, un groupe halogénoalkyle en $C_1$-$C_6$, un groupe alcoxy en $C_1$-$C_6$, un groupe halogénoalcoxy en $C_1$-$C_6$, un groupe alkylthio en $C_1$-$C_6$, un groupe halogénoalkylthio en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$ ou un groupe

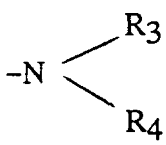

(dans lequel $R_3$ et $R_3$ représentent chacun un atome d'hydrogène ou un groupe alkyle en $C_1$-$C_6$); n représente 0, 1 ou 2; et $Z_1$ est un groupe $-NH_2$, un groupe -NCO, un groupe -NHCOCl ou un groupe $-NHCOOR_5$ (dans lequel $R_5$ est un groupe alkyle ou un groupe aryle), avec les conditions que (a) lorsque n = 0, $R_1$ et $R_2$ ne soient pas tous deux des groupes méthyles, (b) lorsque $R_1$ et $R_2$ sont tous deux des groupes alkyles en $C_1$-$C_3$ ou lorsque $R_1$ est un atome d'hydrogène et $R_2$ est un groupe alkyle en $C_1$-$C_3$, n = 1 et Y est Cl, F, Br, $CH_3$, $OCH_3$, $CF_3$ ou $OCF_2H$, $Z_1$ ne soit pas $-NH_2$ ou $-NHCOOC_6H_5$ et (c) lorsque $R_1$ est un atome d'hydrogène ou un groupe alkyle en $C_1$-$C_3$ et $R_2$ est un groupe alcoxy en $C_1$-$C_2$, n = 1 et Y est $CF_3$ ou $OCF_2H$, $Z_1$ ne soit pas $-NH_2$ ou $-NHCOOC_6H_5$.

**Revendications pour les Etats contractants suivants : AT, ES**

**1.** Procédé pour produire un dérivé substitué de pyridinesulfonamide représenté par la formule générale :

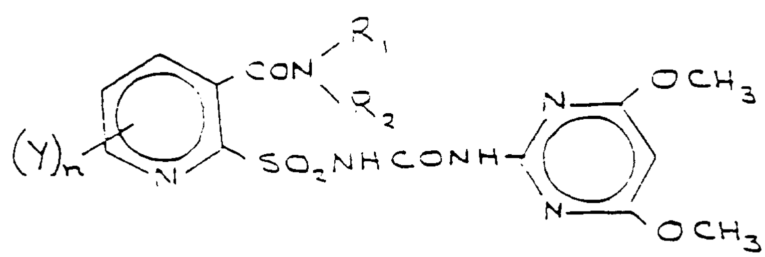

dans laquelle $R_1$ et $R_2$ représentent chacun indépendamment un atome d'hydrogène, un groupe alkyle en $C_1$-$C_6$, un groupe halogénoalkyle en $C_1$-$C_6$, un groupe alcényle en $C_2$-$C_6$, un groupe alcynyle en $C_2$-$C_6$, un groupe alcoxy en $C_1$-$C_6$, un groupe halogénoalcoxy en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$, un groupe cycloalkyle en $C_3$-$C_6$, un groupe halogénocycloalkyle en $C_3$-$C_6$, un groupe alcoxy (en $C_1$-$C_6$) carbonyle, un groupe halogénoalcoxy (en $C_1$-$C_6$) carbonyle, un groupe phényle ou un groupe halogénophényle, pourvu que, lorsque l'un des restes $R_1$ et $R_2$ représente un atome d'hydrogène, l'autre représente l'un des groupes à l'exclusion de l'atome d'hydrogène ; $R_1$ et $R_2$ peuvent former avec l'atome d'azote voisin un hétérocycle ; Y représente un atome d'halogène, un groupe alkyle en $C_1$-$C_6$, un groupe halogénoalkyle en $C_1$-$C_6$, un groupe alcoxy en $C_1$-$C_6$, un groupe halogénoalcoxy en $C_1$-$C_6$, un groupe alkylthio en $C_1$-$C_6$, un groupe halogénoalkylthio en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$ ou un groupe

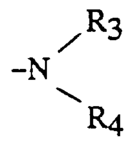

(dans lequel $R_3$ et $R_4$ représentent chacun un atome d'hydrogène ou un groupe alkyle en $C_1$-$C_6$); et n représente 0, 1 ou 2 ; à la condition que lorsque n = 0, $R_1$ et $R_2$ ne soient pas tous deux des groupes méthyles ou un sel de ce composé;

ledit procédé comprenant la réaction de dérivé de pyridine représentée par la formule générale suivante :

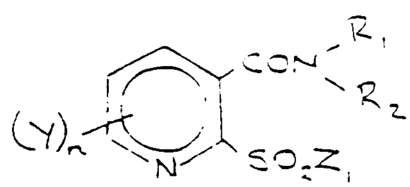

dans laquelle $R_1$ et $R_2$ sont tels que définis ci-dessus et $Z_1$ représente un groupe $-NH_2$, un groupe -NCO, un groupe -NHCOCl ou un groupe $-NHCOOR_5$ (dans lequel $R_5$ représente un groupe alkyle ou un groupe aryle) avec un dérivé de pyrimidine représenté par la formule générale suivante

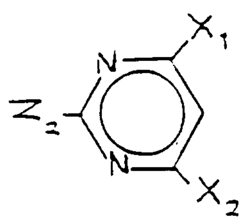

dans laquelle $X_1$ et $X_2$ représentent chacun indépendamment un atome d'halogène ou un groupe méthoxy et $Z_2$ représente un groupe $-NH_2$, un groupe -NCO, un groupe -NHCOCl ou un groupe $-NHCOOR_5$ (dans lequel $R_5$ est tel que défini ci-dessus), pourvu que lorsque $Z_1$ représente le groupe $-NH_2$, $Z_2$ représente le groupe -NCO, le groupe -NHCOCl ou le groupe $-NHCOOR_5$ et que lorsque $Z_2$ représente le groupe $-NH_2$, $Z_1$ représente le groupe -NCO, le groupe -NHCOCl ou le groupe $-NHCOR_5$ ; ensuite, la méthoxylation lorsque $X_1$ et/ou $X_2$ représentent un atome d'halogène; et, si on le désire, le traitement de salification; avec les autres conditions que (a) lorsque $R_1$ et $R_2$ sont tous deux des groupes alkyles en $C_1$-$C_3$ ou $R_1$ est un atome d'hydrogène et $R_2$ est un groupe alkyle en $C_1$-$C_3$, n = 1 et Y est Cl, F, Br, $CH_3$, $OCH_3$, $CF_3$ ou $OCF_2H$ ou bien (b) lorsque $R_1$ est un atome d'hydrogène ou un groupe alkyle en $C_1$-$C_3$ et $R_2$ est un groupe alcoxy en $C_1$ ou $C_2$, n = 1 et Y est un groupe $CF_3$ ou $OCF_2H$, alors $Z_1$ ne soit pas $NH_2$ ou $-NHCOOC_6H_5$ lorsque $Z_2$ est $-NHCOOC_6H_5$ ou $-NH_2$, respectivement.

**2.** Procédé selon la revendication 1, dans lequel $Z_1$ représente un groupe $-NH_2$ et $Z_2$ représente un groupe -NCO, un groupe -NHCOCl ou un groupe $-NHCOR_5$.

**3.** Procédé selon la revendication 1, dans lequel $Z_1$ représente un groupe -NCO, un groupe -NHCOCl ou un groupe $-NHCOOR_5$ et $Z_2$ représente un groupe $-NH_2$.

**4.** Procédé selon la revendication 1, dans lequel la température de réaction du dérivé de pyridine avec le dérivé de pyrimidine est de -20 à +100 °C.

**5.** Procédé selon l'une quelconque des revendications précédentes dans lequel $R_1$ est un atome d'hydrogène ou un groupe alkyle en $C_1$-$C_6$, $R_2$ est un groupe alkyle en $C_1$-$C_6$, Y est un atome d'halogène, un groupe alkyle en $C_1$-$C_6$, un groupe halogénoalkyle en $C_1$-$C_6$, un groupe alcoxy en $C_1$-

$C_6$, ou un groupe alcoxy(en $C_1$-$C_6$) alkyle en $C_1$-$C_6$ et n est égal à 0 ou 1, avec les conditions que (a) lorsque n = 0, $R_1$ et $R_2$ ne soient pas tous deux des groupes méthyles, (b) lorsque $R_1$ et $R_2$ sont tous deux des groupes alkyles en $C_1$-$C_3$ ou lorsque $R_1$ est l'hydrogène et $R_2$ est un groupe alkyle en $C_1$-$C_3$ et n = 1, Y ne soit pas Cl, F, Br, $CH_3$, $OCH_3$, $CF_3$ ou $OCF_2H$ et (c) lorsque $R_1$ est un atome d'hydrogène ou un groupe alkyle en $C_1$-$C_3$ et $R_2$ est un groupe alcoxy en $C_1$-$C_3$ et n est égal à 1, Y ne soit pas $CF_3$ ou $OCF_2H$.

**6.** Procédé selon la revendication 1, dans lequel $R_1$ est un atome d'hydrogène ou un groupe méthyle, $R_2$ est un groupe méthyle, Y est un groupe difluorométhyle en position 6 du noyau pyridine et n est égal à 0 ou 1, avec la condition que lorsque n = 0, $R_1$ et $R_2$ ne soient pas tous deux des groupes méthyles.

**7.** Procédé selon l'une quelconque des revendications 1 à 4, dans lequel $R_1$ et $R_2$ sont tous deux des groupes méthyles, n = 1 et Y est un groupe 6-difluorométhyle.

**8.** Procédé selon l'une quelconque des revendications 1 à 4, dans lequel $R_1$ est un atome d'hydrogène, $R_2$ est un groupe méthyle et n = 0.

**9.** Procédé selon l'une quelconque des revendications 1 à 4, dans lequel $R_1$ et $R_2$ sont tous deux des groupes méthyles n = 1 et Y est un groupe 6-éthyle.

**10.** Procédé selon l'une quelconque des revendications 1 à 4, dans lequel $R_1$ et $R_2$ sont tous deux des groupes méthyles, n = 1 et Y est un groupe 6-méthoxyméthyle.

**11.** Procédé selon l'une quelconque des revendications 1 à 4, dans lequel $R_1$ est un groupe méthyle, $R_2$ est un groupe méthyle, n = 0.

**12.** Procédé pout tuer des mauvaises herbes nuisibles dans un champ maïs pour l'application de 0,1 à 100 g/bar, en ingrédient actif, d'une composition contenant comme ingrédient actif un dérivé substitué de pyridinesulfonamide et un auxiliaire acceptable en agriculture, ledit dérivé de pyridinesulfonamide étant représenté par la formule générale :

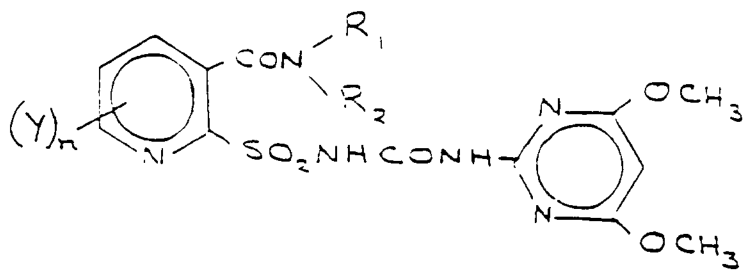

dans laquelle $R_1$ et $R_2$ représentent chacun indépendamment un atome d'hydrogène, un groupe alkyle en $C_1$-$C_6$, un groupe halogénoalkyle en $C_1$-$C_6$, un groupe alcényle en $C_2$-$C_6$, un groupe alcynyle en $C_2$-$C_6$, un groupe alcoxy en $C_1$-$C_6$, un groupe halogénoalcoxy en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$), un groupe cycloalkyle en $C_3$-$C_6$, un groupe halogénocycloalkyle en $C_3$-$C_6$, un groupe alcoxy (en $C_1$-$C_6$) carbonyle, un groupe halogénoalcoxy (en $C_1$-$C_6$) carbonyle, un groupe phényle ou un groupe halogénophényle, pourvu que, lorsque l'un des restes $R_1$ et $R_2$ représente un atome d'hydrogène, l'autre représente l'un des groupes à l'exclusion de l'atome d'hydrogène ; $R_1$ et $R_2$ peuvent former avec l'atome d'azote voisin un hétérocycle ; Y représente un atome d'halogène, un groupe alkyle en $C_1$-$C_6$, un groupe halogénoalkyle en $C_1$-$C_6$, un groupe alcoxy en $C_1$-$C_6$, un groupe halogénoalcoxy en $C_1$-$C_6$, un groupe alkylthio en $C_1$-$C_6$, un groupe halogénoalkylthio en $C_1$-$C_6$, un groupe alcoxy (en $C_1$-$C_6$) alkyle en $C_1$-$C_6$, un groupe halogénoalcoxy (en $C_1$-$C_6$) alkyle en $C_1$-$C_6$ ou un groupe

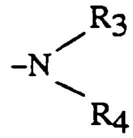

(dans lequel $R_3$ et $R_4$ représentent chacun un atome d'hydrogène ou un groupe alkyle en $C_1$-$C_6$) ; et n représente 0, 1 ou 2; avec les conditions que (a) lorsque n = 0, $R_1$ et $R_2$ ne soient pas tous deux des groupes méthyles, (b) lorsque $R_1$ et $R_2$ sont tous deux des groupes alkyles en $C_1$-$C_3$ ou lorsque $R_1$ est un atome d'hydrogène et $R_2$ est un groupe alkyle en $C_1$-$C_3$ et n = 1, Y ne soit pas Cl, F, Br, $CH_3$, $OCH_3$, $CF_3$ ou $OCF_2H$ et (c) lorsque $R_1$ est un atome d'hydrogène ou un groupe alyle en $C_1$-$C_3$ et $R_2$ est un groupe alcoxy en $C_1$ ou $C_2$ et n = 1, Y ne soit pas $CF_3$ ou $OCF_2H$; ou un sel de ce composé.

**13.** Procédé selon la revendication 12, dans lequel le rapport de mélange du composé ou son sel à l'auxiliaire accepteur dans l'agriculture est de 1:99 à 99:10.

**14.** Procédé selon la revendication 12, dans lequel la composition contient également un autre herbicide.

**15.** Procédé selon la revendication 14, dans lequel ledit autre herbicide est choisi parmi les suivants :
acide 3,6-dichloro-2-méthoxybenzoïque
acide 2,5-dichloro-3-aminobenzoïque
acide (2,4-dichlorophénoxy)acétique
acide (4-chloro-2-méthylphénoxy)acétique
2-chloro-4,6-bis(éthylamino)-1,3,5-triazine
2-chloro-4-éthylamino-6-isopropylamino-1,3,5-triazine
2-(4-chloro-6-éthylamino-1,3,5-triazine-2-ylamino)-2-méthylpropionitrile
2-éthylamino-4-isopropylamino-6-méthylthio-1,3,5-triazine
2-chloro-2',6'-diéthyl-N-(méthoxyméthyl)acétanilide
2-chloro-6'-éthyl-N-(2-méthoxy-1-méthyléthyl)acéto-o-toluidide
2-chloro-N-isopropylacétanilide
2-chloro-N,N-di-2-propénylacétamide
dipropylthiocarbamate de S-éthyle
di-isobutylthiocarbamate de S-éthyle
dipropylthiocarbarnate de S-propyle
N-(1-éthylpropyl)-2,6-dinitro-3,4-xylidine
$\alpha,\alpha,\alpha$-trifluoro-2,6-dinitro-N,N-dipropyl-p-toluidine
2-(3,5-dichlorophényl)-2-(2,2,2-trichloroéthyl)oxiranne
3-isopropyl-(1H)-benzo-2,1,3-thiadiazine-4-one-2,2-dioxyde
3-(3,4-dichlorophényl)-1-méthoxy-1-méthylurée
3,5-dibromo-4-hydroxybenzonitrile
oxyde de 2-chloro-4-trifluorométhylphényle et de 3-éthoxy-4-nitrophényle

**16.** Procédé selon la revendication 14, dans lequel la composition herbicide contient également un herbicide choisi parmi les suivants :
2-chloro-4-éthylamino-4-isopropylamino-1,3,5-triazine
2-(4-chloro-6-éthylamino-1,3,5-triazine-2-ylamino)-2-méthyl-propionitrile
2-chloro-2',6'-diéthyl-N-(méthoxyméthyl)acétanilide
2-chloro-6'-éthyl-N-(2-méthoxy-1-méthyléthyl)acéto-o-toluidide et
N-(1-éthylpropyl)-2,6-dinitro-3,4-xylidine.

**17.** Procédé selon la revendication 14, dans lequel la composition herbicide comprend une quantité efficace comme herbicide d'un composé selon la revendication 14 mais sans les conditions (b) et (c) un adjuvant acceptable en agriculture et au moins un autre herbicide choisi parmi les suivants
acide 2,5-dichloro-3-aminobenzoïque
acide (4-chloro-2-méthylphénoxy)acétique
2-chloro-4,6-bis(éthylamino)-1,3,5-triazine
2-éthylamino-4-isopropylamino-6-méthylthio-1,3,5-triazine
2-chloro-N-isopropylacétanilide

2-chloro-N,N-di-2-propénylacétamide
dipropylthiocarbamate de S-propyle
N-(1-éthylpropyl)-2,6-dinitro-3,4-xylidine
$\alpha,\alpha,\alpha$-trifluoro-2,6-dinitro-N,N-dipropyl-p-toluidine
3-isopropyl-(1H)-benzo-2,1,3-thiadiazine-4-one-2,2-dioxyde
oxyde de 2-chloro-4-trifluorométhylphényle et de 3-éthoxy-4-nitrophényle

**18.** Composition herbicide du type défini dans l'une quelconque des revendications 12 à 17.

**19.** Procédé de fabrication d'une composition herbicide comprenant l'étape de mélange (a) d'un dérivé substitué de pyridinesulfonamide du type défini à la revendication 12 et facultativement un autre herbicide tel que défini à la revendication 14, 15 ou 16 ou (b) d'un dérivé substitué de pyridinesulfonamide tel que défini à la revendication 17 et d'un autre herbicide tel que défini à la revendication 17, avec un adjuvant acceptable en agriculture.